# EUROPÄISCHE PATENTSCHRIFT

(11) **EP 0 568 876 B1**
(45) Veröffentlichungstag und Bekanntmachung des Hinweises auf die Patenterteilung: **28.08.1996**
(21) Anmeldenummer: 93106628.6
(22) Anmeldetag: 23.04.1993
(51) Int. Cl.: C09B 62/08, C09B 62/507, C07C 317/18, C07C 323/12, C07C 381/02

(54) **Reaktivfarbstoffe, deren Herstellung und Verwendung**
Reactive dyes, their preparation and their use
Colorants réactifs, leur préparation et leur utilisation

(30) Priorität: 04.05.1992 DE 4214743; 16.02.1993 DE 4304614
(43) Veröffentlichungstag der Anmeldung: 10.11.1993
(73) Patentinhaber: BAYER AG, 51368 Leverkusen (DE)
(72) Erfinder: Bootz, Konrad, Dr., W-5802 Wetter 4 (DE); Hoppe, Manfred, Dr., W-5067 Kürten-Bechen (DE); Bock, Eckhard, W-5067 Kürten-Bechen (DE); Reddig, Wolfram, Dr., W-5060 Bergisch Gladbach (DE); Eizenhöfer, Thomas, Dr., W-5000 Köln 60 (DE); Harms, Wolfgang, Dr., W-5068 Odenthal (DE); Herd, Karl-Josef, Dr., W-5068 Odenthal-Holz (DE)

(56) Entgegenhaltungen:
- EP-A- 0 070 806
- EP-A- 0 070 807
- EP-A- 0 074 928
- EP-A- 0 141 367
- FR-A- 2 346 418

## Beschreibung

Die Erfindung betrifft neue bi- und polyfunktionelle Reaktivfarbstoffe, ihre Herstellung und Verwendung.

Bifunktionelle Reaktivfarbstoffe sind zwar beispielsweise aus der DE-A-2 614 550, EP-A-70 807, EP-A-70 806 und EP-A-74 928 und tri- und tetrafunktionelle Reaktivfarbstoffe beispielsweise aus EP-A-395 951 bekannt, jedoch weisen die bekannten Reaktivfarbstoffe noch diverse anwendungstechnische Nachteile, beispielsweise eine zu geringe Fixiersbeute, auf.

Die vorliegende Erfindung betrifft neue Reaktivfarbstoffe der Formel 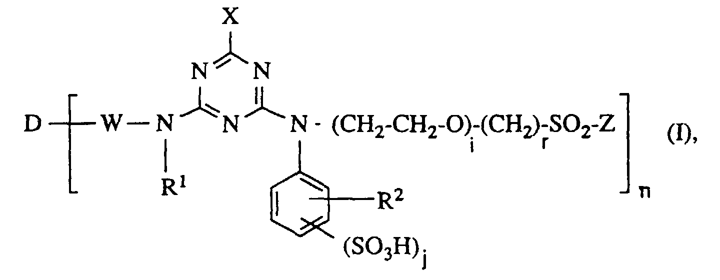 worin
- D: Rest eines organischen Farbstoffes aus der Monoazo-, Polyazo- oder Metallkomplexazoreihe,
- W: direkte Bindung oder Brückenglied,
- R¹: H, C₁-C₄-Alkyl, das durch OR, OSO₃H, SO₃H, COOR oder Halogen substituiert sein kann,
- R: H, CH₃ oder C₂H_{5,}
- R²: H, C₁-C₄-Alkyl, Cl, Br, C₁-C₄-Alkoxy oder COOH,
- i: 0 oder 1,
- j: 0, 1 oder 2,
- r: 2 oder 3, vorzugsweise 2,
- X: F, Cl oder Br,
- Z: -CH=CH₂, -CH₂-CH₂-OSO₃H, -CH₂-CH₂-S₂O₃H, -CH₂-CH₂-O-CO-CH₃, -CH₂-CH₂-OPO₃H₂, -CH₂-CH₂-OH,
- n: 1 oder 2,
bedeuten.

Besonders bevorzugte Reste Z sind -CH=CH₂ und -C₂H₄-OSO₃H.

Für die in der vorliegenden Anmeldung genannten Alkyl-, Aryl-, Aralkyl-, Hetaryl-, Alkoxy-, Halogen und Acylaminoreste sowie für die Brückenglieder gilt folgendes:

Unter Alkylgruppen werden insbesondere solche mit 1 bis 4 C-Atomen verstanden, die gegebenenfalls Substituenten aufweisen können, beispielsweise Halogen wie Cl oder Br, OH, CN, CO₂H, SO₃H oder OSO₃H.

Unter Alkoxyresten werden insbesondere solche mit 1 bis 4 C-Atomen verstanden.

Unter Halogen wird insbesondere Chlor oder Fluor verstanden.

Unter Acylaminoresten werden insbesondere solche mit 1 bis 4 C-Atomen verstanden, wie Formylamino, Acetylamino, Propionylamino, n-Butyrylamino.

Geeignete Brückenglieder W sind beispielsweise: 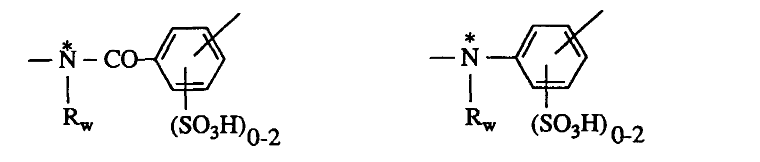 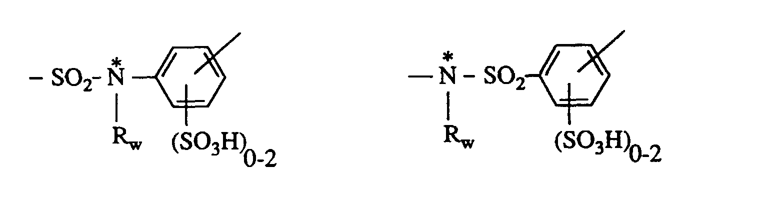 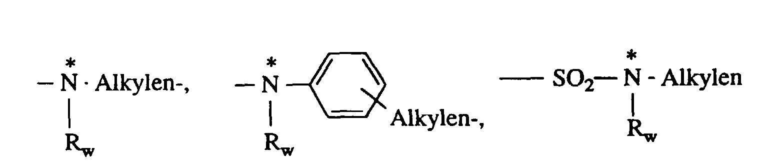 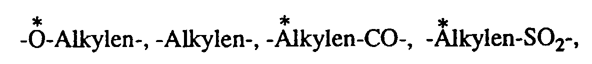 wobei R_{w} für Wasserstoff oder Alkyl steht, und Alkylen einen Alkylenrest mit 1 bis 6 C-Atomen bedeutet, wobei * das Atom bzw. die Gruppe kennzeichnet, welches mit dem Chromophor D verbunden ist

Als Alkylenreste seien aufgeführt: -CH₂-, -CH₂-CH₂-, 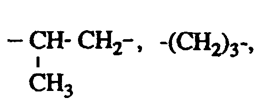 -(CH₂)₄-, -(CH₂)₆-.

Die vorliegende Erfindung betrifft weiterhin die Herstellung der Reaktivfarbstoffe der Formel (I) nach an sich bekannten Methoden:
a) entweder durch Kondensation von Farbstoffen der Formel 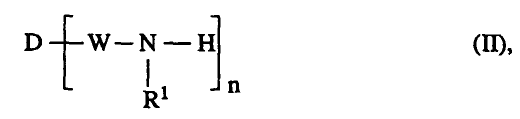 worin
   D, W, R¹ und n die oben angegebene Bedeutung haben,
   mit n-Molen Trihalogentriazinen der Formel 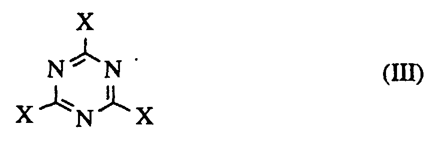 zu Verbindungen der Formel 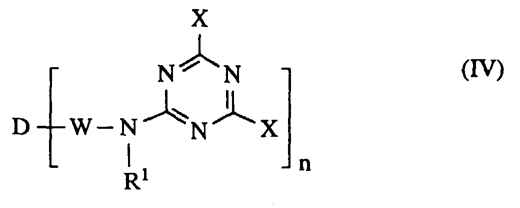 und weitere Kondensation der Verbindungen der Formel (IV) mit n-Molen der Komponenten der Formel 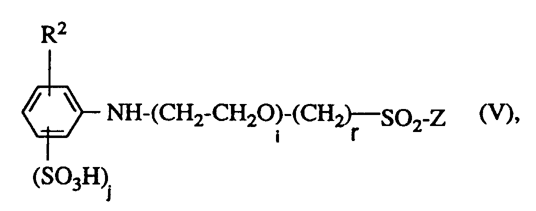 worin
   R², i, j, r, X und Z die oben angegebene Bedeutung haben,
   oder
b) in umgekehrter Reihenfolge durch Kondensation von Trihalogentriazinen der Formel (III) mit den Komponenten der Formel (V) zu den Primärkondensationsprodukten 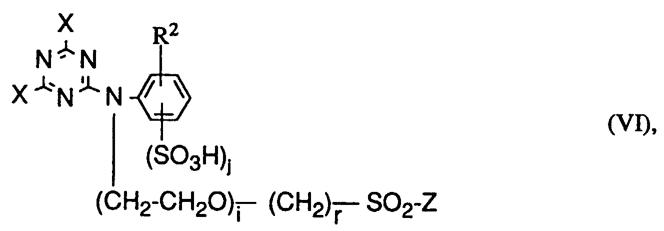 worin
   X, i, R², j, r und Z die oben angegebene Bedeutung haben,
   und weitere Kondensation mit n-Molen der Verbindungen der Formel (VI) mit den Farbstoffen der Formel (II),
   oder
c) durch Kondensation geeigneter Vorprodukte mit den Trihalogentriazinen (III) und den Komponenten der Formel (V) bzw. durch Kondensation geeigneter Vorprodukte mit den Primärkondensationsprodukten der Formel (VI) und anschließender Farbstoffsynthese.

Die Erfindung betrifft weiterhin ein Verfahren zur Herstellung von Verbindungen (V) sowie Zwischenprodukte (V) und zu ihrer Herstellung nützliche Zwischenprodukte der allgemeinen Formel 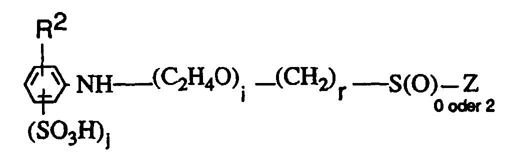 einschließlich ihrer Salze und ihrer Umsetzungsprodukte mit Schutzgruppen,
worin R², Z, j und i die unter Formel (I) angegebene Bedeutung haben und r = 3.

Besonders bevorzugt sind Verbindungen, worin bedeuten
- R²: H
- j,i,: 0
- r: 2.

Die Verbindungen der Formel (V) lassen sich herstellen durch Sulfatieren von Verbindungen der Formel 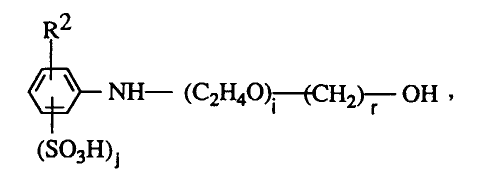 anschließende Umsetzung mit Mercaptoethanol unter Erhalt von 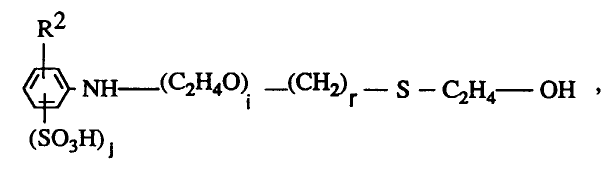 gegebenenfalls Blockierung der NH-Gruppe mit einer Schutzgrupppe, insbesondere Acetyl, und Oxidation, insbesondere mit H₂O₂ zu 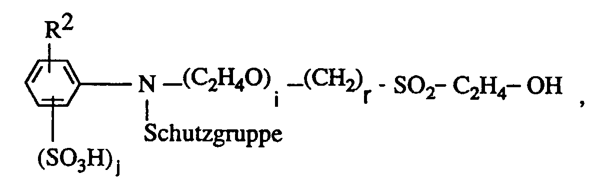 gegebenenfalls Abspalten der Schutzgruppe unter Erhalt von 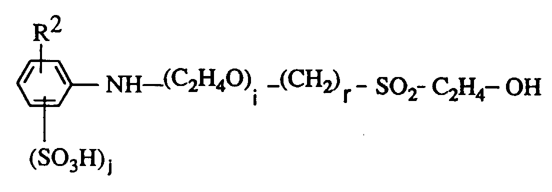 und gegebenenfalls übliche Überführung des Restes SO₂C₂H₄-OH in die Vinylsulfonylgruppe oder Überführung der -CH₂-CH₂-OH Gruppe in die übrigen unter Z aufgeführten abspaltbaren Gruppen.

Die Kondensationen der Ausgangskomponenten mit den Trihalogentriazinen erfolgen unabhängig von der Reihenfolge in wäßrigen oder organisch-wäßrigen Medien in Anwesenheit von säurebindenden Mitteln. In Abhängigkeit von der Natur der Ausgangskomponenten erfolgt dabei die erste Stufe der Kondensation in pH-Bereichen von 2 bis 8, vorzugsweise 3 bis 7, und bei Temperaturen von 0 bis 40°C, vorzugsweise 0 bis 25°C. Der Austausch des zweiten Halogenatoms des Triazins vollzieht sich im pH-Bereich von 4 bis 10, vorzugsweise 5 bis 9, und im Temperaturbereich von 0 bis 60°C, vorzugsweise 0 bis 30°C.

Säurebindende Mittel sind beispielsweise Carbonate, Hydroxide oder Phosphate wie Natriumcarbonat, Natriumhydrogencarbonat, verdünnte Natronlauge, Di- oder Trinatriumphosphat oder Natriumfluorid.

Soll die Kondensation bzw. die Farbstoffsynthese direkt zu einer Farbstofflösung bzw. zu einer flüssigen Farbstoffpräparation führen, kann die Verwendung von Lithiumcarbonaten oder Lithiumhydroxid vorteilhaft sein, gegebenenfalls zusammen mit Lösungsvermittlern und/oder stabilisierenden Puffersystemen. Andere Umwandlungsreaktionen der Farbstoffe oder deren Vorprodukte wie Metallisierungsreaktionen, Sulfierungen oder Einführung von Acylaminogruppierungen können im allgemeinen in beliebigen Stufen der Farbstoffsynthesen vorgenommen werden.

Besonders wertvolle Farbstoffe dieser Reihe sind wasserlösliche Azofarbstoffe, und insbesondere solche, die Sulfonsäure- und/oder Carbonsäuregruppen aufweisen. Die Farbstoffe können sowohl metallfrei als auch metallhaltig sein, wobei unter den Metallkomplexen die Kupfer-, Nickel-, Chrom- und Kobalt-Komplexe bevorzugtes Interesse besitzen.

Geeignete Farbstoffreste D bzw. die den Farbstoffen der Formel (I) zugrundeliegenden aminogruppenhaltigen Farbstoffe sind in der Literatur in sehr großer Zahl beschrieben. Beispielhaft seien hier erwähnt:
EP-A 54 515, EP-A 69 703, EP-A 70 807, EP-A 497 174, DE-A 3 222 726, DE-A 2 650 555, DE-A 3 023 855, DE-A 2 847 938, DE-A 2 817 780, GB-A 2 057 479, DE-A 2 916 715, DE-A 2 814 206, DE-A 3 019 936, EP-A 45 488 sowie Venkataraman: The Chemistry of Synthetic Dyes, BD. VI, Kapitel II, S. 211-325, New York, London; 1972.

Bevorzugte Reste eines organischen Farbstoffes entsprechen beispielsweise folgenden Gruppen
Di-N=N-(M-N=N-)_{0 oder 1} K-
-Di-N=N-(M-N=N-)_{0 oder 1} K
-Di-N=N-(M-N=N-)_{0 oder 1} K-
- Di: für den Rest einer gegebenenfalls durch in der Azochemie übliche Substituenten, insbesondere Hydroxy-, Methyl-, Ethyl-, Methoxy- oder Ethoxygruppen, gegebenenfalls substituierte Alkanoylaminogruppen mit 2-4 C-Atomen, gegebenenfalls substituierte Benzoylaminogruppen oder Halogenatome sowie SO₂-Z substituierte Diazokomponente der Benzol- oder Naphthalinreihe,
- K: für den Rest einer gegebenenfalls durch in der Azochemie übliche Substituenten, insbesondere Hydroxy-, Amino-, Methyl-, Ethyl-, Methoxy- oder Ethoxygruppen, gegebenenfalls substituierte Alkanoylaminogruppen mit 2-4 C-Atomen, gegebenenfalls substituierte Benzoylaminogruppen oder Halogenatome substituierte Kupplungskomponente der Benzol-, Naphtalin- oder Ketomethylenreihe,
- M: für den Rest einer gegebenenfalls durch in der Azochemie übliche Substituenten, insbesondere Hydroxy-, Methyl-, Ethyl-, Methoxy- oder Ethoxygruppen, gegebenenfalls substituierte Alkanoylaminogruppen mit 2-4 C-Atomen, gegebenenfalls substituierte Benzolaminogruppen oder Halogenatome substituierte Mittelkomponente der Benzol- oder Naphthalinreihe steht, und
- Di, M und K: zusammen mindestens zwei Sulfonsäuregruppen, vorzugsweise drei bis vier Sulfonsäuregruppen, enthalten.

Wichtige Azofarbstoffe sind beispielsweise solche der Benzol-azo-naphthalinreihe, der Bis-(Benzolazo)-naphthalinreihe, der Benzol-azo-5-pyrazolonreihe, der Benzol-azo-benzolreihe, der Naphthalin-azo-benzolreihe, der Benzol-azo-aminonaphthalinreihe, der Naphthalin-azo-naphthalinreihe, der Naphthalin-azo-pyrazolon-5-Reihe, der Benzol-azo-pyridonreihe, der Benzol-azo-aminopyridinreihe, der Naphthalin-azo-pyridonreihe, der Naphthalin-azo-amino-pyridinreihe, und der Stilben-azo-benzolreihe, wobei auch hier die sulfonsäuregruppenhaltigen Farbstoffe bevorzugt sind. Im Falle von Metallkomplexazofarbstoffen befinden sich die metallkomplexgebundenen Gruppen vorzugsweise in den o-Stellungen zur Azogruppe, z.B. in Form von o,o'-Dihydroxy-, o-Hydroxy-o'-carboxy, o-Carboxy-o'-amino- und o-Hydroxy-o'-amino-azogruppierungen.

Bevorzugt sind Reaktivfarbstoffe der Formel 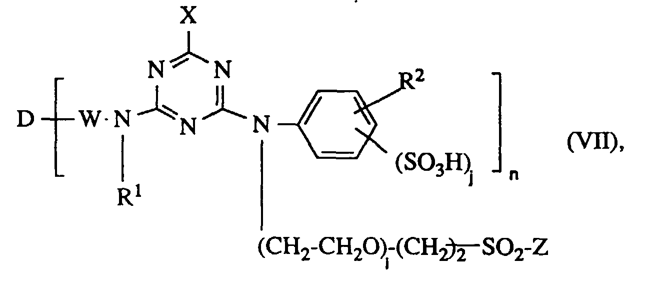 worin
- D: Rest eines organischen Farbstoffes
aus der Monoazo-, Polyazo-, Metallkomplexazoreihe ist, der keine weiteren faserreaktiven Gruppen enthält,
- R¹, R²: unabhängig voneinander H, CH₃ oder C₂H₅ sind,
- W: direkte Bindung oder Brückenglied, bevorzugt Alkylen bzw. direkte Bindung,
- i: 0 oder 1,
- j: 0 oder 1,
- X: F oder Cl,
- Z: CH₂-CH₂-OSO₃H, CH=CH₂,
- n: 1 oder 2 ist.

Besonders bevorzugt sind Reaktivfarbstoffe der Formel 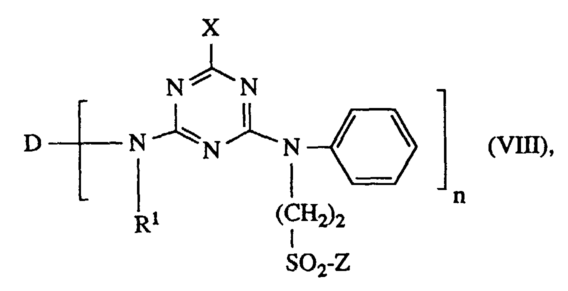 worin
- D: Rest eines organischen Farbstoffes
aus der Monoazo-, Polyazo, Metallkomplexazoreihe ist, der keine weiteren faserreaktiven Gruppen enthält,
- X: F,
- Z: -CH₂-CH₂-OSO₃H,
- R¹: H,
- n: 1 ist.

Bevorzugte Farbstoffe sind die der folgenden Formeln (1) bis (42), worin generell B einen Rest der Formel 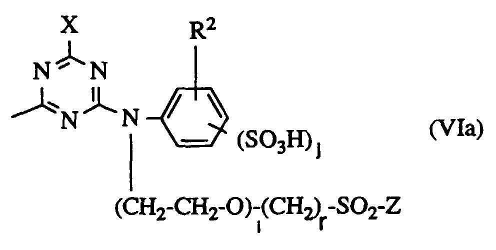 beinhaltet und X, i, R², j, r und Z die unter Formel (I) angegebene Bedeutung haben:

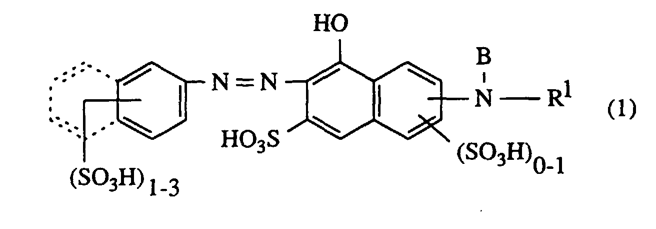

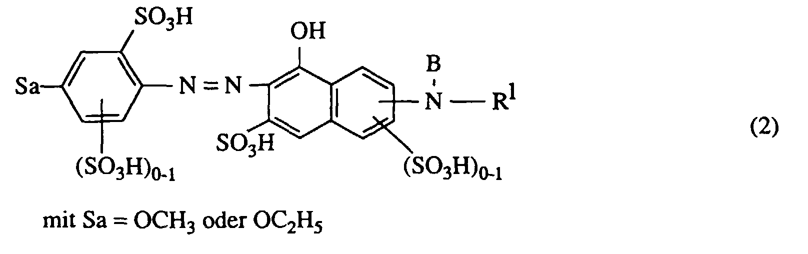

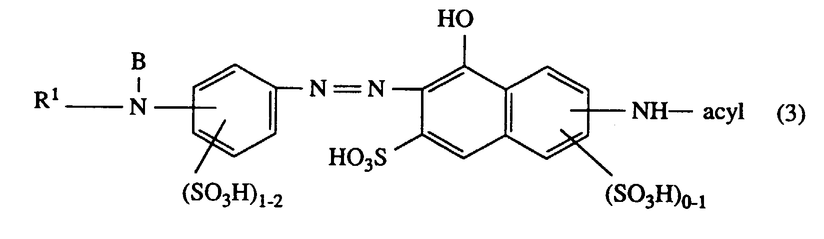

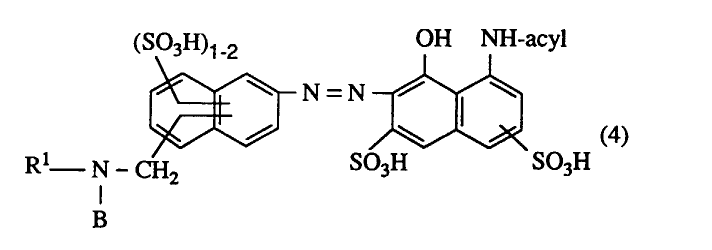

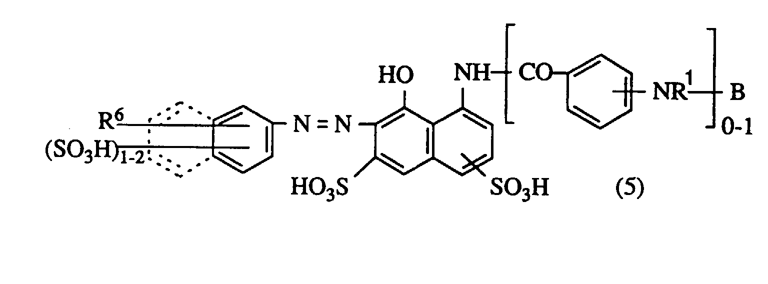

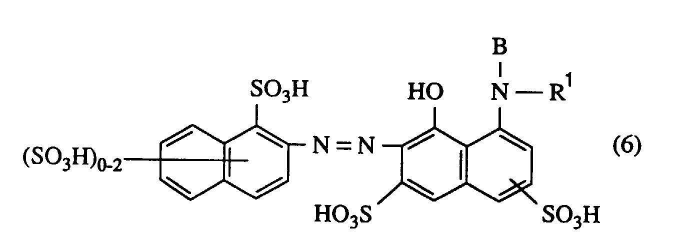

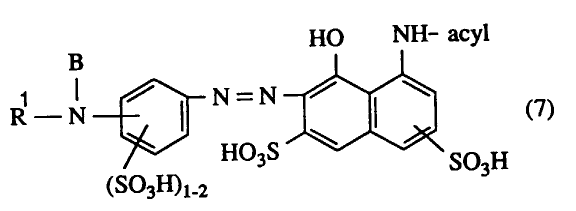

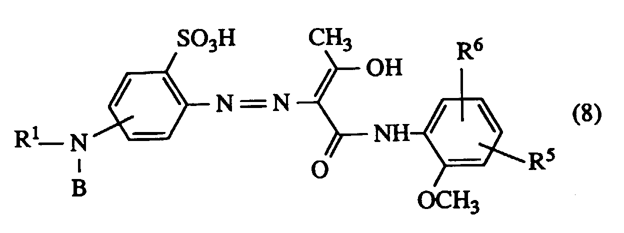

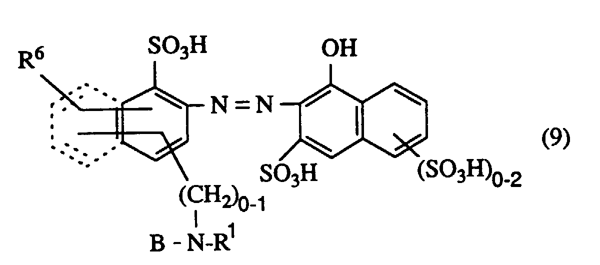

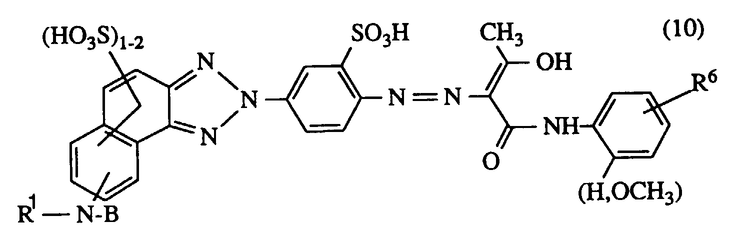

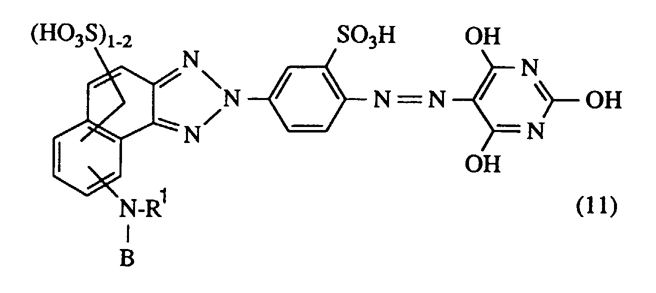

Metallkomplexe von Farbstoffen der Formeln (12) bis (42):

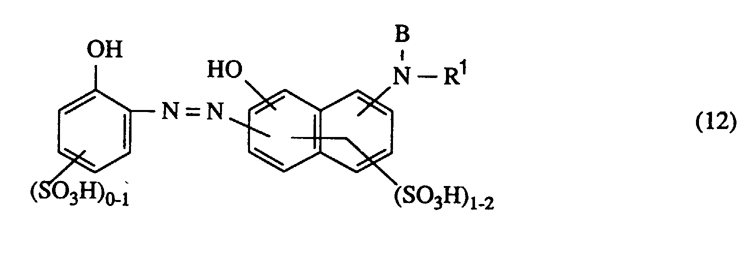

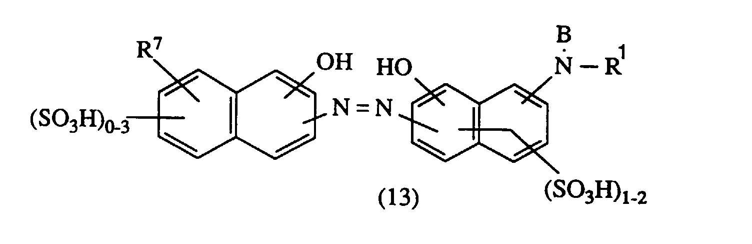

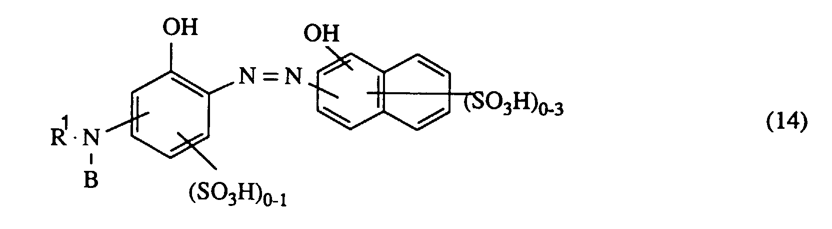

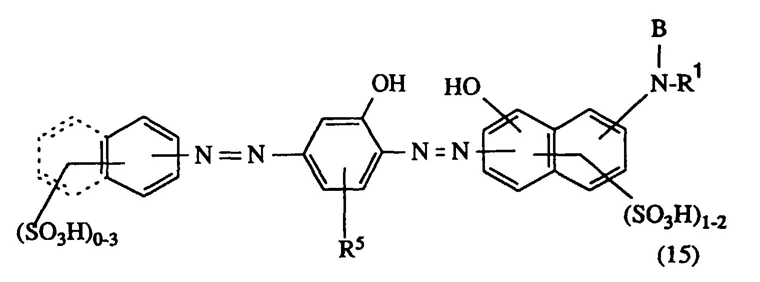

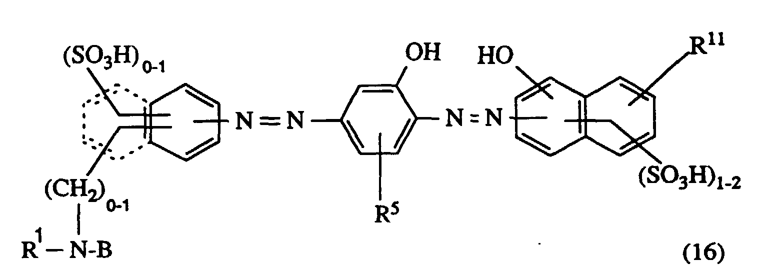

Als Metallatom sind Cu (1:1-Komplex) oder Cr und Co (1:2-Komplex) bevorzugt. Cr- und Co-Komplexe können die Azoverbindung der oben angegebenen Formel einmal oder zweimal enthalten, d.h., sie können symmetrisch oder mit beliebigen anderen Ligandengruppen unsymmetrisch aufgebaut sein. 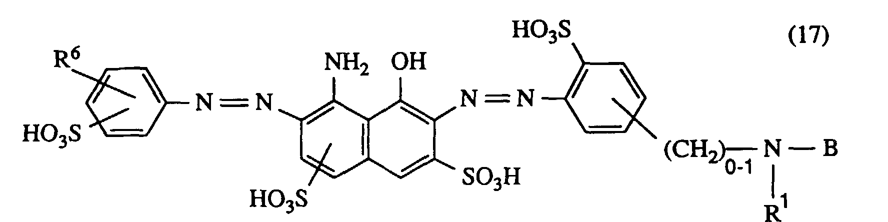 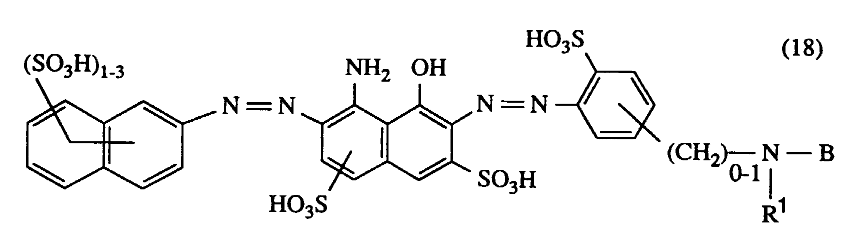 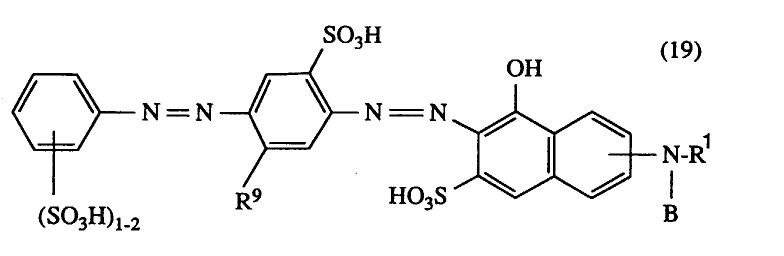 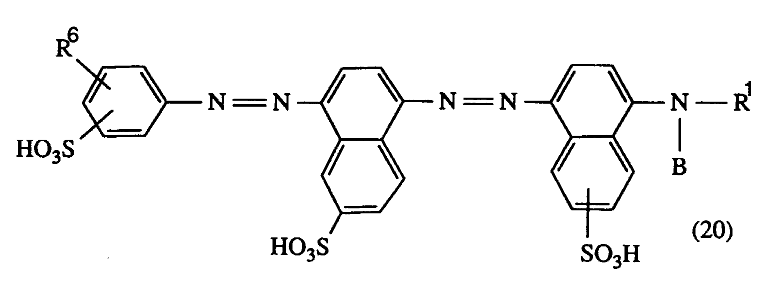 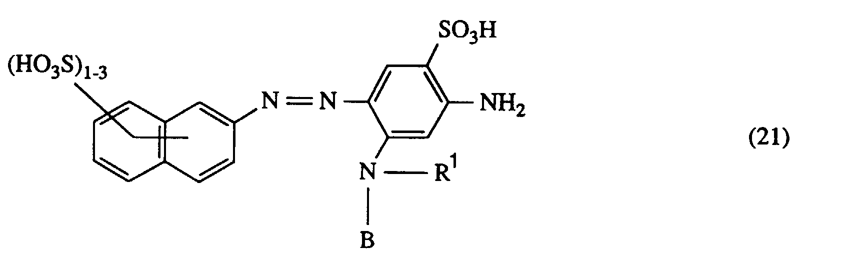 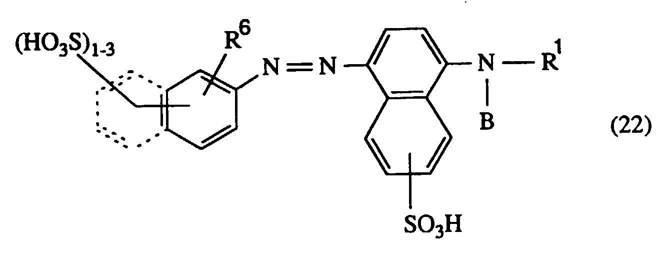 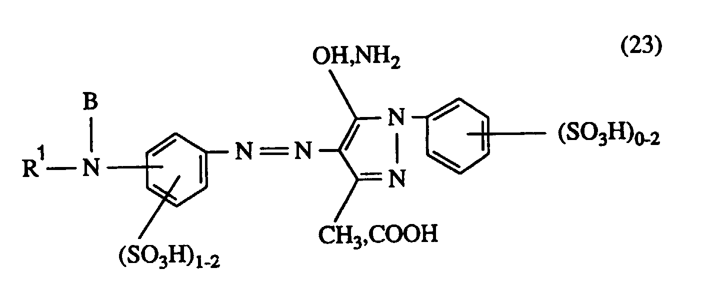 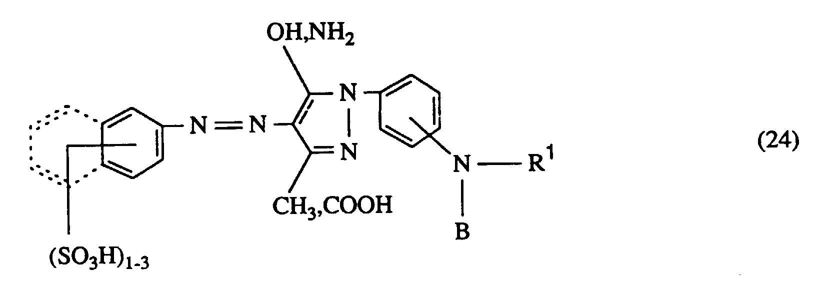 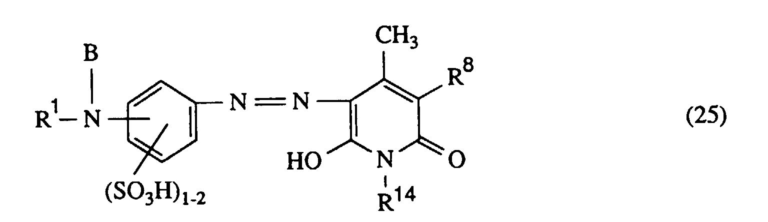 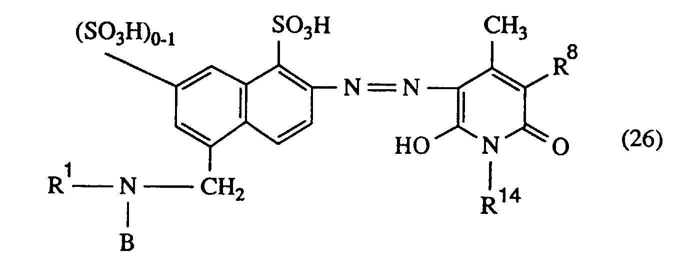 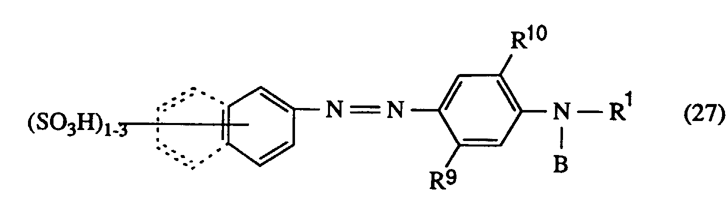 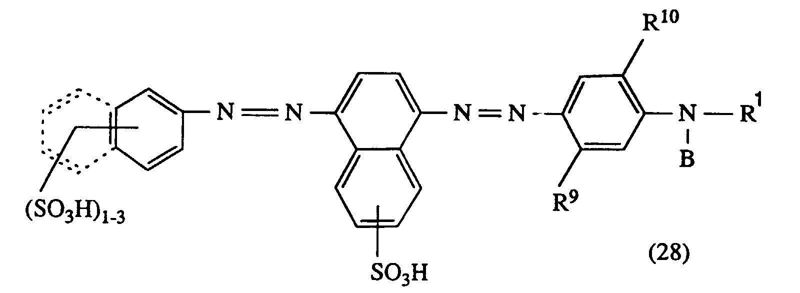 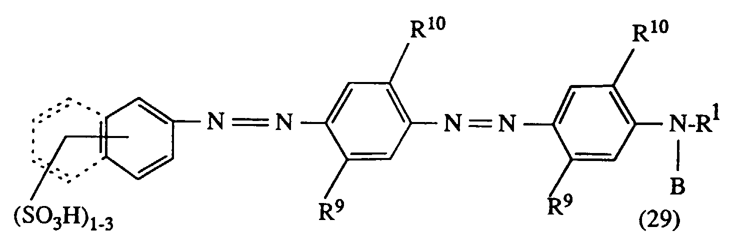 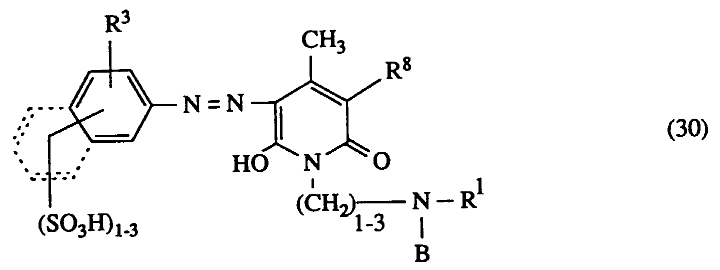 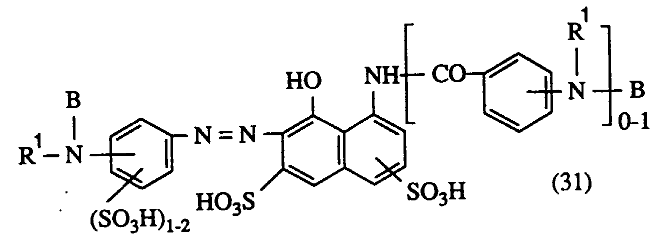 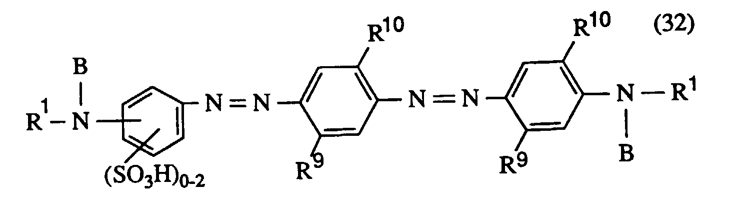 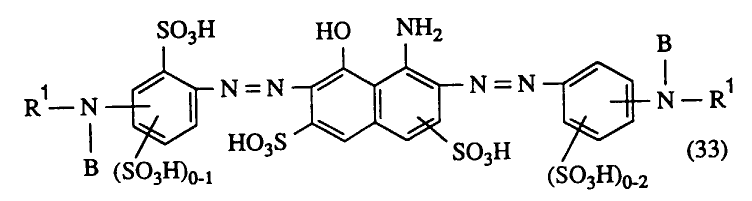 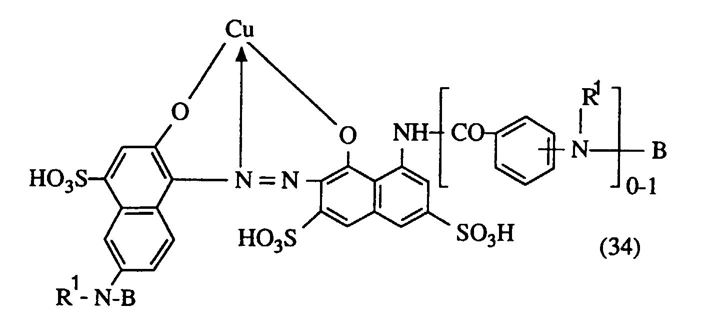 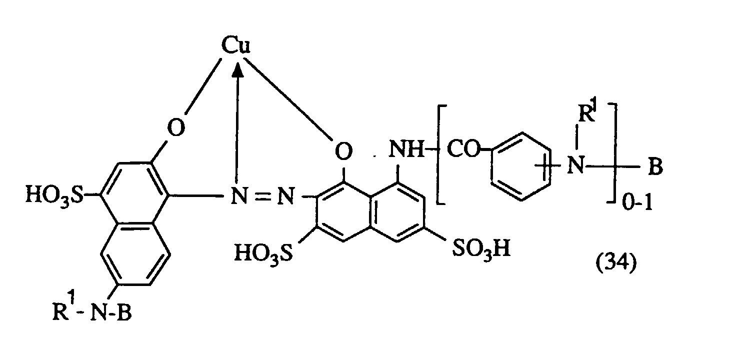 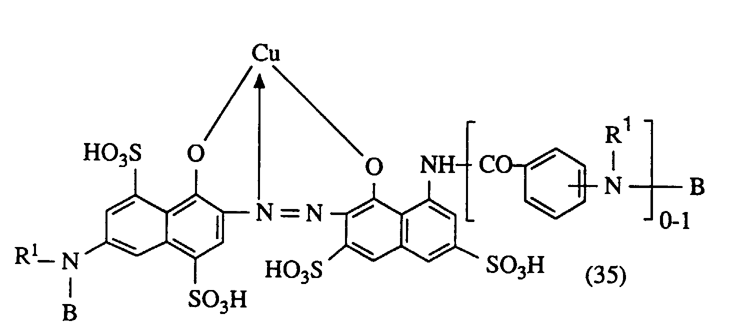 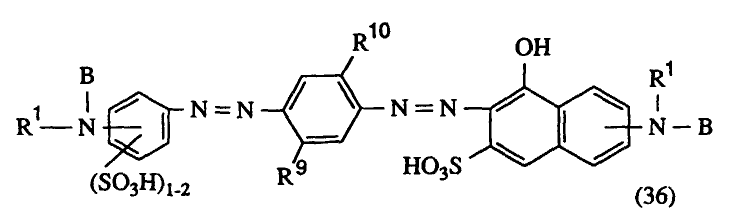 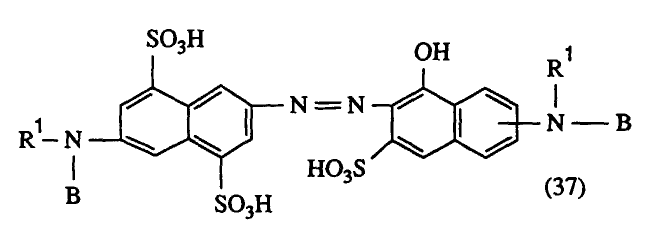 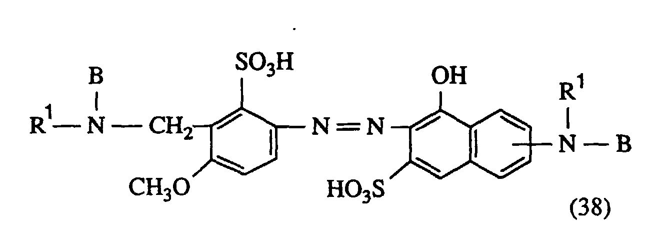 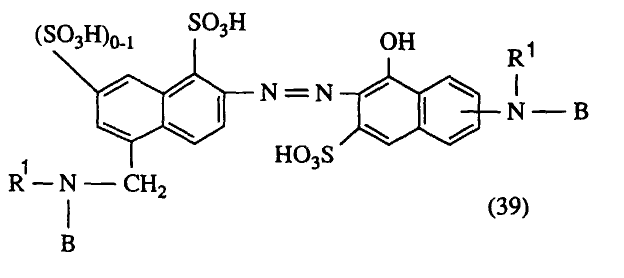 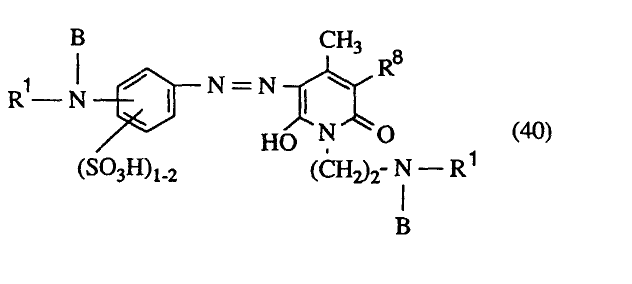 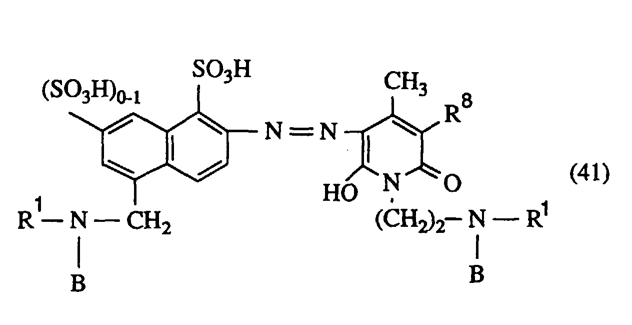 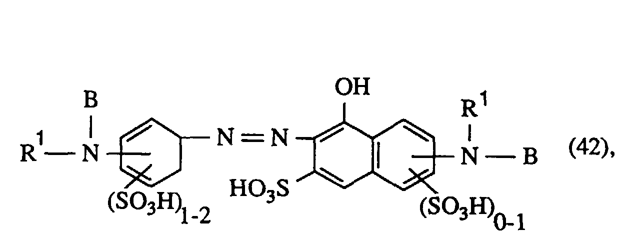 worin
Acyl z.B. Acetyl oder gegebenenfalls substituiertes Benzoyl ist,
- R¹⁴=: H, C₁-C₂ Alkyl, gegebenenfalls substituiert durch SO₃H, NH₂,
- R¹ =: H, CH₃ oder C₂H₅,
- R³=: H oder Sulfo,
- R⁵ =: H, CH₃, OCH₃ oder Cl,
- R⁶ =: H, C₁-C₄-Alkyl, C₁-C₄-Alkoxy, Cl, Br, COOH, SO₃H.
- R⁷ =: H, OH, NH₂, NHCOCH₃, NHCOPh, Cl, C₁-C₄-Alkoxy oder C₁-C₄-Alkyl ist,
- R⁸ =: H, SO₃H, CH₂SO₃H, Cl, C₁-C₄-Alkylsulfonyl, CN, Carbonamid, insbesondere CONH₂,
- R⁹ =: H, C₁-C₄-Alkyl, C₁-C₄-Alkoxy, Cl, Br, Acylamino, insbesondere C₁-C₄-Alkylcarbonylamino oder Arylcarbonylamino wie gegebenenfalls substituiertes Phenylcarbonylamino, C₁-C₄-Alkylsulfonylamino, Aminocarbonylamino, C₁-C₄-Alkylsulfonylamino, Arylsulfonylamino,
- R¹⁰=: H, C₁-C₄-Alkyl, C₁-C₄-Alkoxy, OH, SO₃H.

Die durch Strichelungen angedeuteten kondensierten Ringe stehen für alternativ mögliche Naphthalinsysteme.

Ganz besonders bevorzugt sind Reaktivfarbstoffe der Formel (I),
worin
X = Cl,
i = 0,
j = 0 oder 1,
Z = -CH₂-CH₂-OSO₃H oder -CH=CH₂,
R² = H.

Ebenso ganz besonders bevorzugt sind Reaktivfarbstoffe der Formel (I),
worin
X =F,
i =0,
j =0,
Z =-CH₂-CH₂-OSO₃H, -CH=CH₂,
R² =H
und

Reaktivfarbstoffe der Formel (I)
worin
Z =-CH₂-CH₂-OSO₃H ist.

Bevorzugt sind weiterhin Reaktivfarbstoffe der Formel (I),
worin
der Rest D, vorzugsweise ein Rest eines organischen Farbstoffes aus der Monoazo- oder Polyazoreihe, ein- oder zweimal mit der Gruppe 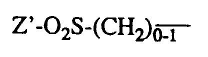 substituiert ist und
worin
- Z': -CH=CH₂, -CH₂-CH₂-OSO₃H, -CH₂-CH₂-Cl, -CH₂-CH₂-Br, -CH₂-CH₂-S₂O₃H, -CH₂-CH₂-O-CO-CH₃, -CH₂-CH₂-OPO₃H₂ oder -CH₂-CH₂-OH bedeutet.

Besonders bevorzugt sind Reaktivfarbstoffe der Formel (I),
worin
der Rest D, vorzugsweise ein Rest eines organischen Farbstoffes aus der Monoazo- oder Polyazoreihe, ein- oder zweimal mit der Gruppe 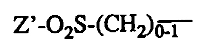 substituiert ist,
- Z': die obige Bedeutung hat und n = 1 ist.

Besonders bevorzugt sind weiterhin Reaktivfarbstoffe der Formel (I),
in der
- n: = 1,
- W: = direkte Bindung und
- D: ein Rest der allgemeinen Formel (IX) ist, 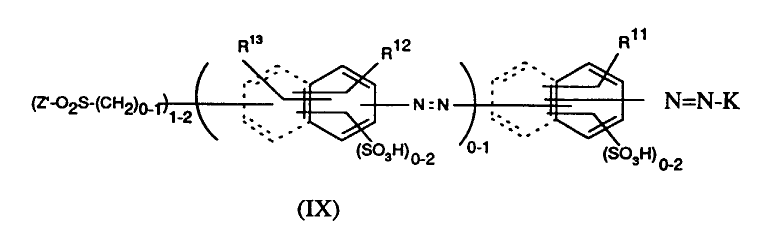
worin
- R¹¹: H, C₁-C₄-Alkyl, C₁-C₄-Alkoxy, Cl, Br, Acylamino, insbesondere C₁-C₄-Alkylcarbonylamino oder Arylcarbonylamino, wie gegebenenfalls substituiertes Phenylcarbonylamino, C₁-C₄-Alkylsulfonylamino, Aminocarbonylamino, C₁-C₄-Alkylsulfonylamino, Arylsulfonylamino,
- R¹²: H, C₁-C₄-Alkyl, Cl, Br, C₁-C₄-Alkoxy oder COOH,
- R¹³: H, C₁-C₄-Alkyl, C₁-C₄-Alkoxy, SO₃H, Cl oder Br,
- K: ein bivalenter Rest der allgemeinen Formeln (Xa) - (Xd) 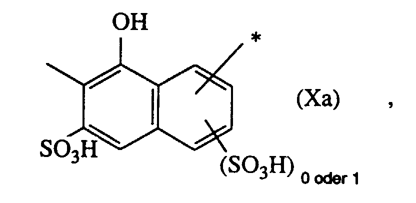 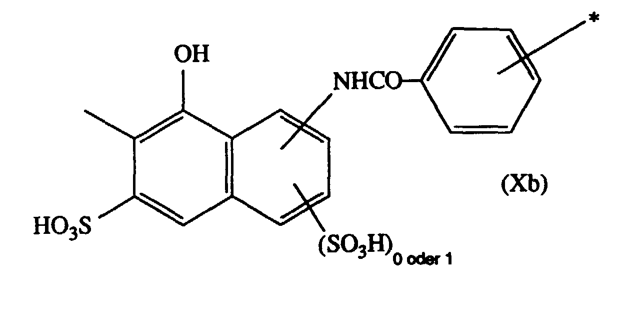 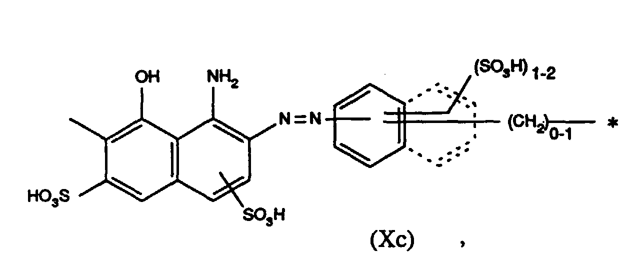 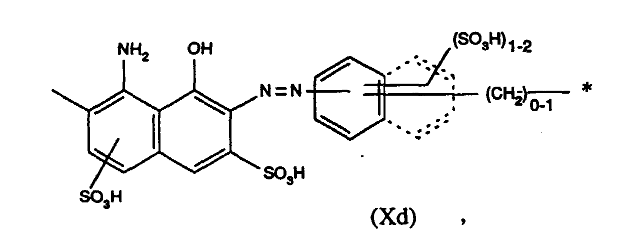
bedeutet,
wobei die mit * gekennzeichneten Bindungen an die Gruppe -NR¹-B gebunden sind. B hat die Bedeutung von Formel (VIa).

Besonders bevorzugte Reste für Z und Z' sind unabhängig voneinander -CH=CH₂ oder -C₂H₄-OSO₃H.

Geeignete den Resten D der Formel (IX) zugrundeliegende Diazokomponenten für die Farbstoffe der Formeln (I) sind z.B.:
Anilin-4-β-sulfatoethylsulfon,
Anilin-4-β-thiosulfatoethylsulfon,
Anilin-4-vinylsulfon,
Anilin-3-β-sulfatoethylsulfon,
Anilin-3-vinylsulfon,
2-Methoxyanilin-5-β-sulfatoethylsulfon,
2-Methoxyanilin-5-β-thiosulfatoethylsulfon,
2-Methoxyanilin-5-vinylsulfon,
4-Methoxyanilin-3-β-sulfatoethylsulfon,
4-Methoxyanilin-3-vinylsulfon,
2,5-Dimethoxyanilin-4-β-sulfatoethylsulfon,
2,5-Dimethoxyanilin-4-vinylsulfon,
2-Methoxy-5-methylanilin-4-β-sulfatoethylsulfon,
Anilin-2-β-sulfatoethylsulfon,
3-(3- oder 4-Aminobenzoyl)-aminophenyl-β-sulfatoethylsulfon,
2-Methoxy-5-methylanilin-4-vinylsulfon,
6-Carboxyanilin-3-β-sulfatoethylsulfon,
6-Carboxyanilin-3-vinylsulfon,
2-Sulfoanilin-4-β-sulfatoethylsulfon,
2-Sulfoanilin-4-vinylsulfon,
2,4-Disulfoanilin-5-vinylsulfon,
2-Naphthylamin-8-β-sulfatoethylsulfon,
2-Naphthylamin-6-β-sulfatoethylsulfon,
1-Sulfo-2-naphthylamin-6-β-sulfatoethylsulfon,
1-Naphthylamin-4-β-sulfatoethylsulfon,
1-Sulfo-2-naphthylamin-5-β-sulfatoethylsulfon,
6-Sulfo-2-naphthylamin-8-β-sulfatoethylsulfon,
2-Amino-3-sulfo-naphthalin-6,8-bis-(β-sulfatoethylsulfon),
1-Naphthylamin-5-β-sulfatoethylsulfon,
2-Naphthylamin-5-β-sulfatoethylsulfon,
2-Naphthylamin-8-β-sulfatoethylsulfon,
8-Sulfo-2-naphthylamin-6-β-sulfatoethylsulfon,
4-Aminobenzyl-β-sulfatoethylsulfon,
3-Aminobenzyl-β-sulfatoethylsulfon,
4-Aminobenzylvinylsulfon,
3-Aminobenzylvinylsulfon,
3-Amino-4-sulfobenzyl-β-sulfatoethylsulfon,
4-Amino-3-sulfobenzylvinylsulfon,
2'-(β-Sulfatoethylsulfonyl)-3-sulfo-4-aminoazobenzol,
3'-(β-Sulfatoethylsulfonyl)-3-sulfo-4-aminoazobenzol,
4'-Methoxy-3'-(β-sulfatoethylsulfonyl)-3-sulfo-4-aminoazobenzol,
4'-Vinylsulfonyl-2',3-disulfo-4-aminoazobenzol,
2'-(β-Sulfatoethylsulfonyl)-6-methyl-3-sulfo-4-aminoazobenzol,
3'-(β-Sulfatoethylsulfonyl)-6-methyl-3-sulfo-4-aminoazobenzol,
4'-(β-Sulfatoethylsulfonyl)-6-methyl-3-sulfo-4-aminoazobenzol,
4'-(β-Sulfatoethylsulfonyl)-2,6-dimethyl-3-sulfo-4-aminoazobenzol,
3'-(β-Sulfatoethylsulfonyl)-6-methoxy-3-sulfo-4-aminoazobenzol,
3',4'-Bis-(β-sulfatoethylsulfonyl)-6-methoxy-3-sulfo-4-aminoazobenzol,
4'-(β-Sulfatoethylsulfonyl)-6-methoxy-3-sulfo-4-aminoazobenzol,
4'-(β-Sulfatoethylsulfonyl)-2-methyl-5-methoxy-3-sulfo-4-aminoazobenzol,
4'-(β-Sulfatoethylsulfonyl)-2,5-dimethoxy-3-sulfo-4-aminoazobenzol,
3'-(β-Sulfatoethylsulfonyl)-2,5-dimethoxy-3-sulfo-4-aminoazobenzol,
2-(4'-Amino-3'-sulfophenylazo)-6-(β-sulfatoethylsulfonyl)-naphthalin,
2-(4'-Amino-6'-methyl-3'-sulfophenylazo)-1-sulfo-6-(β-sulfatoethylsulfonyl)-naphthalin,
2-(4'-Amino-6'-methyl-3'-sulfophenylazo)-6-(β-sulfatoethylsulfonyl)-naphthalin,
2-(4'-Amino-3'-sulfophenylazo)-8-sulfo-6-(β-sulfatoethylsulfonyl)-naphthalin,
2-(4'-Amino-6'-methyl-3'-sulfophenylazo)-1,7-disulfo-5-(β-sulfatoethylsulfonyl)-naphthalin.

Geeignete, den Resten K zugrundeliegende Kupplungskomponenten H-K-NHR¹ sind beispielsweise: 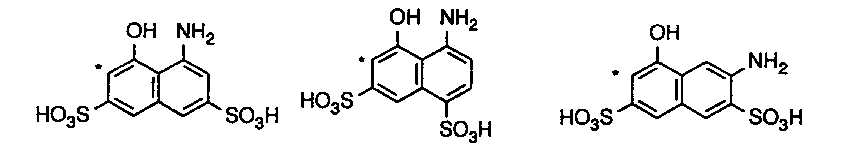 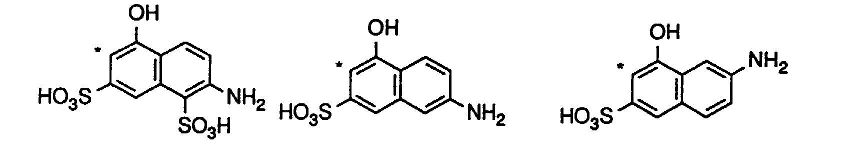 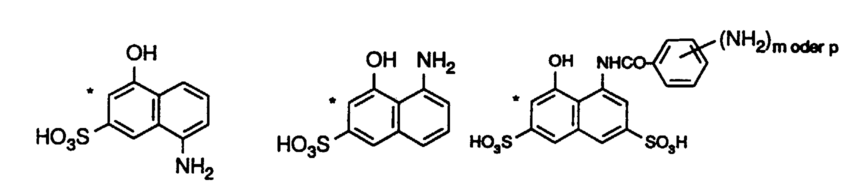 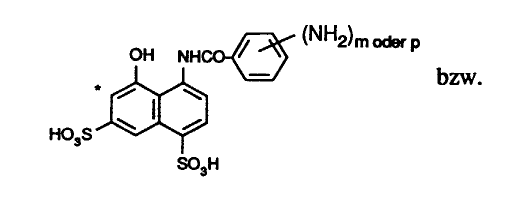 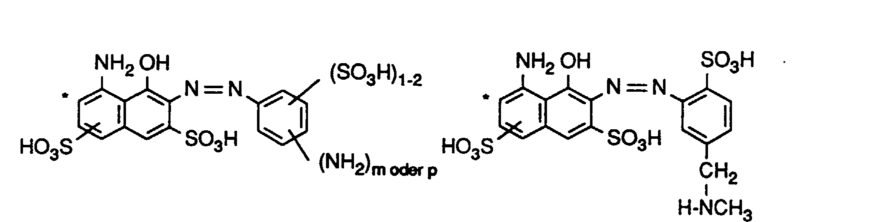 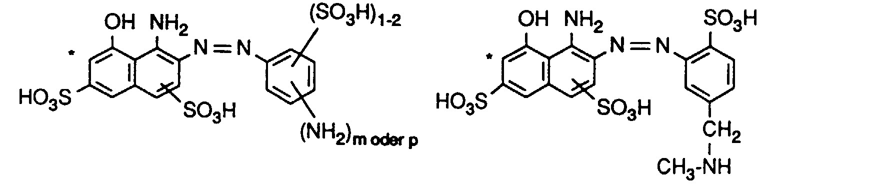 oder 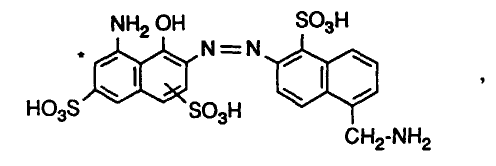 wobei * die Kupplungsstelle markiert und m bzw. p für die meta- bzw. para-Position der Aminogruppe zur Azogruppe steht.

Bevorzugt sind weiterhin Reaktivfarbstoffe der Formel (I) mit einem Farbstoffrest D der Formel (IX),
worin
- R¹, R²: = unabhängig voneinander H, CH₃ oder C₂H₅,
- j: = 0 oder 1,
- X: = F oder Cl und
- Z, Z': = unabhängig voneinander CH₂-CH₂-OSO₃H oder CH=CH₂ bedeuten.

Besonders bevorzugt sind Reaktivfarbstoffe der Formel (I) mit einem Farbstoffrest D der Formel (IX)
worin
R¹, R²= H,
i= 0,
j= 0,
r= 2,
Z',Z= -CH₂CH₂OSO₃H
bedeutet.

Bevorzugte Farbstoffe im Sinne der Formel (I) mit einem Farbstoffrest D der Formel (IX) sind die der folgenden Formeln (43) bis (52), worin generell B einen Rest der Formel 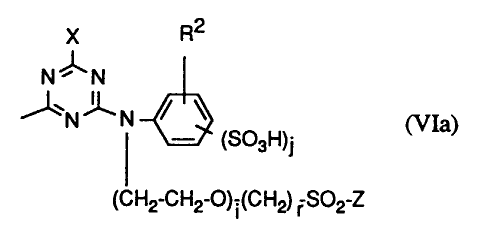 beinhaltet und X, i, R², j, r und Z die oben angegebene Bedeutung haben: 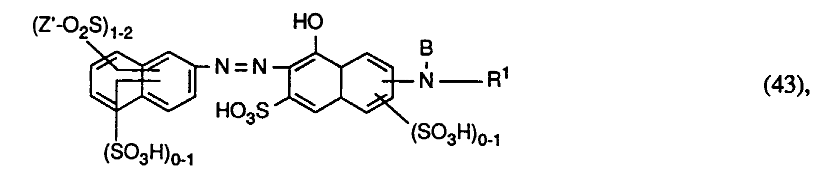 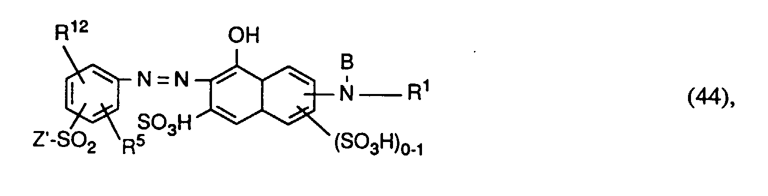 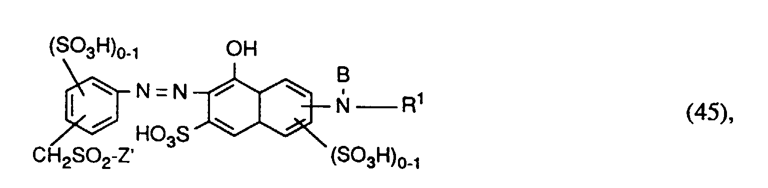 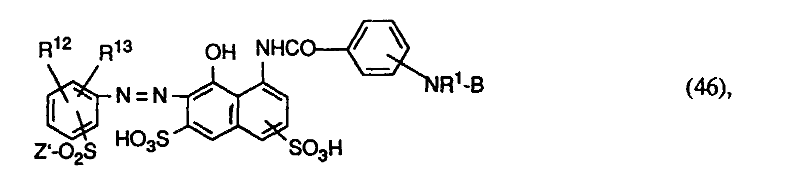 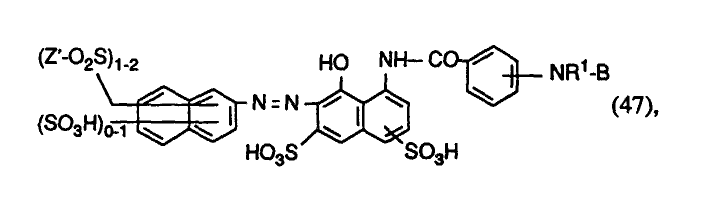 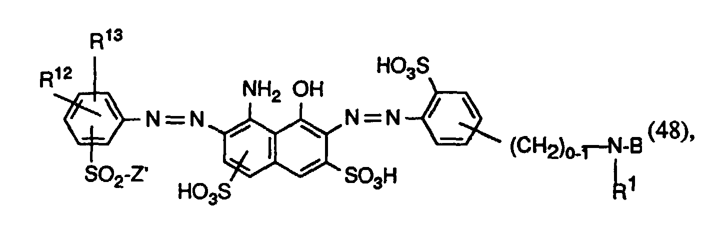 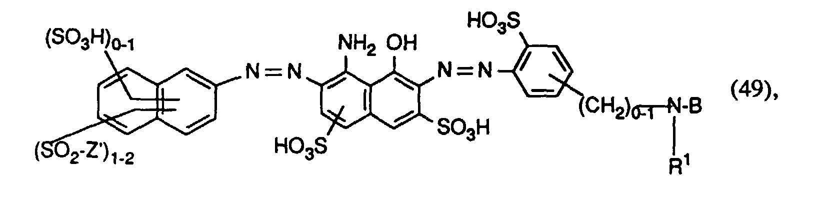 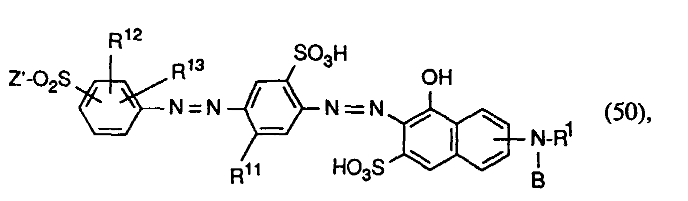 worin
- R¹ =: H, CH₃ oder C₂H₅,
- R¹¹=: H, C₁-C₄-Alkyl, C₁-C₄-Alkoxy, Cl, Br, Acylamino, insbesondere C₁-C₄-Alkylcarbonylamino oder Arylcarbonylamino wie gegebenenfalls substituiertes Phenylcarbonylamino, C₁-C₄-Alkylsulfonylamino, Aminocarbonylamino, C₁-C₄-Alkylsulfonylamino, Arylsulfonylamino,
- R¹²=: H, C₁-C₄-Alkyl, C₁-C₄-Alkoxy, Cl, Br oder COOH,
- R¹³=: H, C₁-C₄-Alkyl, C₁-C₄-Alkoxy, Cl, Br, SO₃H.

Die durch Strichelungen angedeuteten kondensierten Ringe stehen für alternativ mögliche Naphthalinsysteme.

Die Reaktivfarbstoffe der Formel (I) eignen sich zum Färben und Bedrucken der verschiedensten Substrate wie Seide, Leder, Wolle, synthetischen Polyamidfasern, insbesondere aber cellulosehaltiger Materialien faseriger Struktur wie Leinen, Zellstoff, regenerierte Zellulose und vor allem Baumwolle. Sie eignen sich sowohl für das Ausziehverfahren als auch zum Färben nach den üblichen Foulardfärbeverfahren, wonach die Ware mit wäßrigen und gegebenenfalls auch salzhaltigen Farbstofflösungen imprägniert wird, und die Farbstoffe nach einer Alkalibehandlung oder in Gegenwart von Alkali, gegebenenfalls unter Wärmeeinwirkung, fixiert werden.

Die Reaktivfarbstoffe der Formel (I) zeichnen sich durch hohe Reaktivität und ausgezeichnetes Fixiervermögen aus. Aufgrund ihrer Bi- bzw. Polyfunktionalität ergeben sie auch aus langer Flotte hohe Fixierausbeuten. Sie sind charakterisiert durch relative Unabhängigkeit der Ergiebigkeit von der Färbetemperatur und können daher nach dem Ausziehverfahren bei niedrigen bis mittleren Färbetemperaturen eingesetzt werden. Beim Klotz-Dämpf-Verfahren erfordern sie nur kurze Dämpfzeiten. Sie ergeben Färbungen hoher Farbstärke mit guten Licht- und Naßechtheiten.

### Beispiel 1

A) 0,15 Mol der Verbindung der Formel (Herstellung siehe Beispiel 115-118) 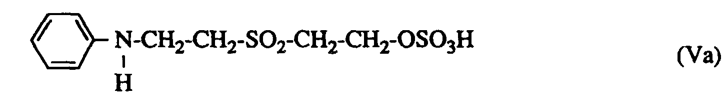 wurden in 100 Teilen Wasser und 100 Teilen Eis verrührt und neutral gelöst. Innerhalb von 10 Minuten tropfte man bei O°C und pH 4-4,5 0,165 Mol 2,4,6-Trifluor-1,3,5-triazin zu. Man erhält ca. 250 Teile einer wässrigen Kondensationslösung mit folgender Struktur: 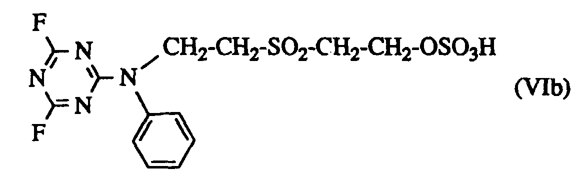
B) 0,1 Mol 1-Amino-8-hydroxy-3,6-naphthalindisulfonsäure wurden in 150 Teilen Wasser suspendiert und mit Lithiumhydroxylösung neutral gelöst. Man gab 0,1 Mol der obigen Kondensationslösung hinzu. Mit Lithiumcarbonat wurde bei 20°C ein pH-Wert von 4-4,5 gehalten. Nach 8 Stunden war die Reaktion beendet. Es lag eine Lösung vor.
C) 0,1 Mol 2-Amino-1-naphthalinsulfonsäure wurden in geeigneter Weise diazotiert und bei pH 7,5-8,5 auf das H-Säure-Kondensationsprodukt gekuppelt Der Farbstoff wurde durch Zugabe von Kaliumchlorid ausgesalzen und isoliert. Nach Trocknung erhielt man ca. 35 g eines salzhaltigen Farbstoffkupplers, dem die Struktur 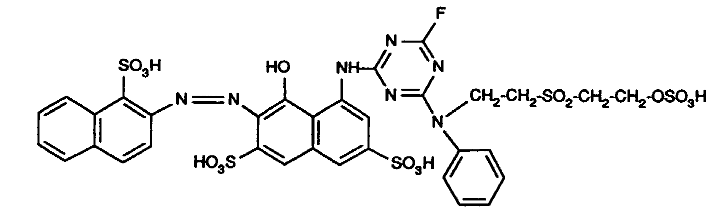 zukommt (λₘₐₓ = 521 und 544 nm (H₂O)) und das Baumwolle nach den für Reaktivfarbstoffe üblichen Färbe- oder Druckverfahren in roten Farbtönen färbt.

### Beispiel 2

0,1 Mol 2-Amino-1,5-naphthalindisulfonsäure wurden in 200 Teilen Wasser und 100 Teilen Eis bei 0°C suspendiert. Man gab 28 Teile konzentrierte Salzsäure zu. Innerhalb von 15 Minuten tropfte man eine Lösung von 7 Teilen Natriumnitrit in 70 Teilen Wasser zu. Nach 30-minütigem Nachrühren war die Diazotierung beendet. Es resultierte eine hellgelbe Suspension. Der Nitritüberschuß wurde mit Amidosulfonsäure zerstört.

Diese Suspension wurde nun über einen Zeitraum von 15-20 Minuten zu 0,1 Mol des H-Säure-Kondensationsproduktes aus Beispiel 1 B zudosiert. Gekuppelt wurde bei 20°C und einem pH-Wert von 7-8. Der Farbstoff wurde durch Zugabe von Ethanol ausgefällt und isoliert Nach Trocknung erhielt man ca. 40 g eines Farbstoffpulvers, dem die Struktur 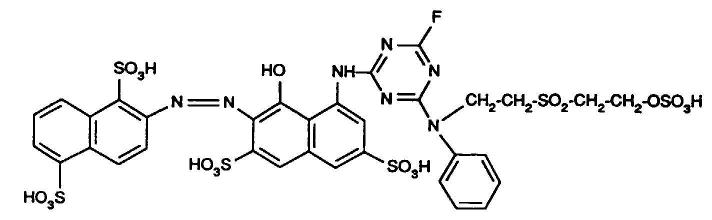 zukommt (λₘₐₓ: 518 und 539 nm (H₂O)) und das Baumwolle nach den für Reaktivfarbstoffe üblichen Färbe- und Druckverfahren in roten Farbtönen färbt.

Weitere rote Reaktivfarbstoffe erhält man durch Kondensation der folgenden Komponenten.

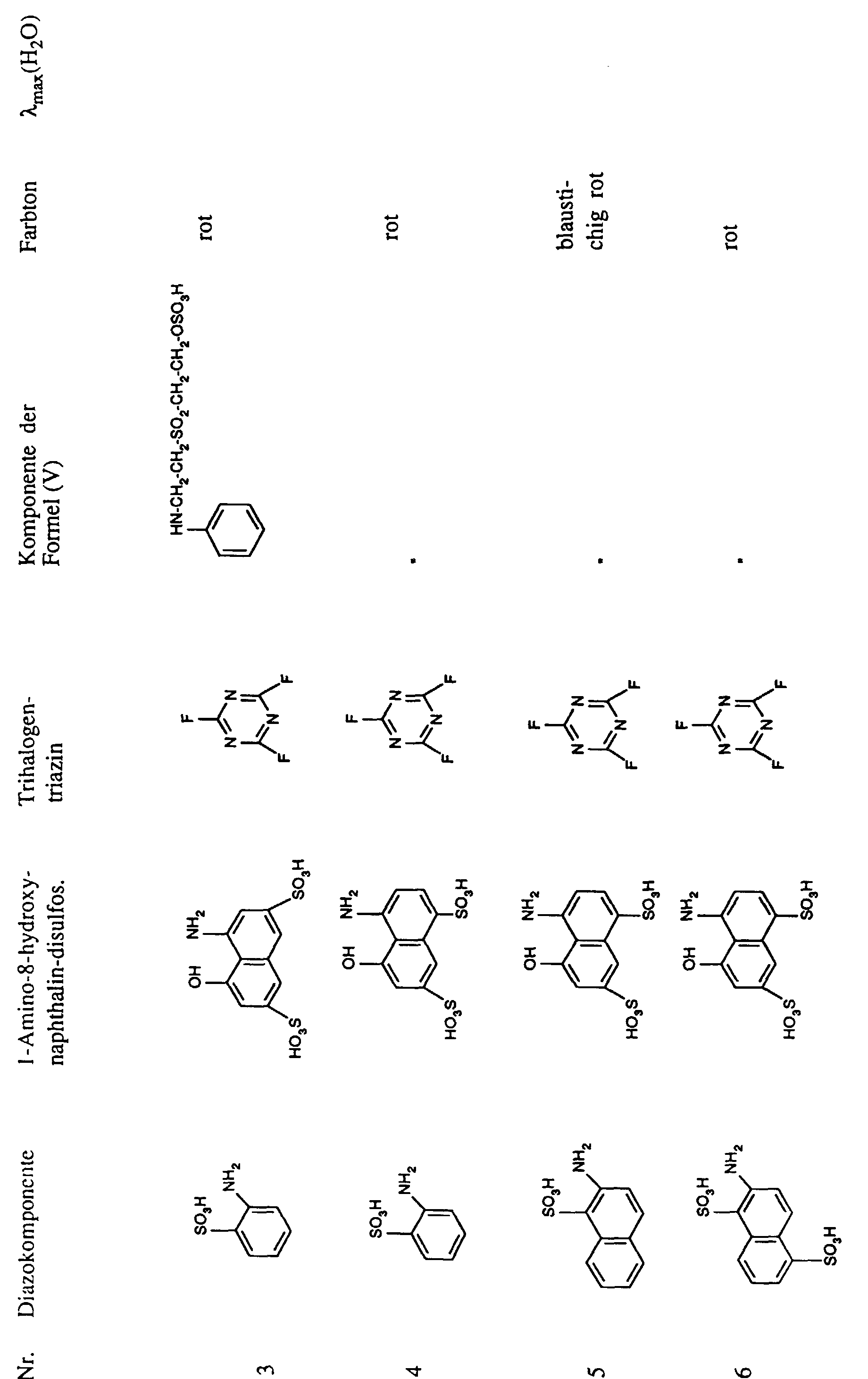

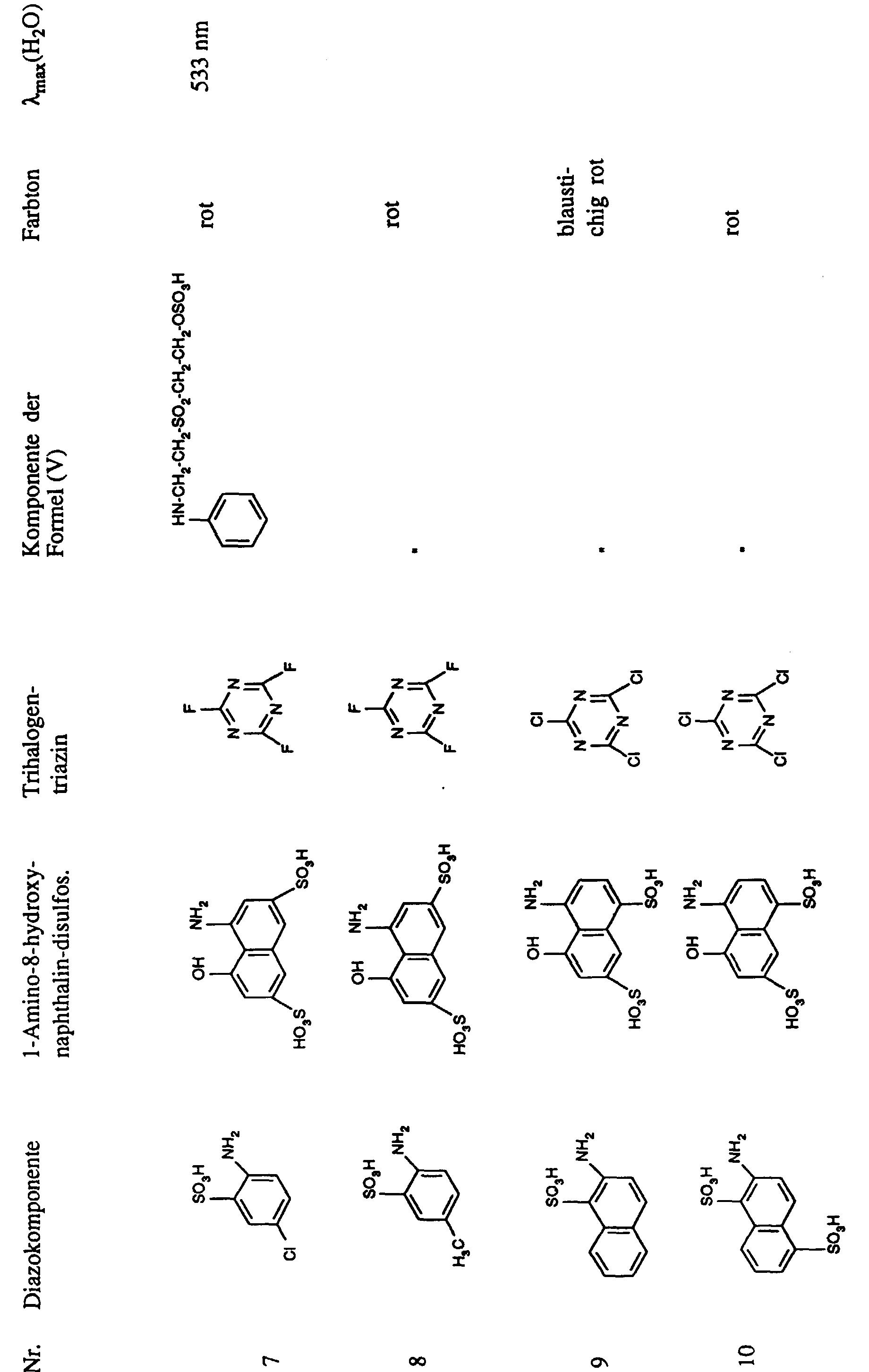

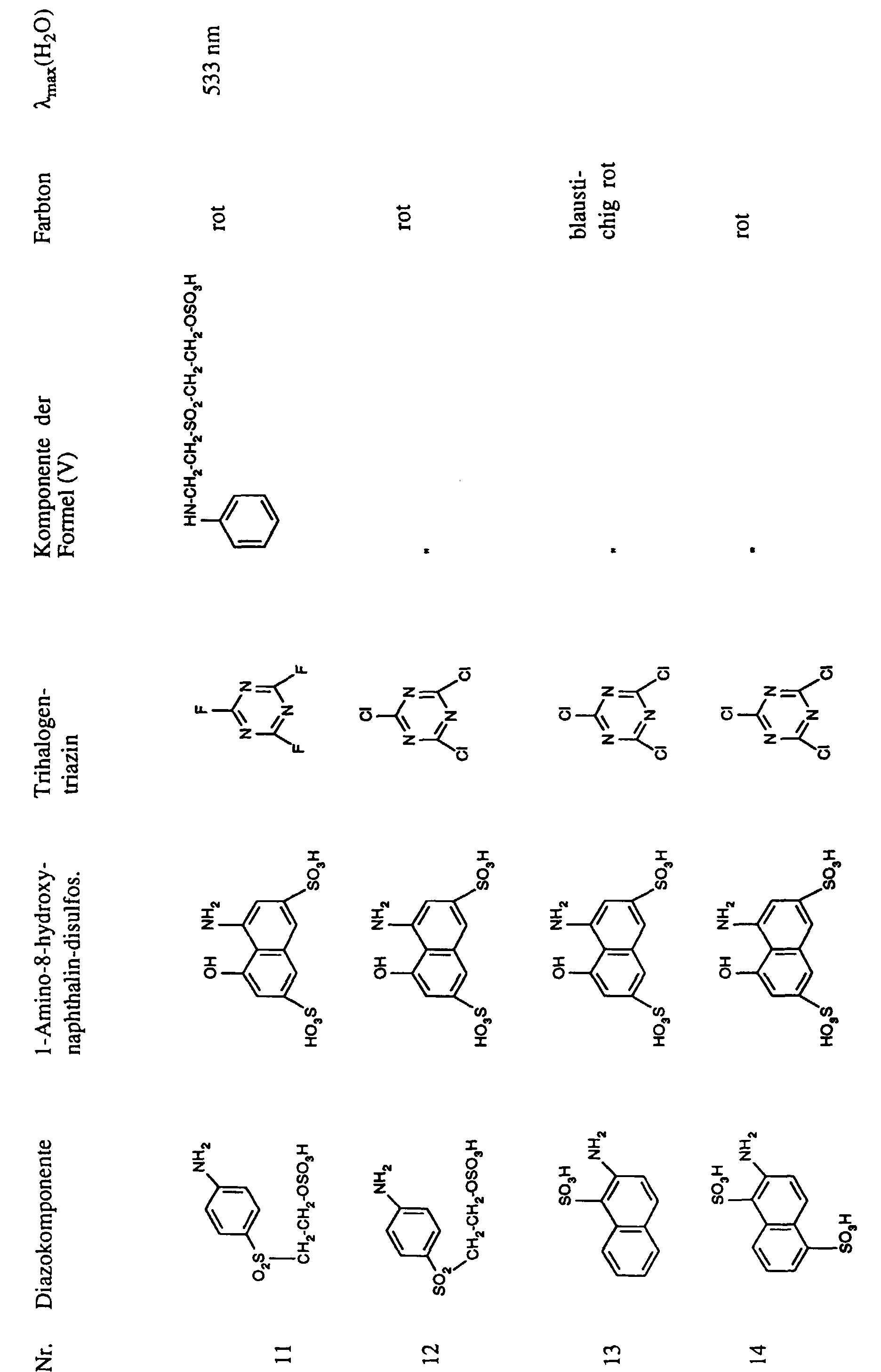

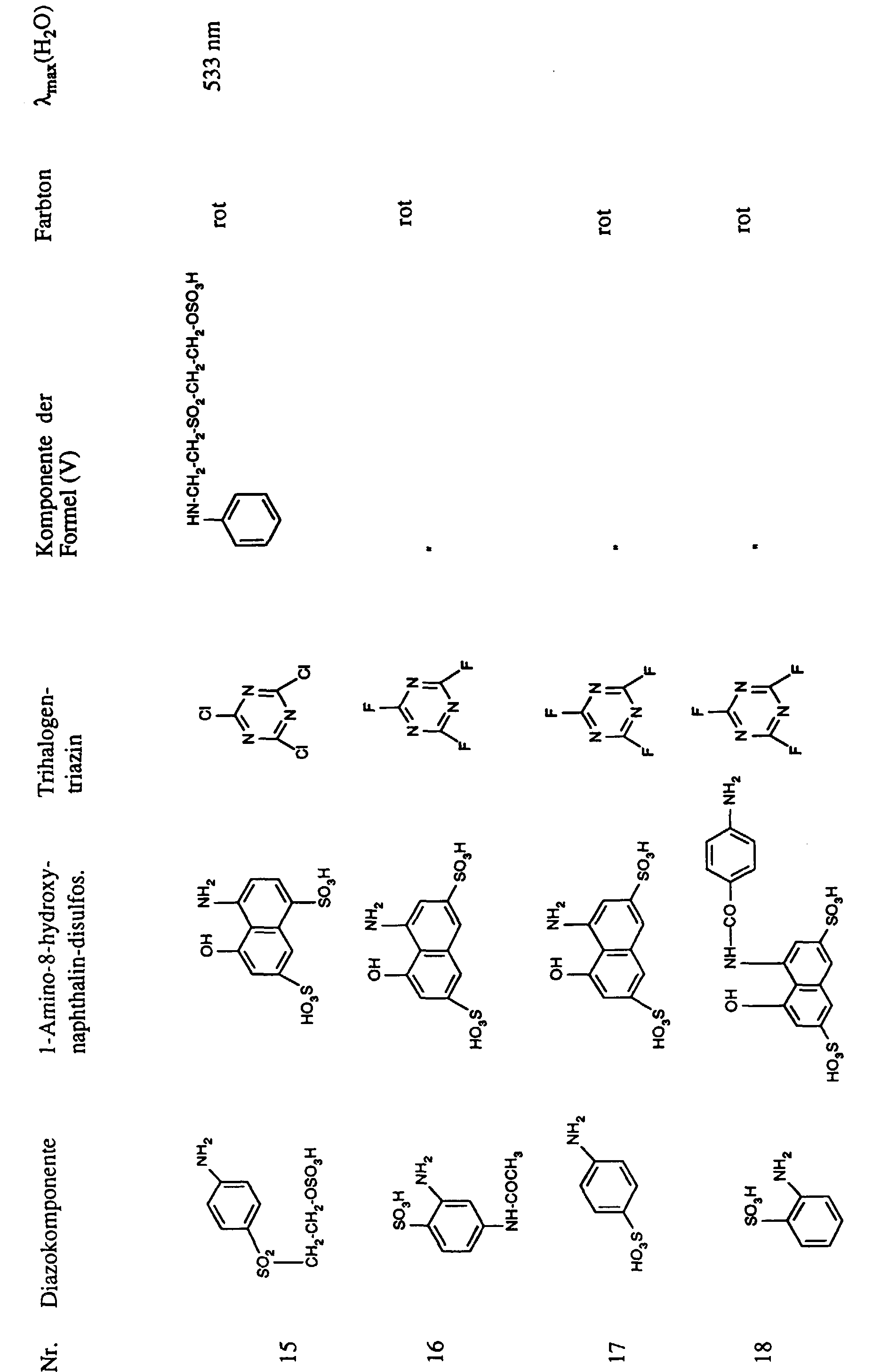

### Beispiel 19

0,1 Mol der Monoazoverbindung mit der Formel 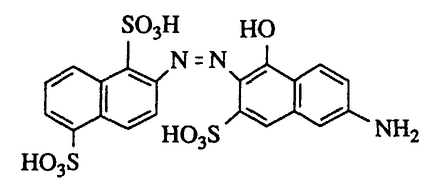 wurden in ca. 500 Teilen Wasser neutral gelöst. Man gab 0,15 Mol der in Beispiel 1 hergestellten Kondensationslösung zu. Mit Sodalösung wurde bei 20°C ein pH-Wert von 7-8 gehalten. Nach 2 Stunden war die Reaktion zum größten Teil beendet. Der Farbstoff wurde durch Zugabe von Kaliumchlorid ausgesalzen, isoliert und getrocknet. Man erhielt ca. 72 g eines salzhaltigen Farbstoffpulvers, dem die Struktur 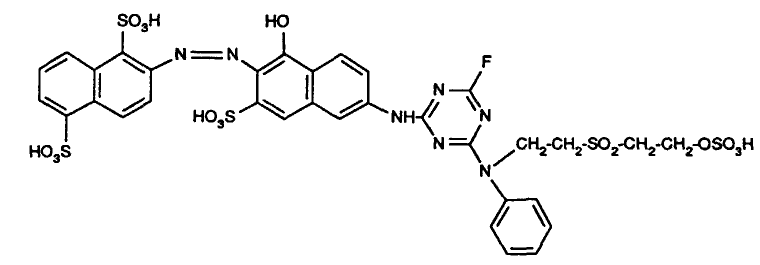 zukommt (..λₘₐₓ = 485 nm (H₂O)) und das Baumwolle nach den für Reaktivfarbstoffe üblichen Färbe- oder Druckverfahren in orangen Farbtönen färbt.

### Beispiel 20

0,1 Mol 2-Amino-5-methoxy-benzolsulfonsäure wurden in 150 Teilen Wasser und 50 Teilen Eis neutral gelöst. Man gab 28 Teile konzentrierter Salzsäure zu. Innerhalb von 15 Minuten tropfte man eine Lösung von 7 Teilen Natriumnitrit in 70 Teilen Wasser zu. Nach 30-minütigem Nachrühren war die Diazotierung beendet. Der Nitritüberschuß wurde mit Amidosulfonsäure zerstört

0,12 Mol 7-Amino-4-hydroxy-naphthalinsulfonsäure-2 wurden in 300 Teilen Wasser suspendiert und mit 10 %iger Lithiumhydroxidlösung bei pH = 6-6,5 gelöst. Man gab 0,18 Mol der in Beispiel 1A hergestellten Kondensationslösung zu. Mit Sodalösung wurde bei 20°C ein pH-Wert von 4-5 gehalten. Nach 2 Stunden war die Kondensation beendet Man erhielt 900 Teile einer Lösung. Das Produkt wurde mit 180 Teilen Kaliumchlorid ausgesalzen und isoliert Man erhielt ca. 220 Teile einer grauen Paste. Die Paste wurde in 600 Teilen Wasser gelöst. Man tropfte die obige Diazotierung hinzu und hielt gleichzeitig durch Zugabe von NaHCO₃ einen pH-Wert von 6-6,5. Anschließend stellte man mit Sodalösung einen pH-Wert von 7,5-8 ein. Die Lösung rührte über Nacht.

Der Farbstoff wurde durch Zugabe von Natriumchlorid ausgesalzen und isoliert Nach Trocknung erhielt man ca. 105 g eines salzhaltigen Farbstoffpulvers, dem die Struktur 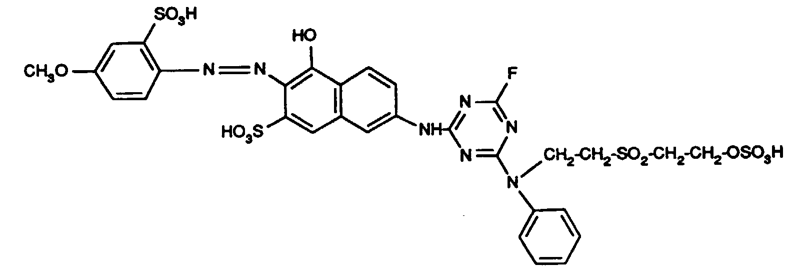 zukommt und das Baumwolle nach den für Reaktivfarbstoffe üblichen Färbe- oder Druckverfahren in Scharlachtönen färbt.

Weitere Reaktivfarbstoffe werden durch Kondensation von Trifluortriazin und den folgenden Komponenten analog Beispiel 19 oder 20 erhalten.

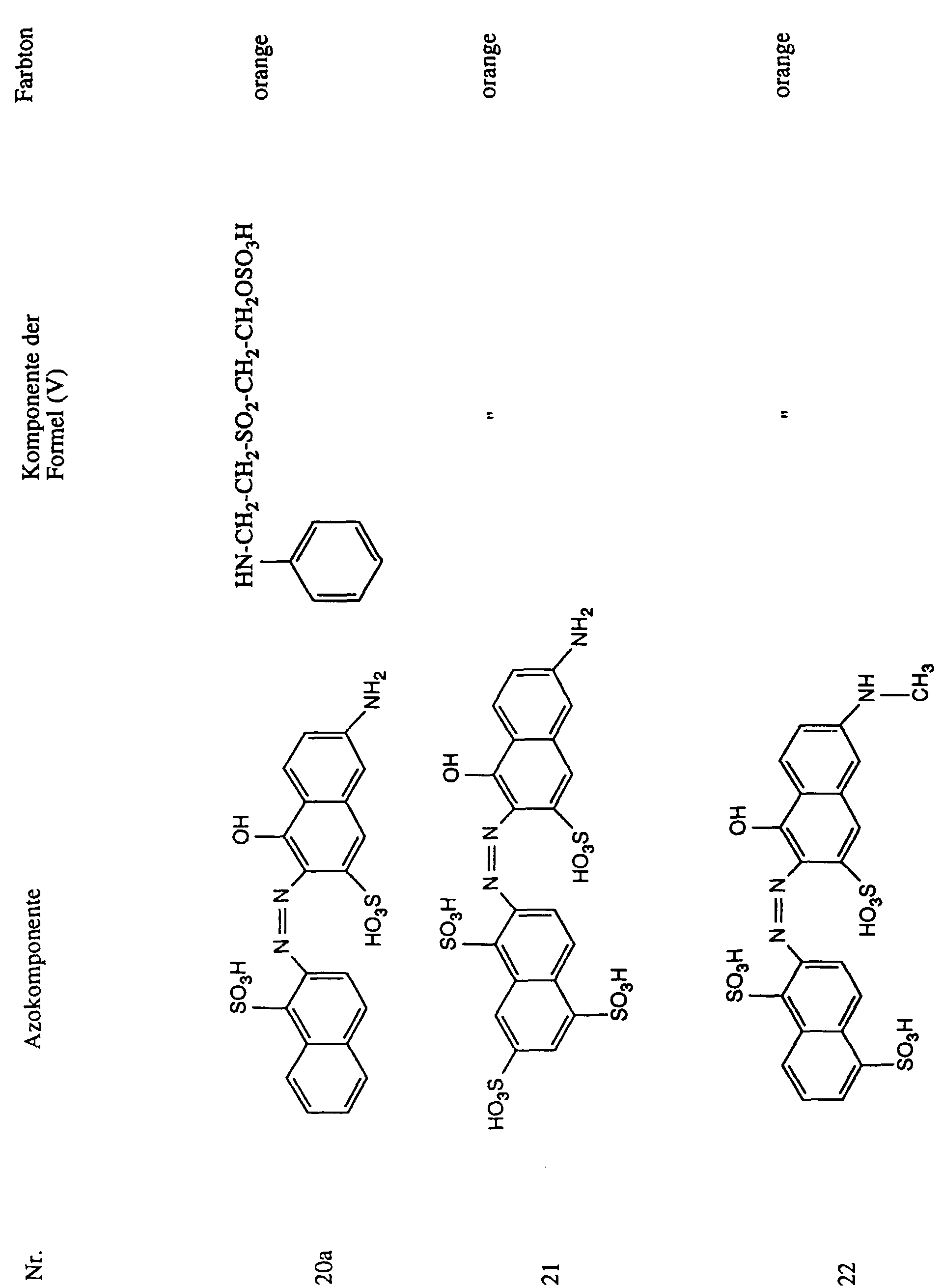

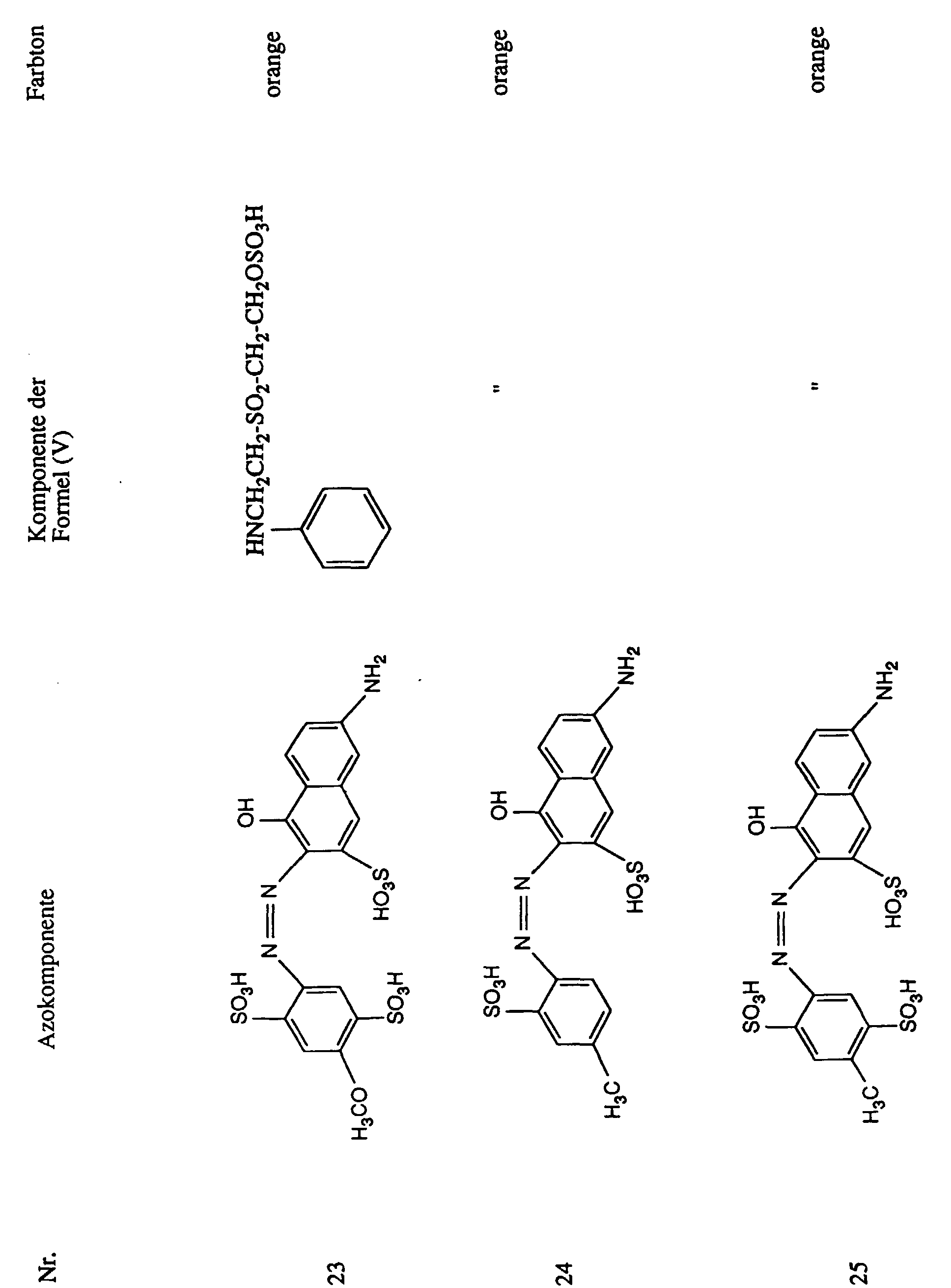

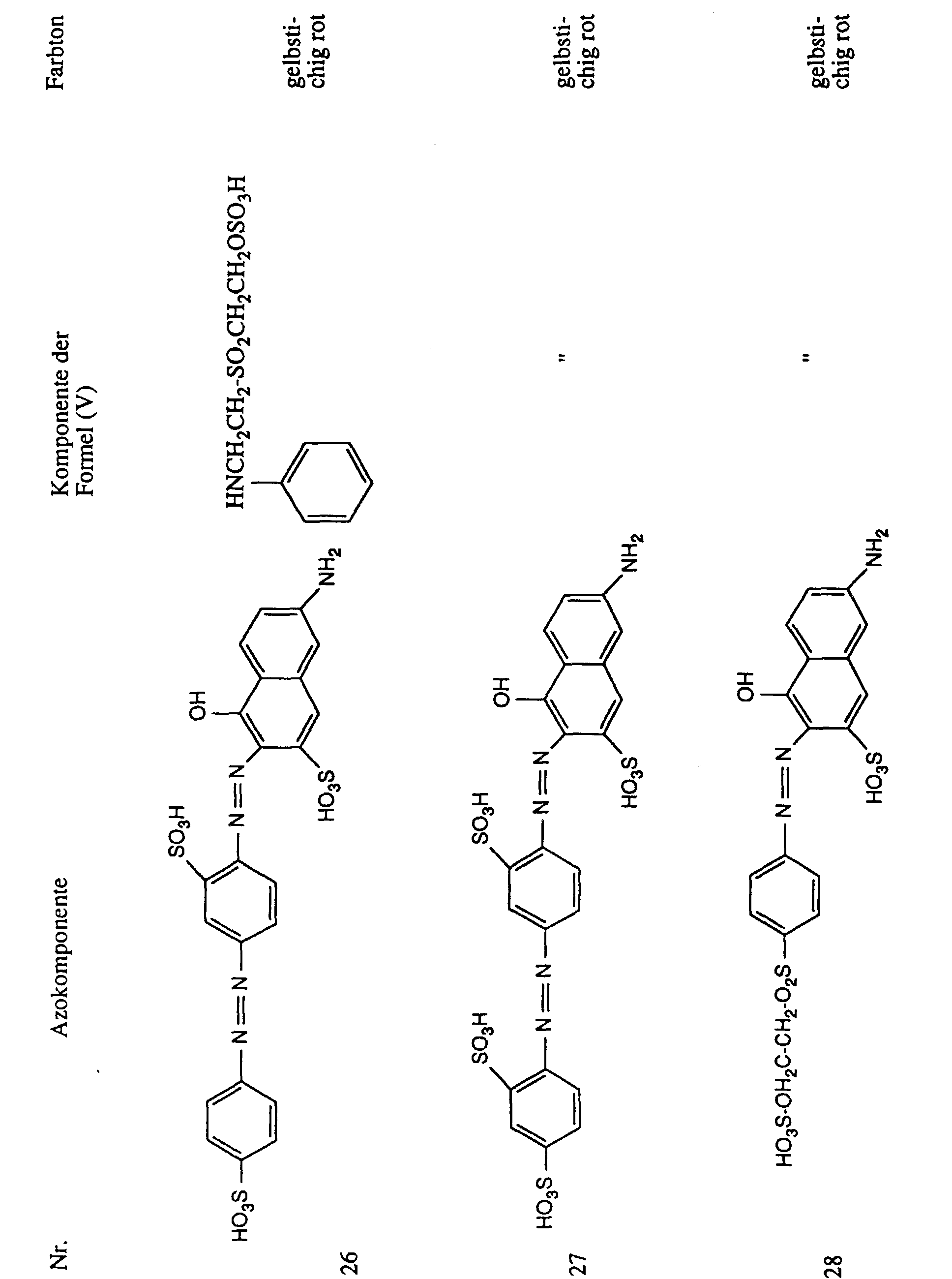

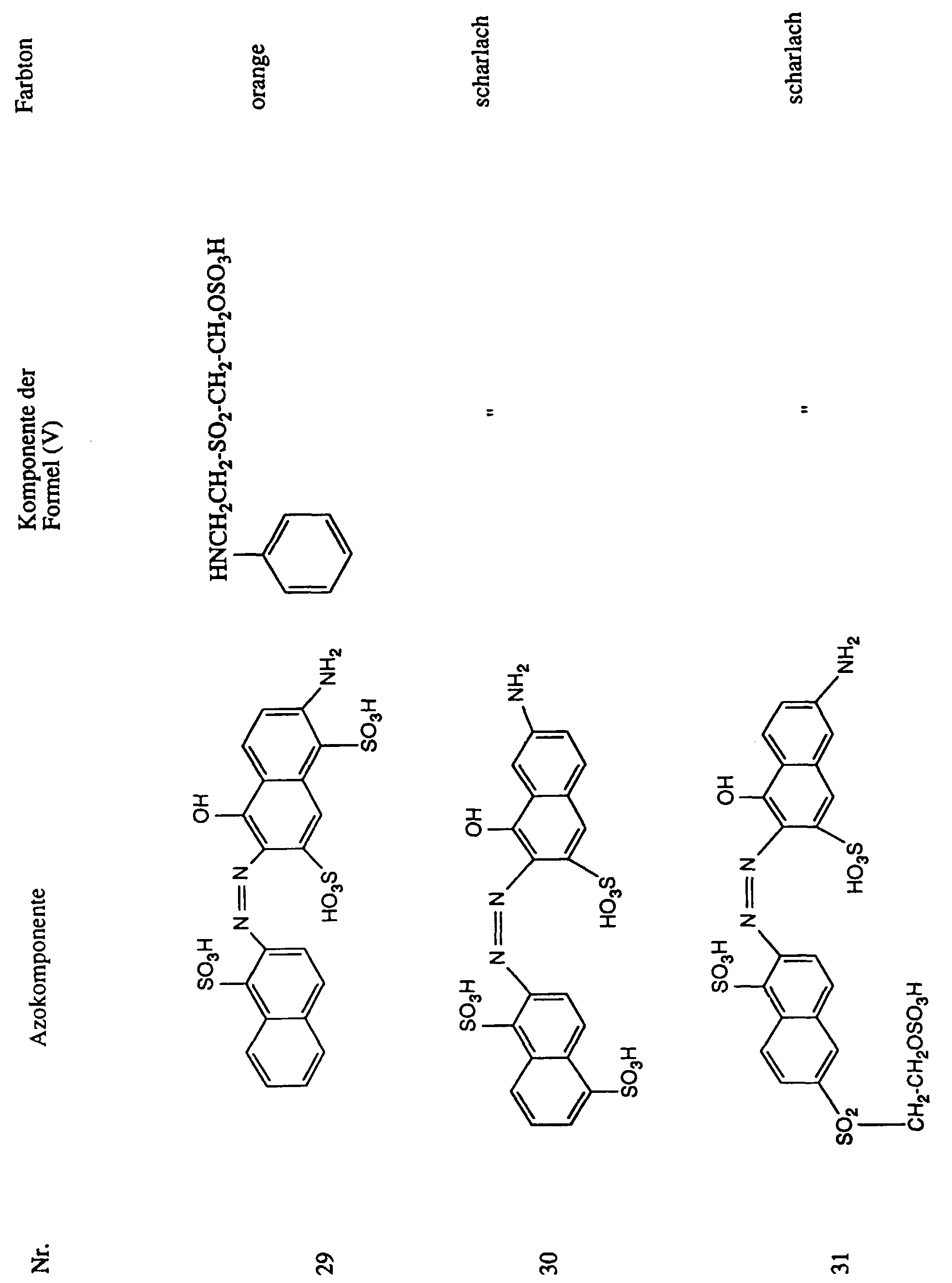

### Beispiel 32

0,1 Mol des Monoazofarbstoffes der Formel 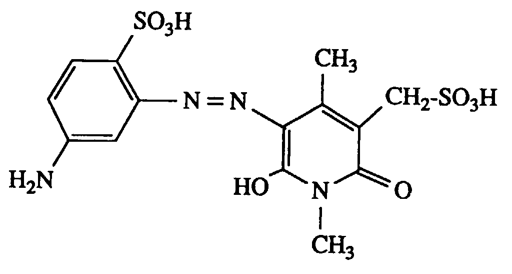 wurden in 500 Teilen Wasser neutral gelöst. Man gab 0,2 Mol der in Beispiel 1A hergestellten Kondensationslösung zu. Mit Sodalösung wurde über 8 Stunden bei 20°C ein pH-Wert von 7-8 gehalten. Man erhielt ca. 1100 Teile einer Farbstofflösung. Der Farbstoff wurde mit Kaliumchlorid ausgesalzen, isoliert und getrocknet. Man erhielt ca. 105 g eines salzhaltigen Farbstoffpulvers, dem die Struktur 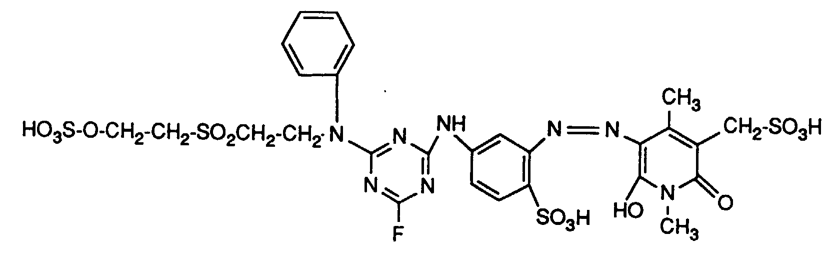 zukommt (λₘₐₓ = 420 nm (H₂O)) und Baumwolle nach dem für Reaktivfarbstoffe üblichen Färbe- oder Druckverfahren in gelben Farbtönen färbt.

Weitere gelbe Reaktivfarbstoffe erhält man durch Kondensation der folgenden Komponenten:

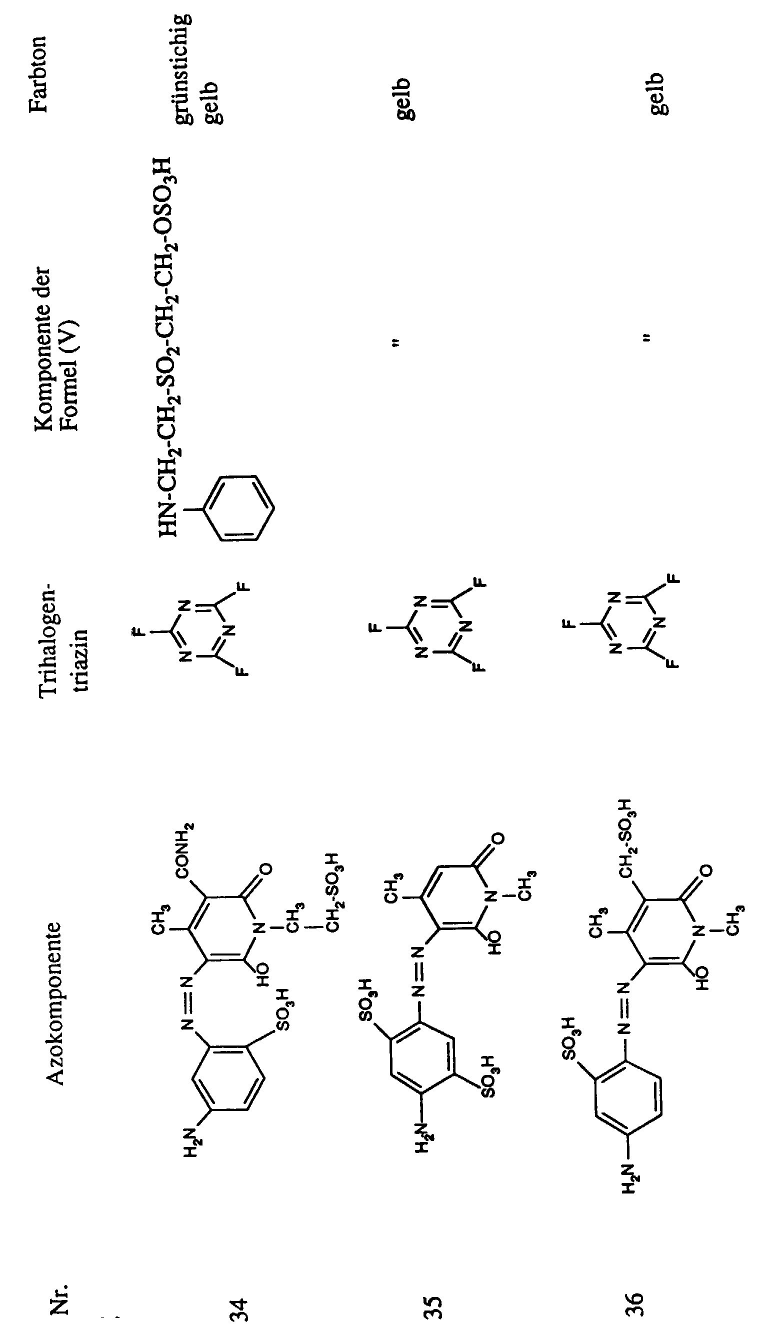

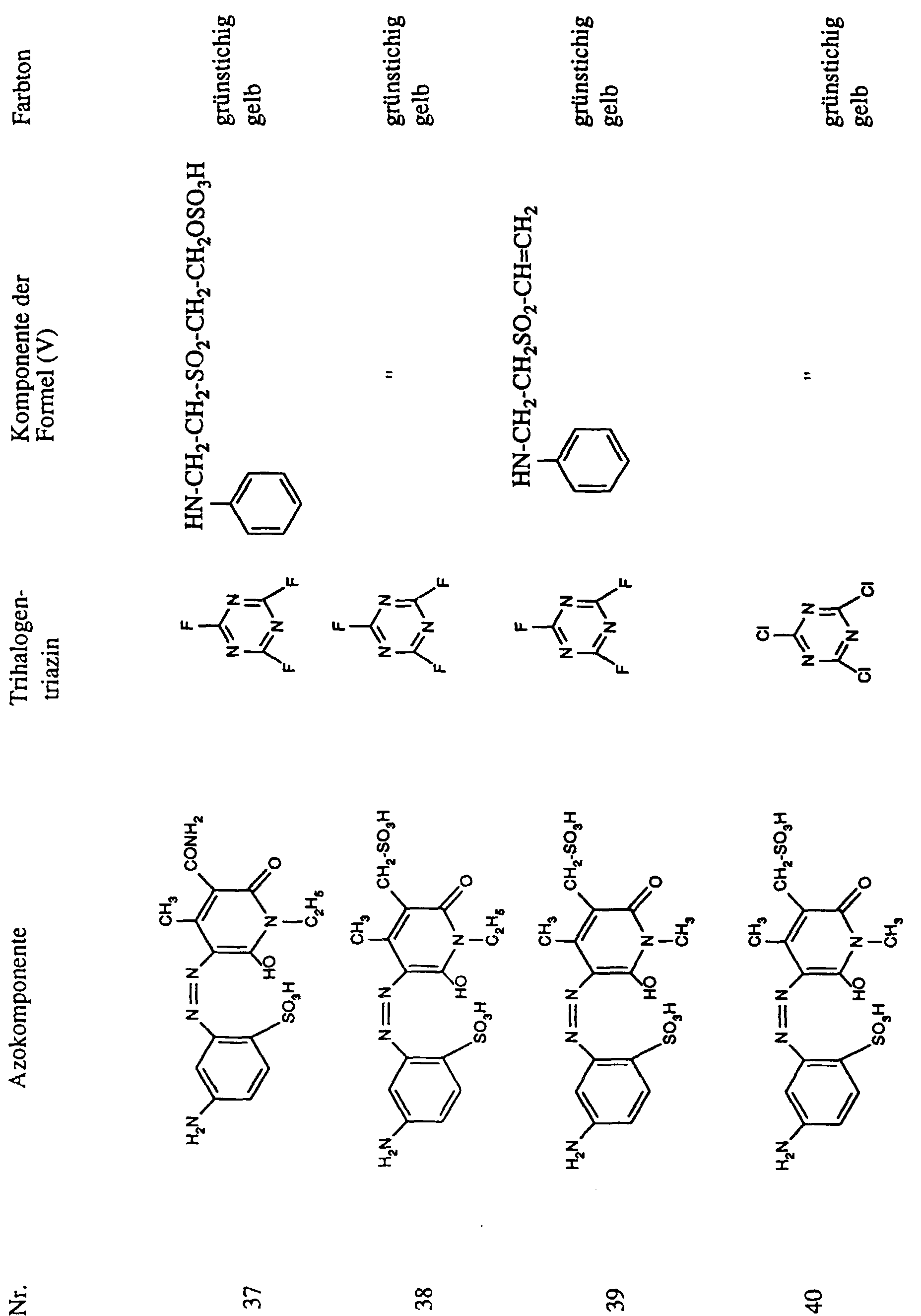

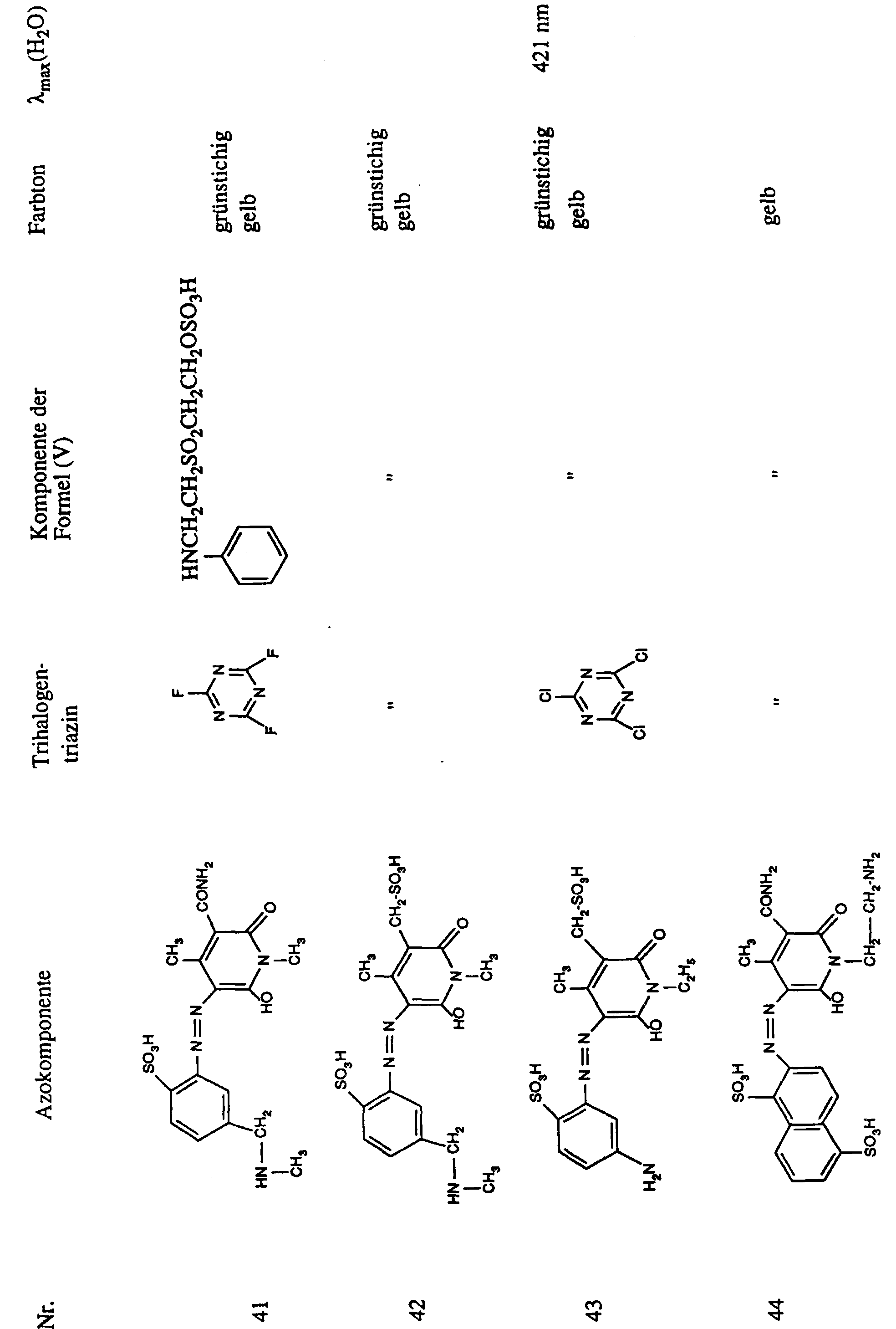

### Beispiel 45

0,1 Mol des Monoazofarbstoffes der Formel 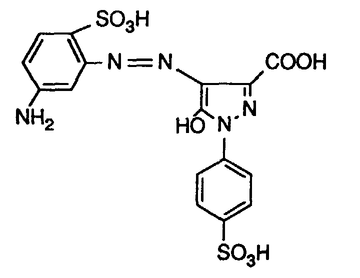 wurden in 400 Teilen Wasser neutral gelöst. Man gab 0,15 Mol der in Beispiel 1A hergestellten Kondensationslösung zu. Mit Sodalösung wurde über 8 Stunden bei 20°C ein pH-Wert von 7-8 gehalten. Man erhielt ca. 1100 Teile einer Farbstofflösung. Der Farbstoff wurde mit Kaliumchlorid ausgesalzen, isoliert und getrocknet Man erhielt ca. 105 g eines salzhaltigen Farbstoffpulvers, dem die Struktur 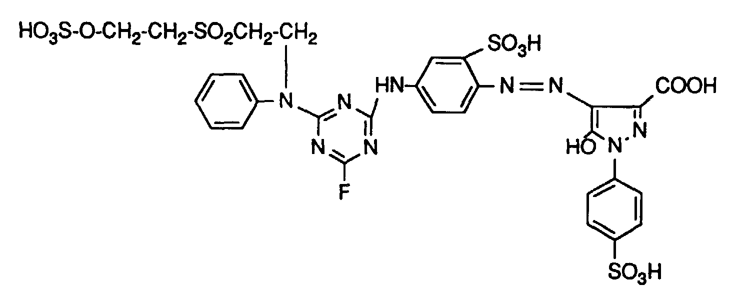 zukommt und Baumwolle nach dem für Reaktivfarbstoffe üblichen Färbe- oder Druckverfahren in gelben Farbtönen färbt.

Weitere gelbe Farbstoffe mit ähnlichen Eigenschaften erhält man, wenn man die folgenden Aminophenylazopyrazolone mit Cyanurfluorid und der angegebenen Verbindung der Formel (V) kondensiert

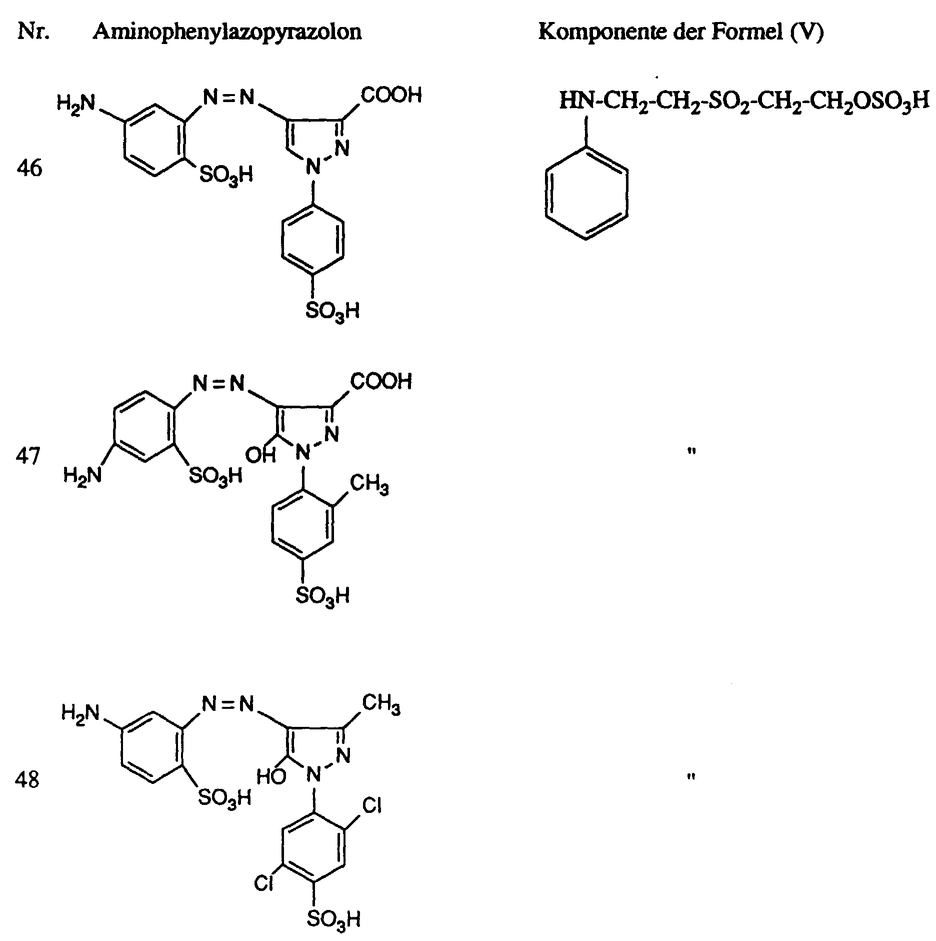

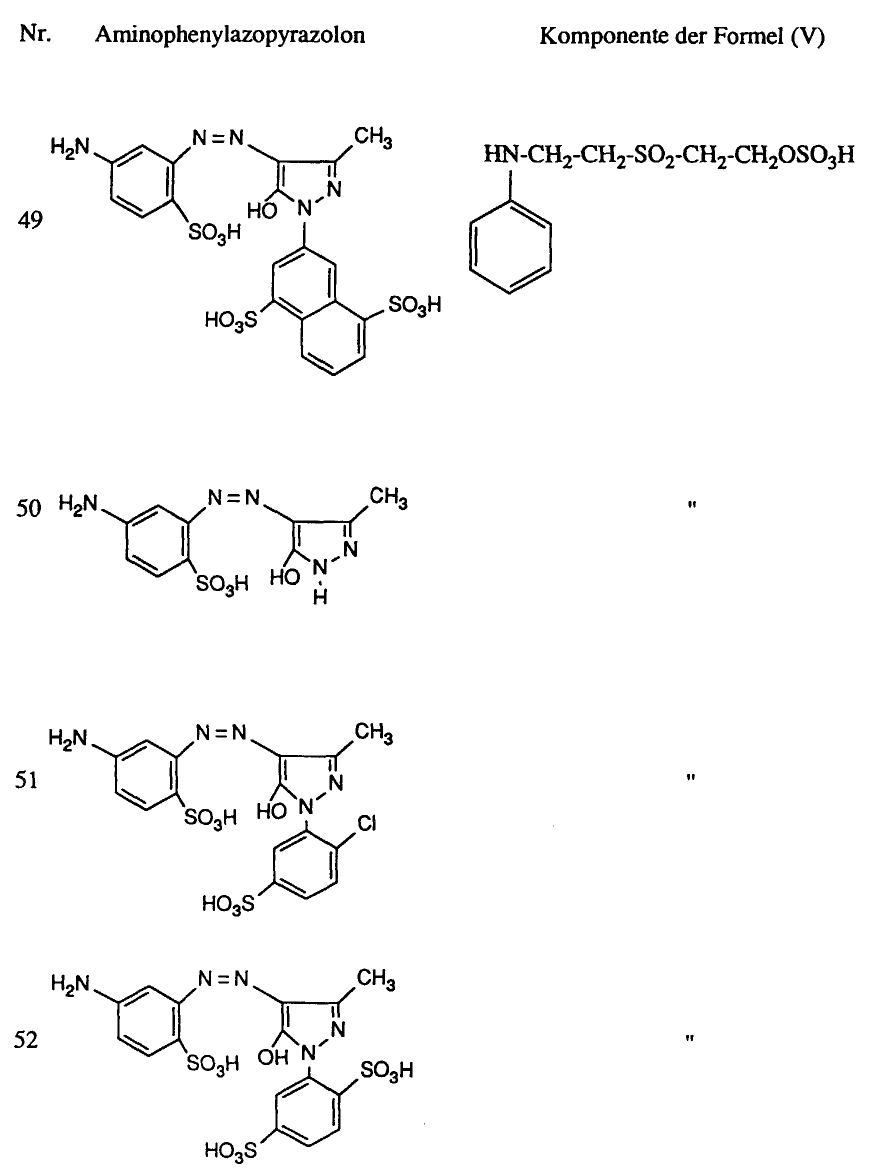

### Beispiel 53

45,3 g des Kupplungsproduktes von 2-Aminonaphthalin-6,8-disulfonsäure auf 3-Aminoacetanilid werden in 600 ml Wasser bei pH 7 unter Erwärmen auf 50°C gelöst. Nach dem Abkühlen auf 20°C werden 0,15 Mol der nach Beispiel 1A hergestellten Komponente (VIb) der Formel 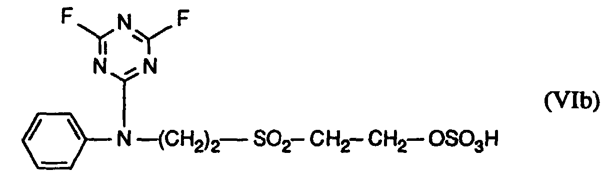 zugegeben und über 8 h der pH-Wert mit Sodalösung bei 7-7,5 gehalten.
Die erhaltene Lösung wird nach Zusatz von 1,5 g Phosphat-Puffer von pH 6 im Vakuum bei 35 bis 40°C eingedampft oder sprühgetrocknet.
Der erhaltene Farbstoff der Formel 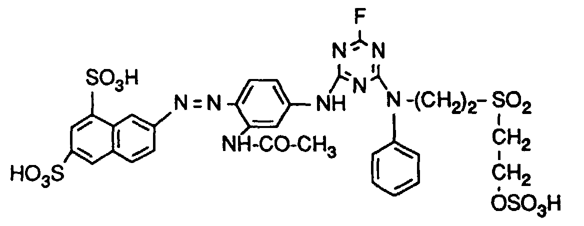 färbt Baumwolle aus langer Flotte mit einer optimalen Färbetemperatur von 50°C in goldgelben Tönen.
λ_{max.} = 389 nm in Wasser.

Weitere rotstichig gelb färbende Farbstoffe erhält man durch Kondensation folgender p-Aminoazoverbindungen mit Cyanurfluorid oder Cyanurchlorid und Verbindungen der Formel (V).

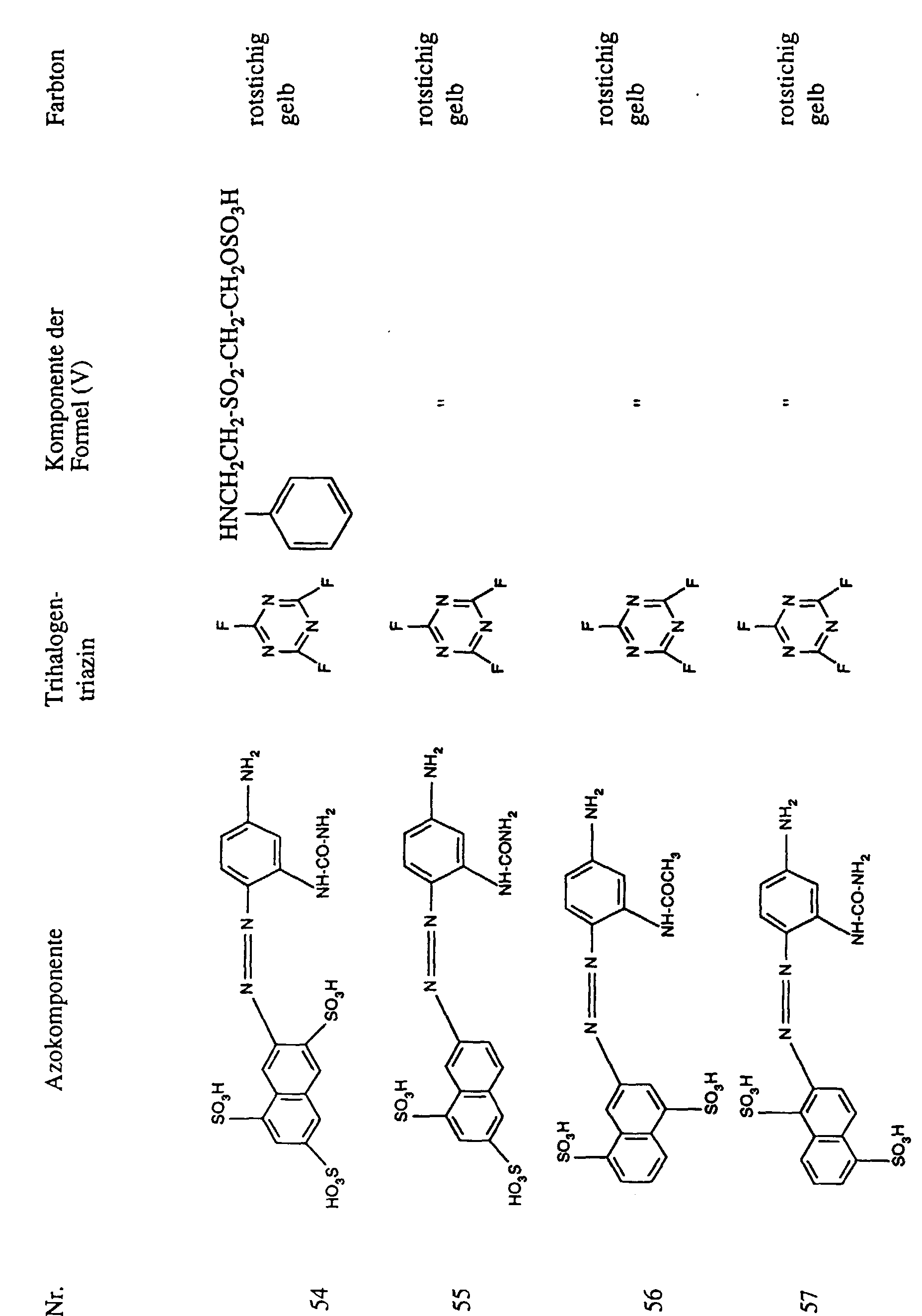

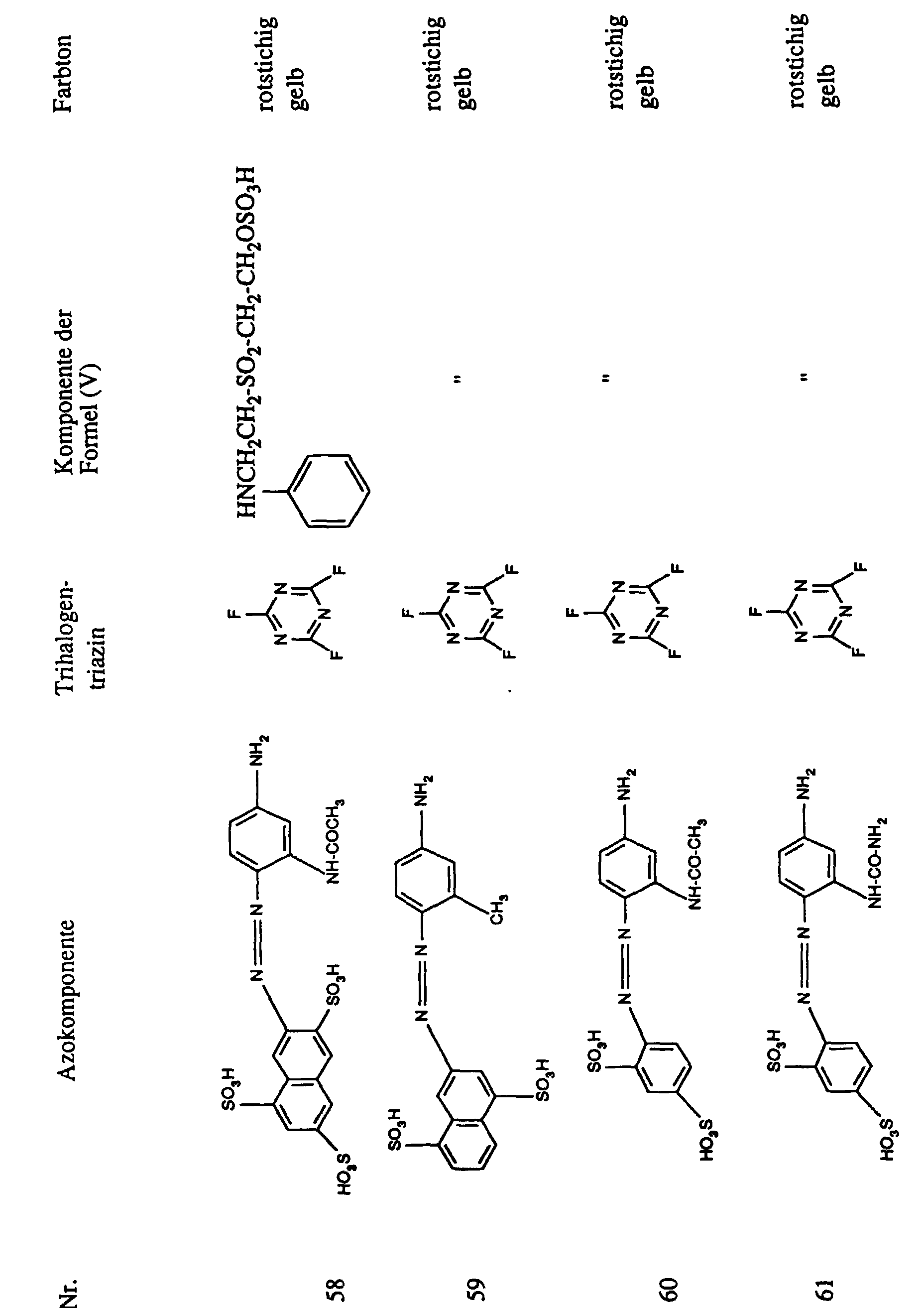

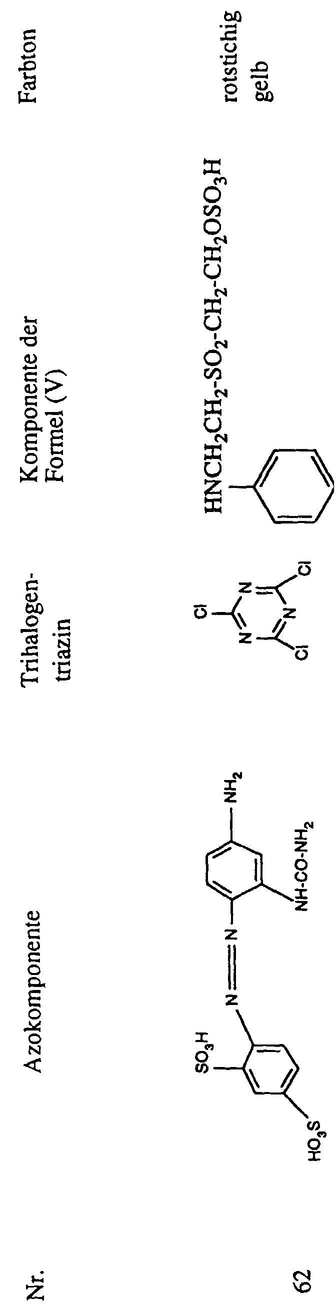

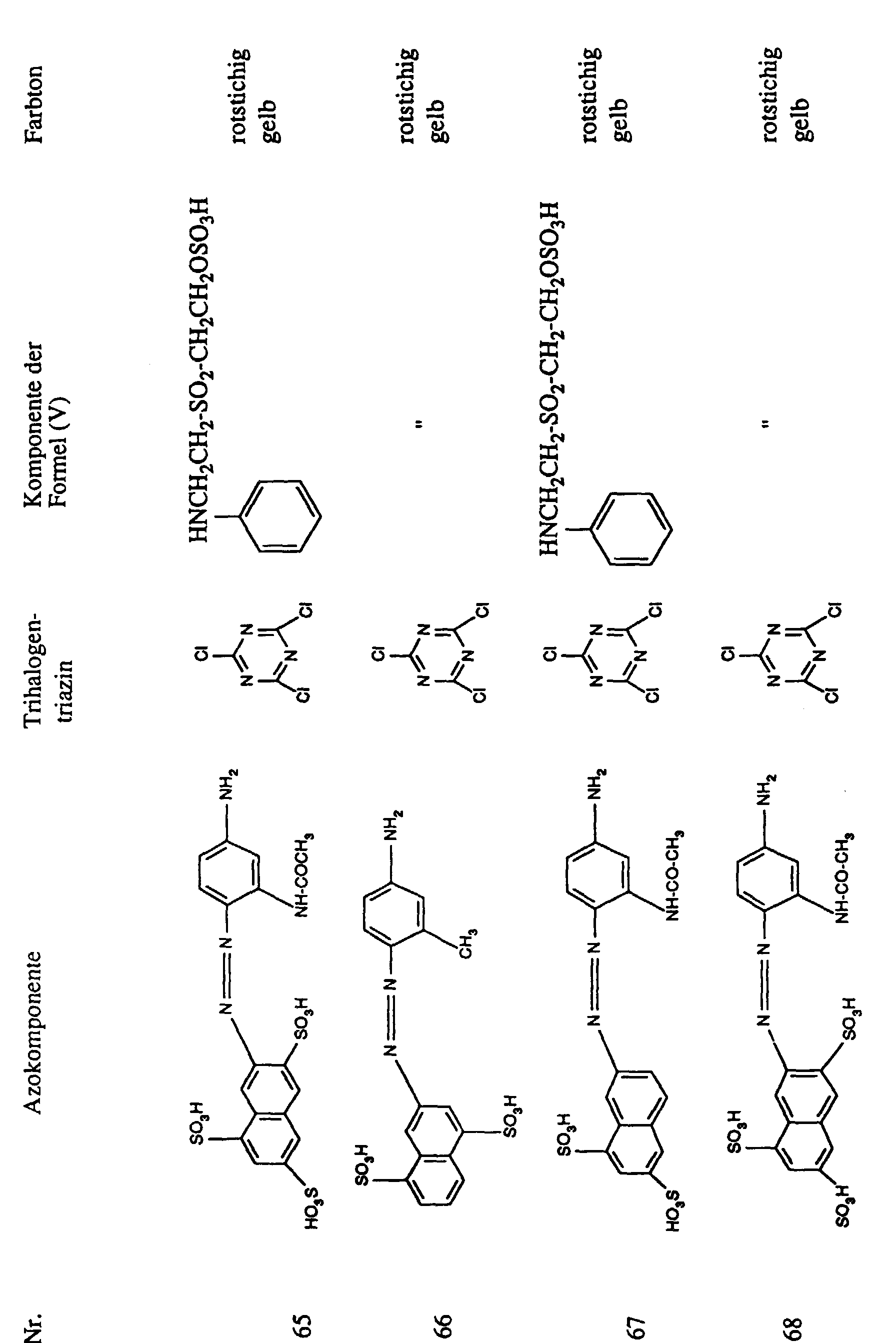

### Beispiel 69

Gemäß Beispiel 17 kondensiert man 0,15 Mol Komponente der Formel 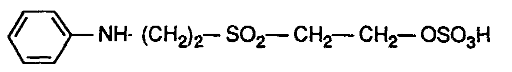 mit Cyanurfluorid. Die erhaltene Lösung des Kondensationsproduktes läßt man in eine auf pH 7 gestellte, 0 bis 5°C gekühlte Lösung von 0,1 Mol Azoverbindung der Formel 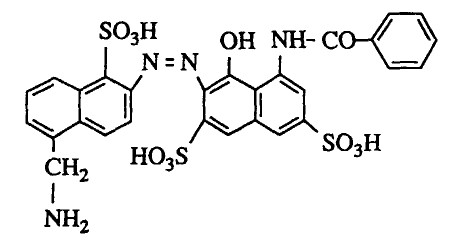 in 300 ml Wasser einlaufen und hält dabei den pH-Wert in der Mischung mit verdünnter Sodalösung auf 6,5-7,5. Die Temperatur steigt auf 20-25°C. Nach beendeter Kondensation wird der Farbstoff durch Aussalzen und Absaugen isoliert und nach Pufferung auf einen pH-Wert von 6,5 im Vakuum bei 45°C getrocknet. Er entspricht der Formel 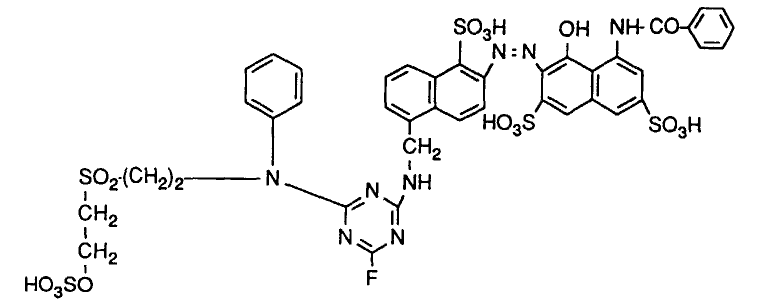 und färbt Baumwolle aus langer Flotte in blaustichig roten Tönen mit guter Fixierausbeute.

Ähnliche rote Farbstoffe erhält man durch Umsetzung folgender Komponenten.

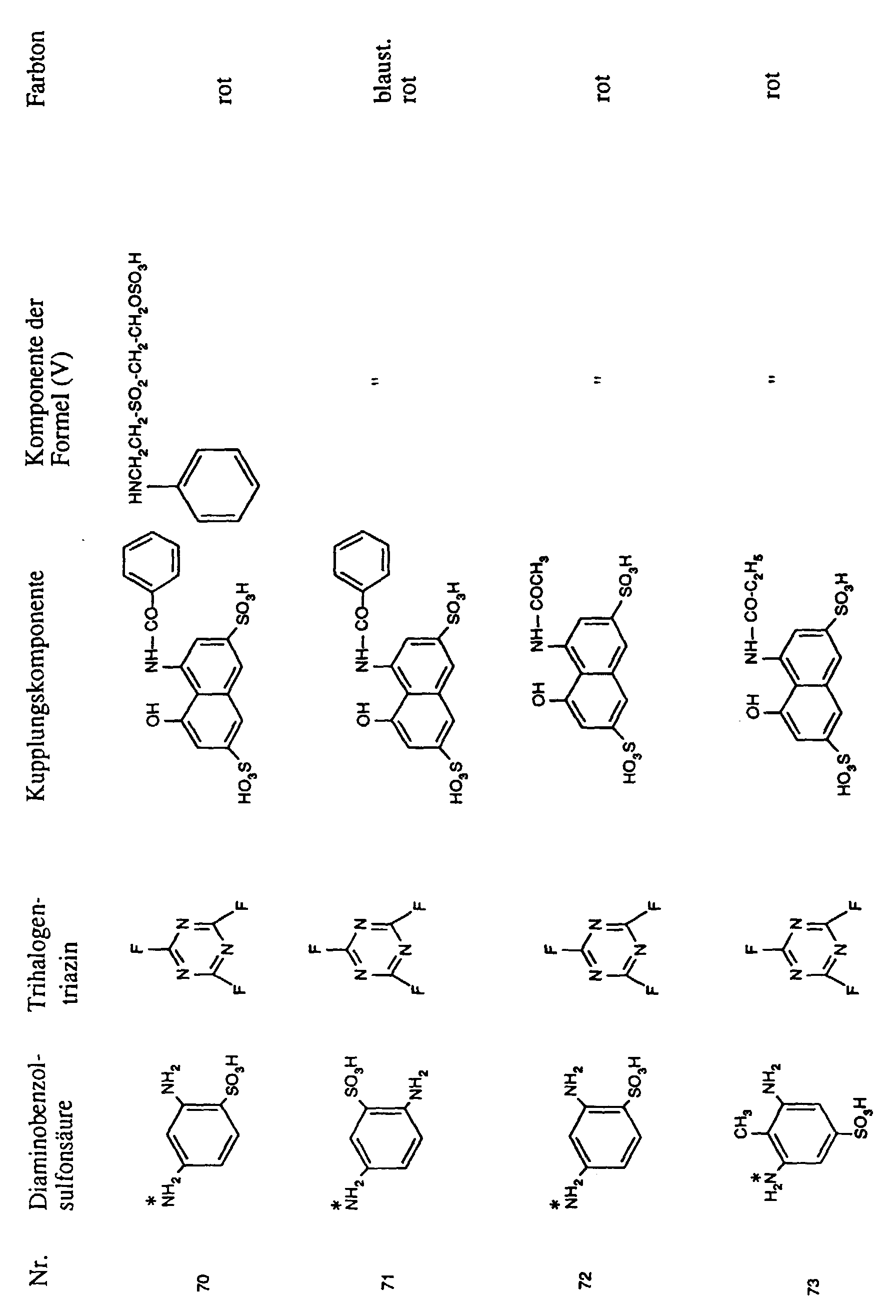

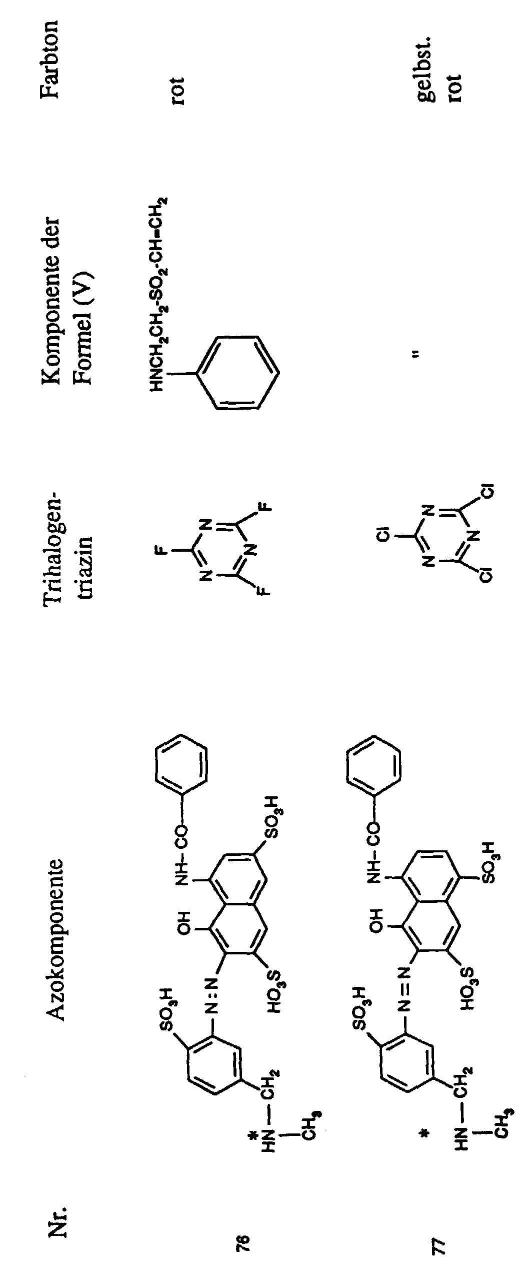

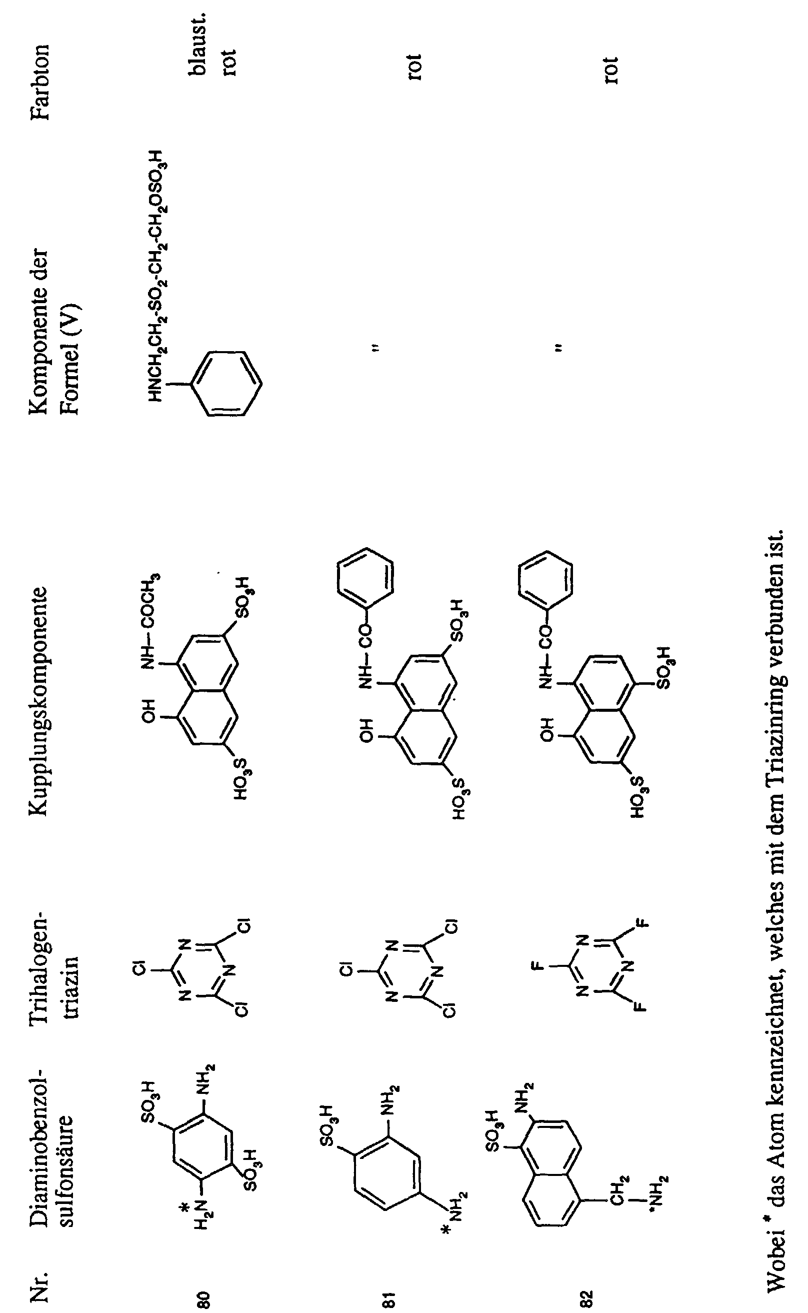

### Beispiel 83

36,7 g der auf bekanntem Weg hergestellten Amino-disazoverbindung der Formel 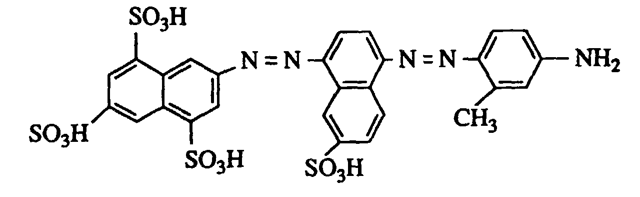 werden in 400 ml Wasser bei einem pH-Wert von 6,0 gelöst. Dazu wird 0,15 Mol der in Beispiel 1A hergestellten Reaktivkomponente (VIb) gegeben und ca. 8 h bei 20°C und pH 8 kondensiert.

Der erhaltene Farbstoff der Formel 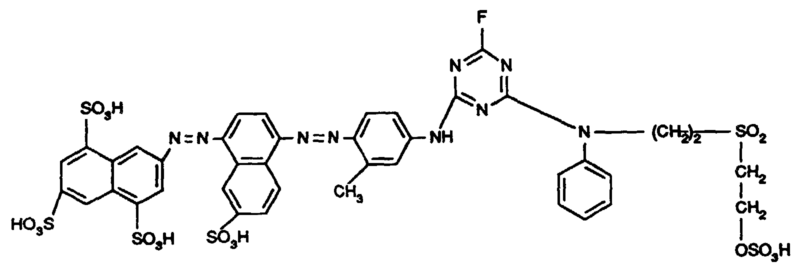 wird mit Natriumchlorid ausgesalzen, abgesaugt und nach Pufferung bei pH 6 im Vakuum bei 45°C getrocknet. Er färbt Baumwolle nach den für Reaktivfarbstoffe bekannten Verfahren ergiebig in braunen Tönen.

### Beispiel 83a

Setzt man anstelle der Aminodisazoverbindung des Beispiels 83 die Verbindung der Formel 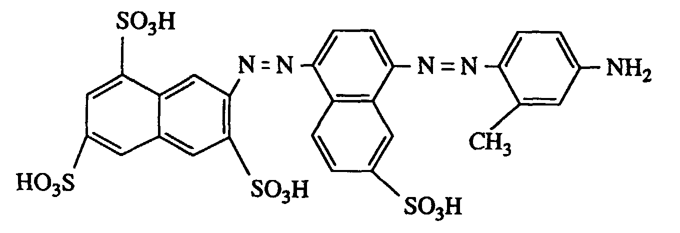 ein, so erhält man einen Farbstoff der Formel 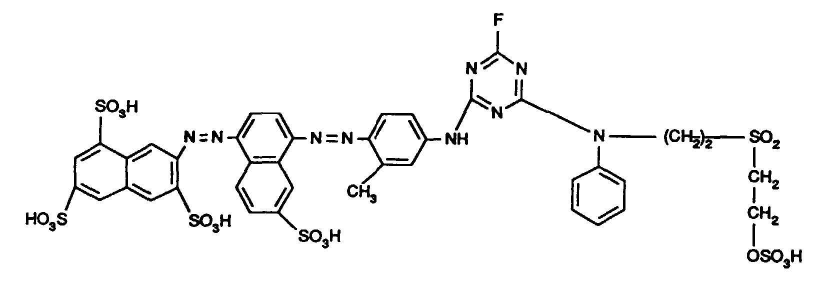 der Baumwolle ebenfalls ergiebig in braunen Tönen färbt.

Weitere braune Reaktivfarbstoffe erhält man durch Kondensation der folgenden Komponenten.

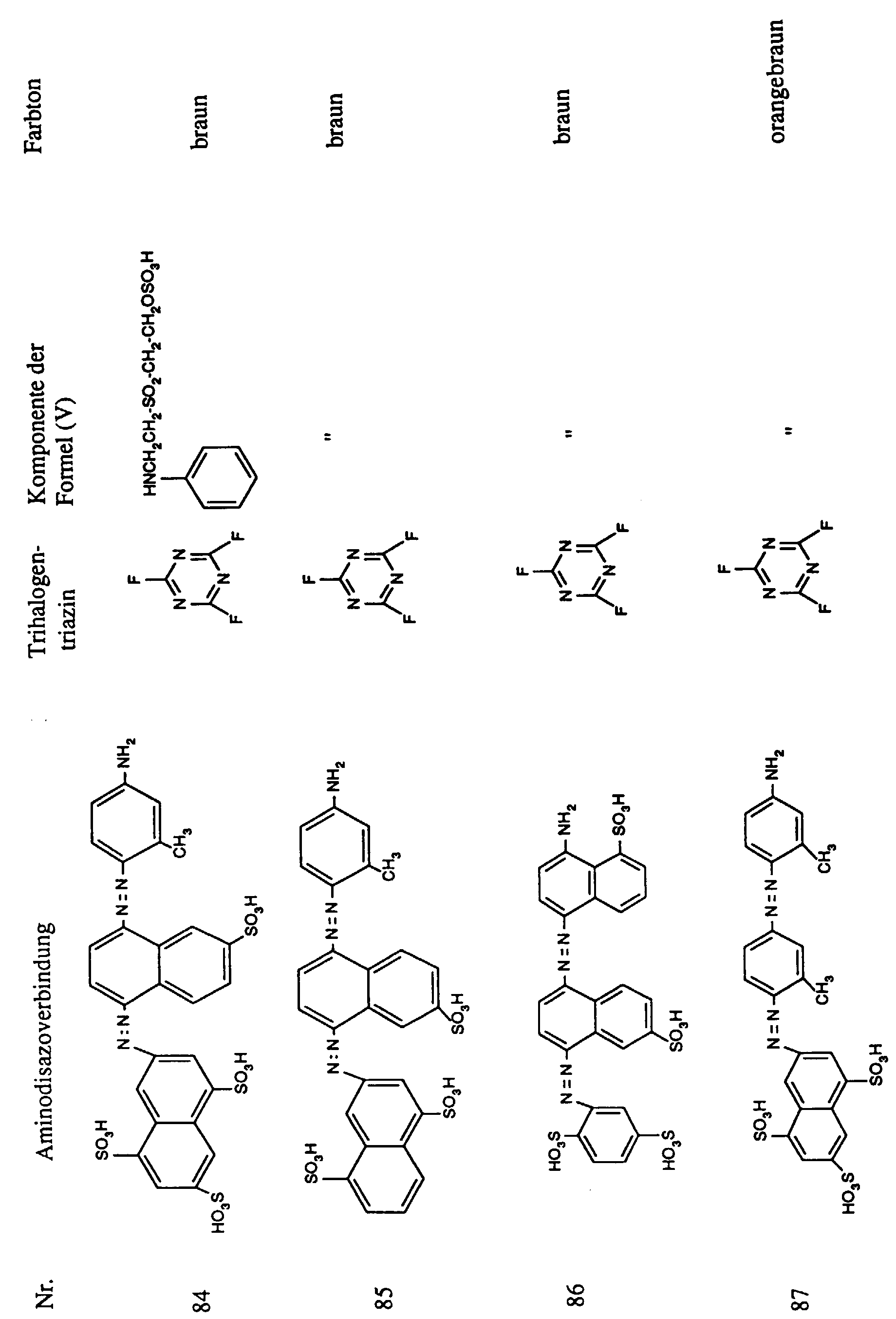

### Beispiel 88

50,3 g Aminoazoverbindung (0,1 Mol) der Formel 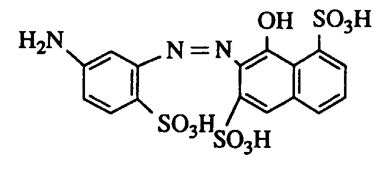 werden in 400 ml Wasser neutral gelöst. Dazu gibt man 0,15 Mol der in Beispiel 1A hergestellten Kondensationslösung. Mit Na₂CO₂-Lösung wird über 8 Stunden ein pH-Wert von 7-8 gehalten. Danach wird der Farbstoff der Formel 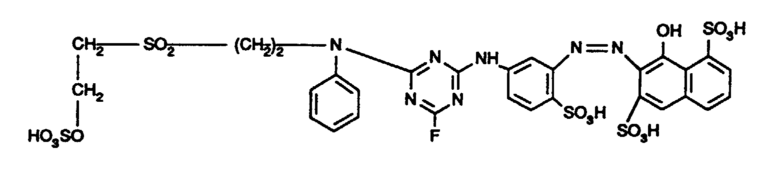 durch Aussalzen und Absaugen isoliert. Nach schonender Trocknung erhält man ein Pulver, das Baumwolle nach den üblichen Methoden ergiebig in Scharlachtönen färbt.

Weitere Reaktivfarbstoffe auf der Basis von Aminoazonaphtholverbindungen erhält man durch Kondensation nachstehender Komponenten.

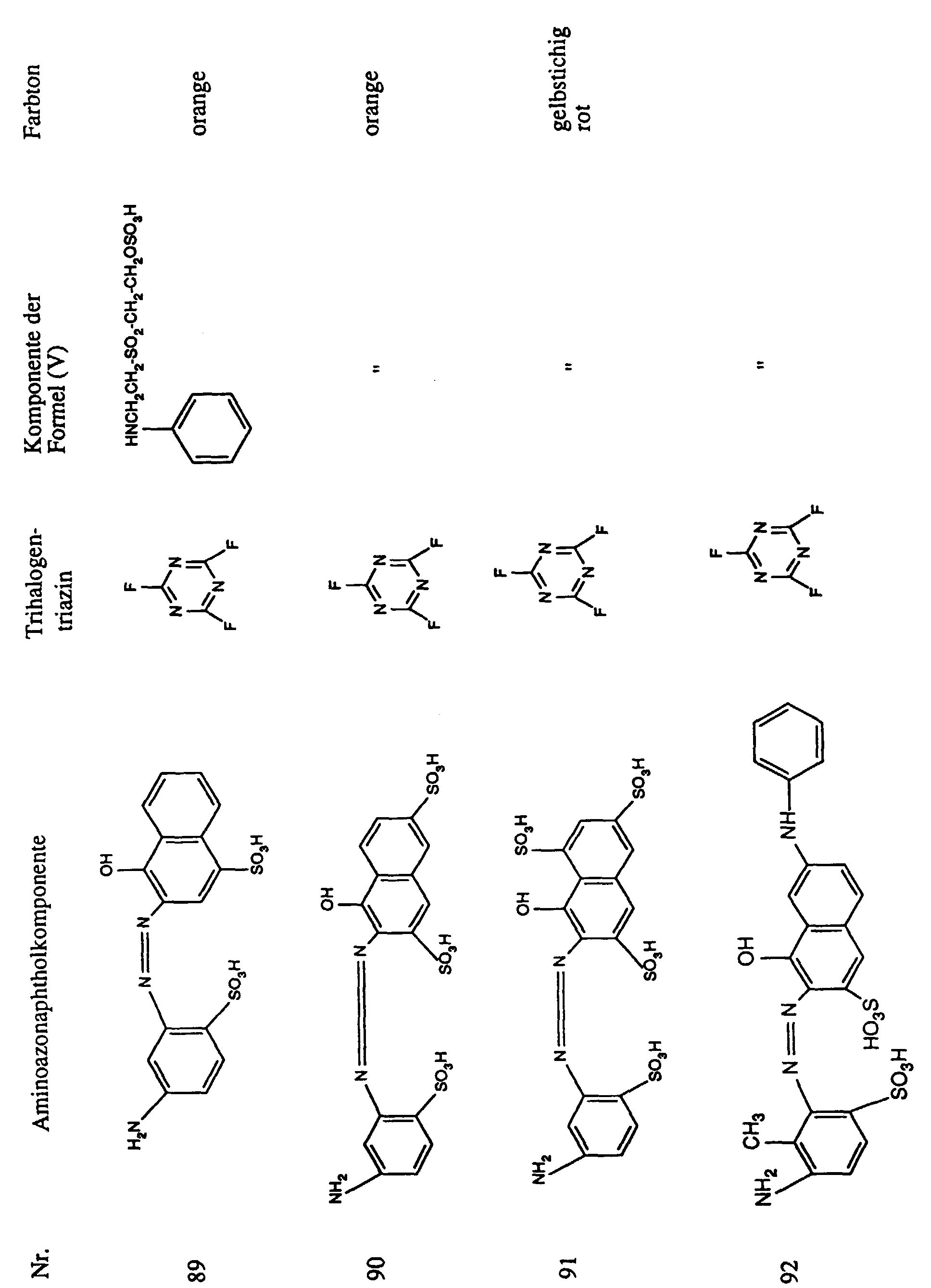

### Beispiel 93

35,2 g der bekannten Verbindung (0,05 Mol) 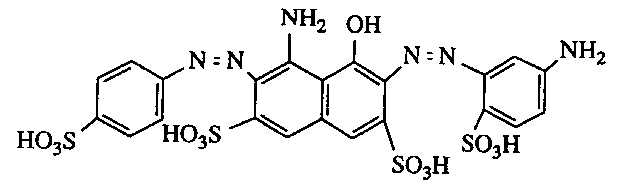 werden in 350 ml Wasser gelöst und der pH-Wert der Lösung auf 7,0 gestellt. Man gibt zu dieser Lösung 0,06 Mol der im Beispiel 1A hergestellten Kondensationslösung. Über 6-7 Stunden wird bei pH 7-8 und 20-25°C umgesetzt.

Nach abgeschlossener Kondensation wird der Farbstoff nach Pufferung auf pH 6 entweder direkt durch Sprühtrocknung oder durch Aussalzen, Absaugen und Vakuumtrocknung bei 40°C isoliert. Der Farbstoff hat die Formel 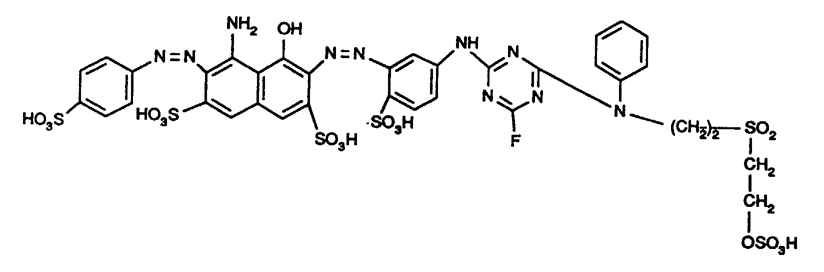 und färbt Baumwolle nach den für Reaktivfarbstoffe üblichen Färbetechniken sehr ergiebig in marineblauen Tönen.

Weitere ähnliche Cellulosefasern marineblau bis schwarz färbende Reaktivfarbstoffe werden erhalten, wenn man die in nachfolgender Zusammenstellung angegebenen Aminodisazokomponenten der allgemeinen Formel 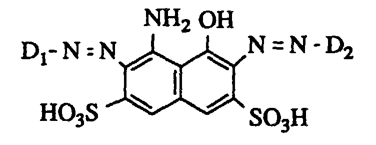 mit den Trihalogentriazinen und Verbindungen der Formel (V) kondensiert.

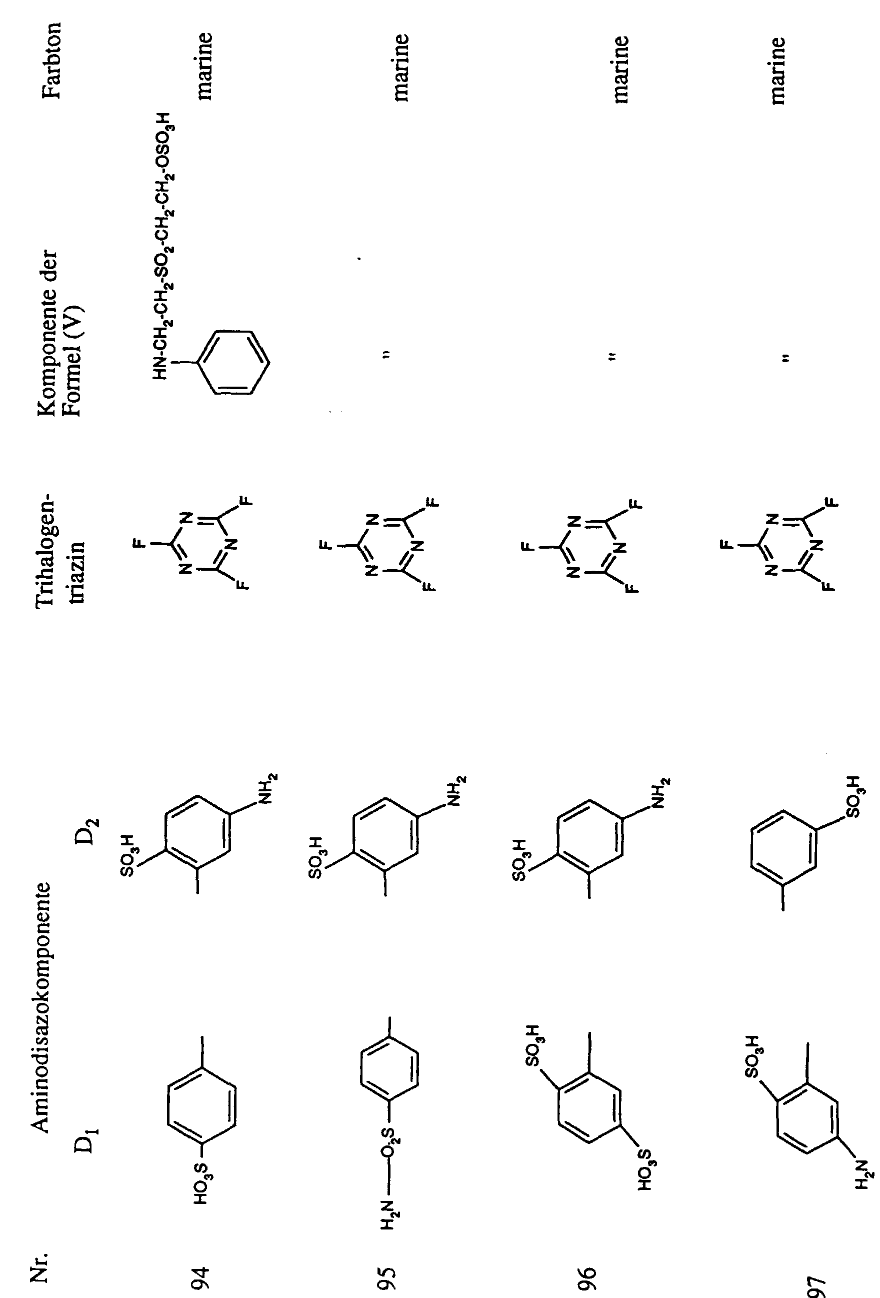

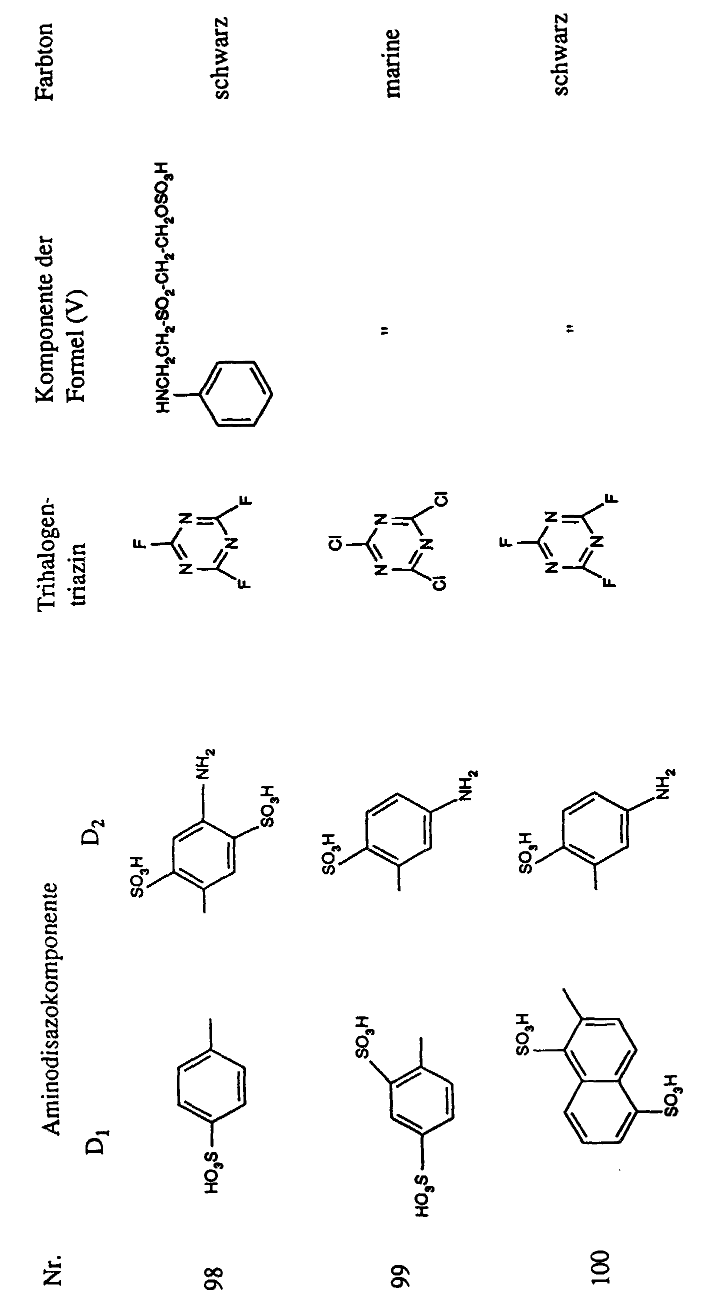

### Beispiel 101

50 mmole der Kupferkomplexverbindung der Formel 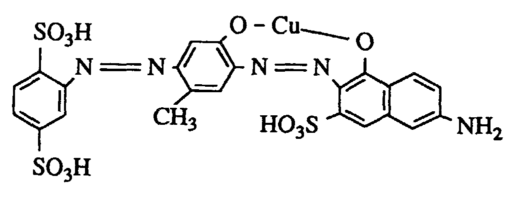 werden in 600 ml Wasser bei pH 6,5 gelöst. Zu der Farbstofflösung werden 60 mmol der in Beispiel 1A hergestellten Verbindung der Formel 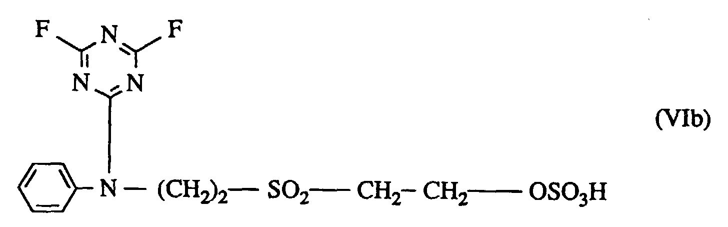 zugegeben und bei 20-25°C und pH 6,5-7 wird die Kondensation durchgeführt. Nach beendeter Umsetzung wird der Farbstoff der Formel 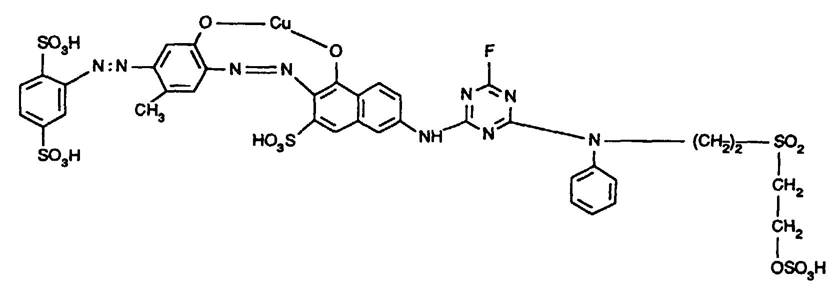 ausgesalzen, isoliert und nach Pufferung auf pH 6 im Vakuum bei 45° getrocknet.

Das Produkt färbt Cellulosefasern nach den für Reaktivfarbstoffe üblichen Farbtechniken mit sehr guten Fixierausbeute in marineblauen Tönen.

Weitere Reaktivfarbstoffe, die Baumwolle nach den üblichen Färbetechniken sehr ergiebig färben, erhält man, wenn man die in nachfolgender Zusammenstellung angegebenen, bekannten Kupferkomplexverbindungen nach den in Beispielen 101 dargelegten Verfahrensweisen mit den Trihalogentriazinen und den Komponenten der Formel (V) kondensiert.

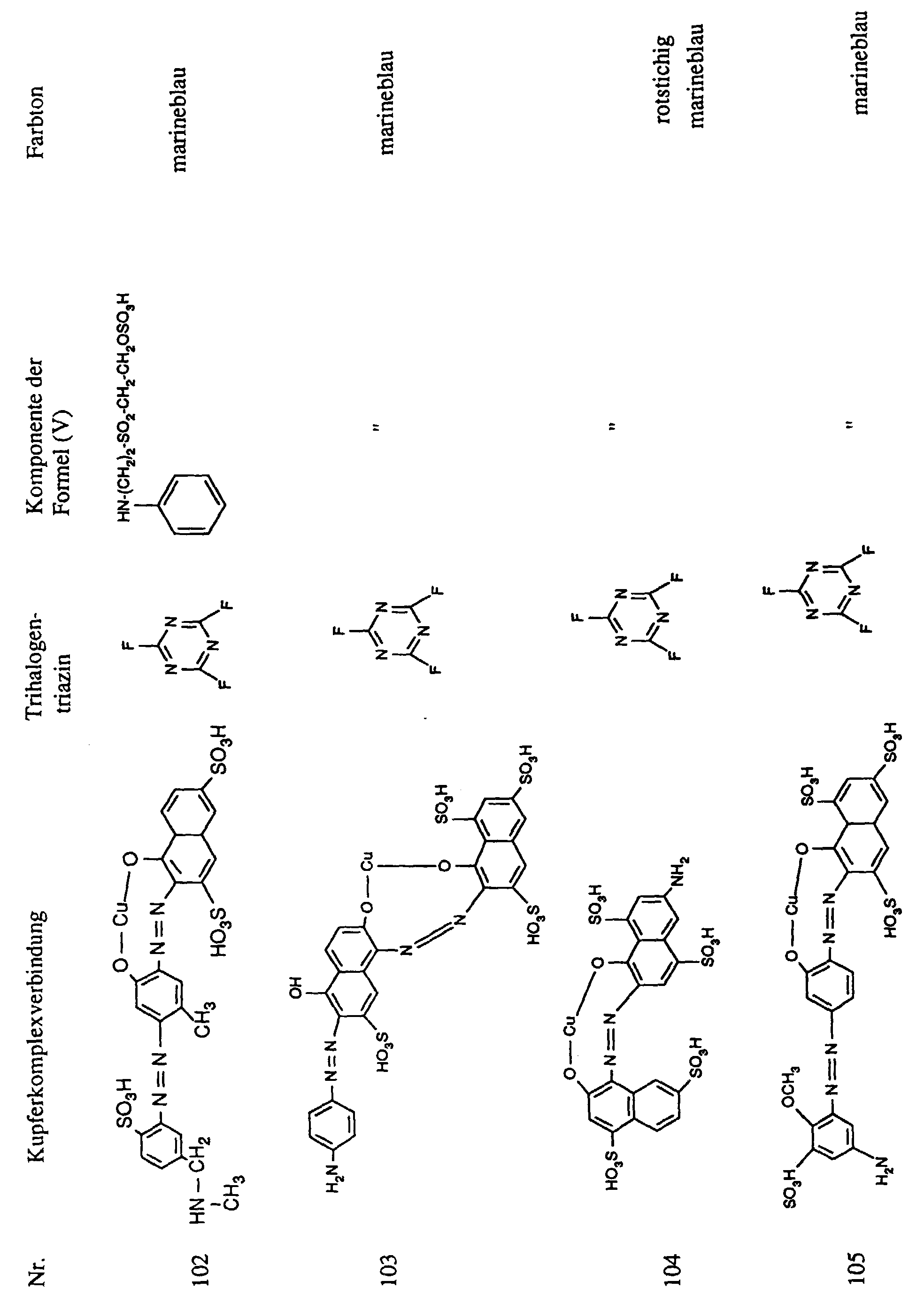

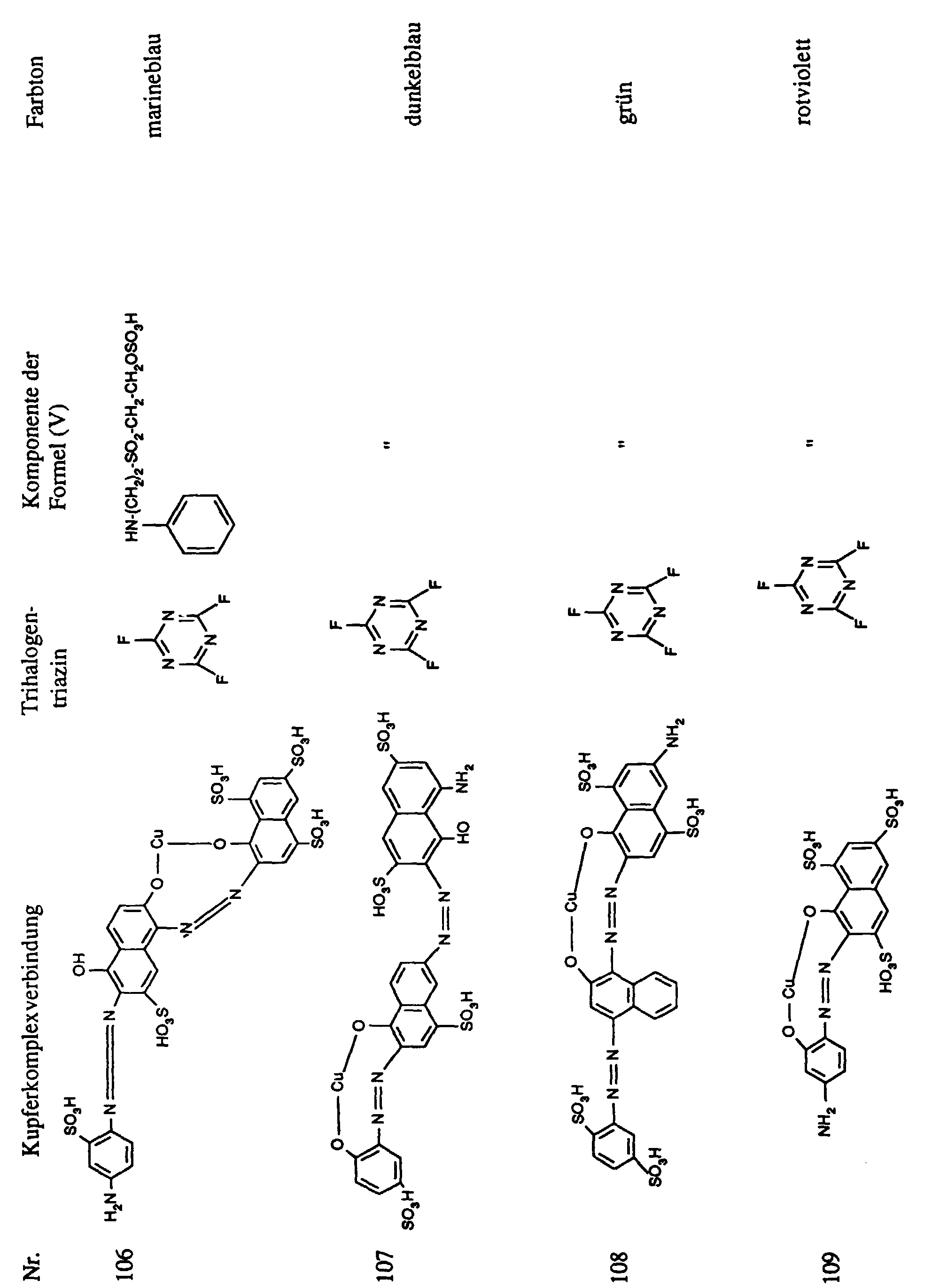

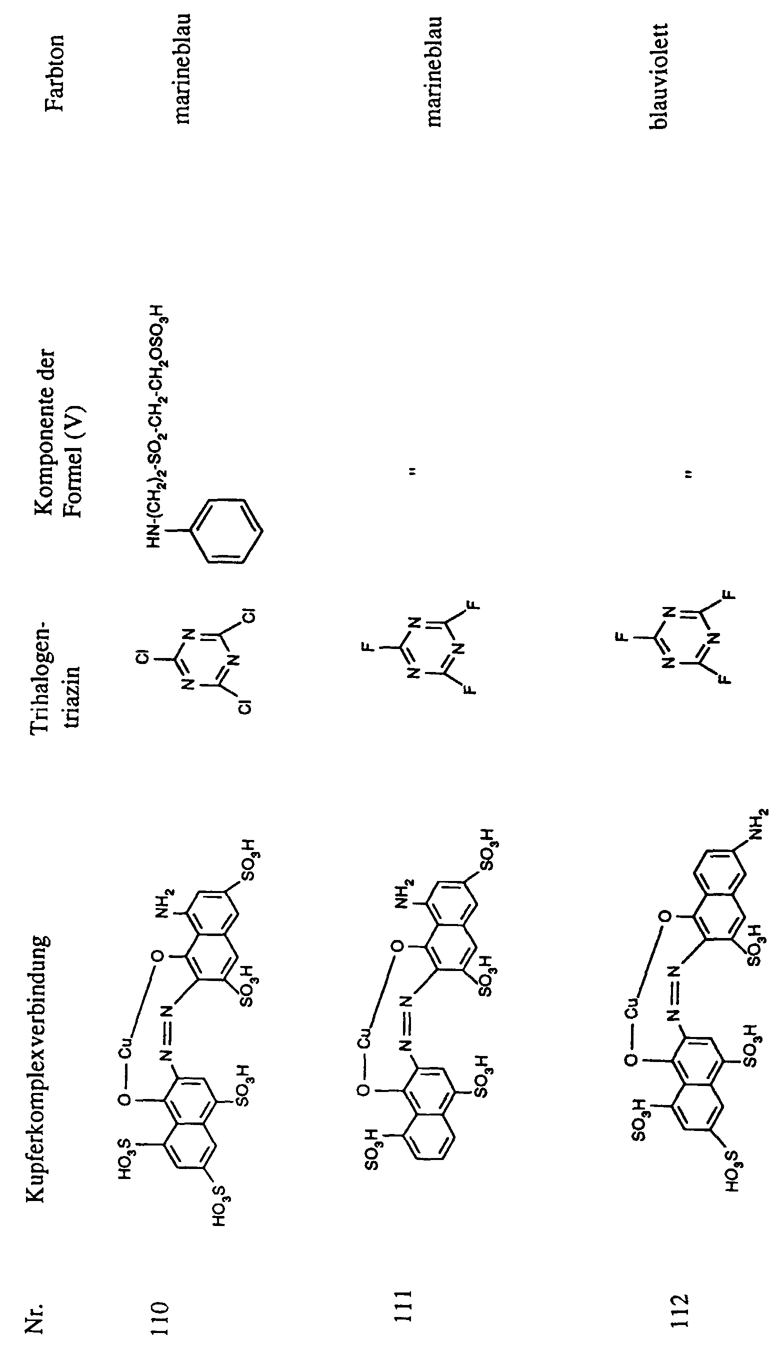

Weitere interessante Farbstoffe z.B. aus der ortho-Disazometallkomplexreihe sind:

### Beispiel 113

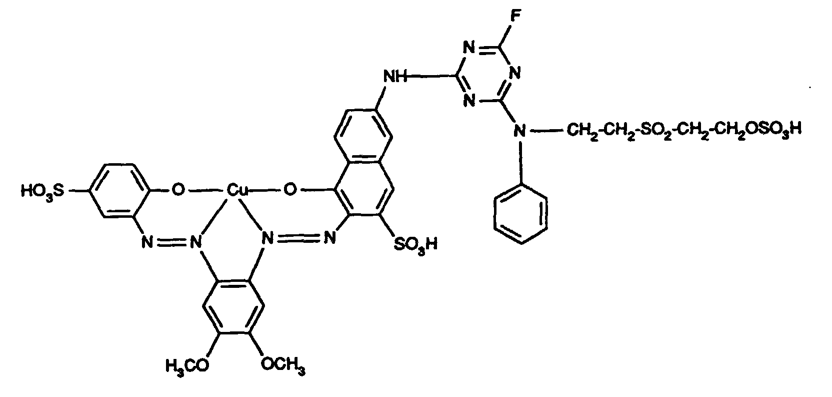

der Baumwolle in oliven Farbtönen färbt, oder aus der ortho-Aminoazometallkomplexreihe z.B. der Farbstoff der Formel

### Beispiel 114

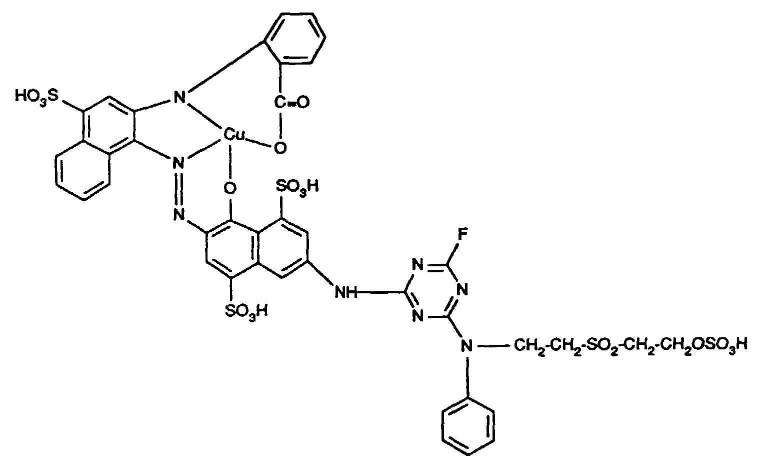

der Baumwolle in grünen Tönen färbt.

### Beispiel 115

3 Mol 2-Phenylaminoethanol der Formel 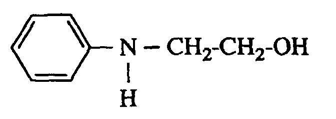 wurden innerhalb von 1 Stunde bei 20-30°C in 1400 Teile Schwefelsäure (100 %ig) eingetropft. Es wurde 2 Stunden nachgerührt. Die so erhaltene Schmelze trug man bei 0°C auf Eis aus und rührte 1 Stunde bei 0-5°C nach. Das ausgefallene Produkt wurde isoliert. Man erhielt ca. 1500 Teile des Esters als hellbeige Paste, der folgende Formel zukommt:

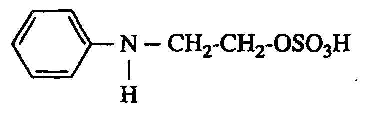

Die gesamte Paste wurde in 2500 Teile Wasser suspendiert und mit 50 %iger Kaliumhydroxidlösung auf pH 7 gestellt. Bei 60°C tropfte man in 20 Minuten 3 Mol Mercaptoethanol und anschließend 3 Mol Kaliumhydroxid (50 %ige Lösung) zu. Man kochte 8 Stunden am Rückfluß. Das entstandene Öl wurde abgetrennt. Die wäßrige Phase wurde mit Ether ausgeschüttelt. Nach Abdestillieren des Ethers wurde das Produkt vereinigt. Man erhielt 460 Teile der Thioverbindung als Öl mit der Formel 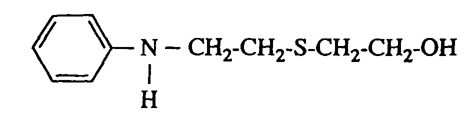 ¹H-NMR (D₆-DMSO)
- δ = 2,58-2,75: ppm (6H, m)
- δ = 3,22: ppm (2H, t)
- δ = 4,80: ppm (1H, s)
- δ = 6,50-6,61: ppm (3H, m)
- δ = 7,05: ppm (2H, d)

### Beispiel 116

460 Teile des Thioethers wurden in 1000 Teilen Aceton und 1000 Teilen Wasser aufgenommen. Man tropfte in 1 Stunde 2,6 Mol Essigsäureanhydrid zu. Die Temperatur stieg auf ca. 45°C an. Es wurde 2 Stunden nachgerührt und 14 g Natriumwolframat zugesetzt. Dann tropfte man in 1 Stunde 4,9 Mol 35 %iges H₂O₂ zu. Die Temperatur stieg auf 50-55°C an. Das Aceton wurde abdestilliert und die verbleibende Lösung auf 10°C abgekühlt. Das entstandene Produkt fiel aus und wurde isoliert. Man erhielt 1020 Teile einer Paste, der folgende Formel zukommt: 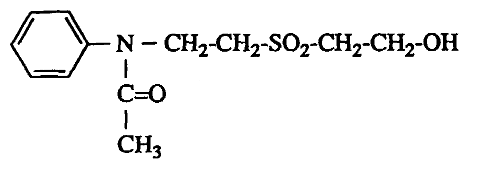 ¹H-NMR (D₆-DMSO)
- δ = 1,77: ppm (1H, s)
- δ = 3,21-3,38: ppm (4H, m)
- δ = 3,76: ppm (2H, q)
- δ = 4,05: ppm (2H, t)
- δ = 5,10: ppm (1H, t)
- δ = 7,38-7,55: ppm (5H, m)

### Beispiel 117

1020 Teile der Paste wurden in 1500 Teilen Wasser suspendiert und mit 300 Teilen 30 %iger Salzsäurelösung versetzt. Man kochte 3 Stunden am Rückfluß. Die Lösung wurde auf 10°C abgekühlt und mit Natronlauge auf pH 9 gestellt. Das erhaltene Öl wurde abgeschieden. Die wäßrige Phase wurde mit Essigsäureethylester ausgeschüttelt. Nach Abdestillieren des Essigsäureethylesters wurden die Fraktionen des entstandenen Öls der Formel 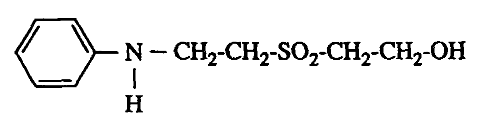 vereinigt und getrocknet. Man erhielt 450 Teile des Sulfons als Öl.
¹H-NMR (D₆-DMSO)
- δ = 3,23-3,55: ppm (6H, m)
- δ = 3,85: ppm (2H, t)
- δ = 5,30: ppm (1H, s)
- δ = 5,78: ppm (1H, t)
- δ = 6,60: ppm (3H, d)
- δ = 7,10: ppm (2H, t)

### Beispiel 118

450 Teile der obigen Substanz wurden innerhalb von 1 Stunde bei 20-30°C in 1800 Teile Schwefelsäure eingetropft. Nach 8 Stunden trug man die Schmelze auf Eis aus und rührte ca. 1 Stunde bei 0-5°C nach. Das hellbeige, kristalline Produkt wurde isoliert. Man erhielt 910 Teile Paste. Die entstandene Verbindung besitzt folgende Formel:

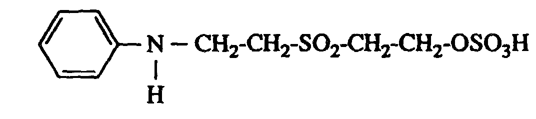

Die Ausbeute beträgt 45-50 % über alle Stufen (Beispiel 115 bis 118).
¹H-NMR (D₆-DMSO)
- δ = 3,43: ppm (2H, t)
- δ = 3,50: ppm (2H, t)
- δ = 3,60: ppm (2H, t)
- δ = 4,11: ppm (2H, t)
- δ = 7,00: ppm (3H, d, d)
- δ = 7,30: ppm (2H, t)
- δ = 8,98: ppm (2H, s)

### Beispiel 119

A) 0,15 Mol der Verbindung der Formel 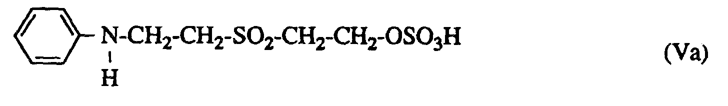 wurden in 100 Teilen Wasser und 100 Teilen Eis verrührt und neutral gelöst. Innerhalb von 10 Minuten tropfte man bei O°C und pH 4-4,5 0,165 Mol 2,4,6-Trifluor-1,3,5-triazin zu. Man erhält ca. 250 Teile einer wässrigen Kondensationslösung mit folgender Struktur: 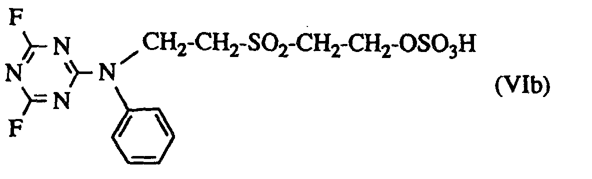
B) 0,1 Mol 1-Amino-8-hydroxy-3,6-naphthalindisulfonsäure wurden in 150 Teilen Wasser suspendiert und mit Lithiumhydroxydlösung neutral gelöst. Man gab 0,1 Mol der obigen Kondensationslösung hinzu. Mit Lithiumcarbonat wurde bei 20°C ein pH-Wert von 4-4,5 gehalten. Nach 8 Stunden war die Reaktion beendet. Es lag eine lösung vor.
C) 0,1 Mol 1-Sulfo-2-naphthylamin-6-β-sulfatoethylsulfon wurden in geeigneter Weise diazotiert und bei pH 7,5-8,5 auf das H-Säure-Kondensationsprodukt gekuppelt. Der Farbstoff wurde durch Zugabe von Kaliumchlorid ausgesalzen und isoliert Nach Trocknung erhielt man ca. 35 g eines salzhaltigen Farbstoffkupplers, dem die Struktur 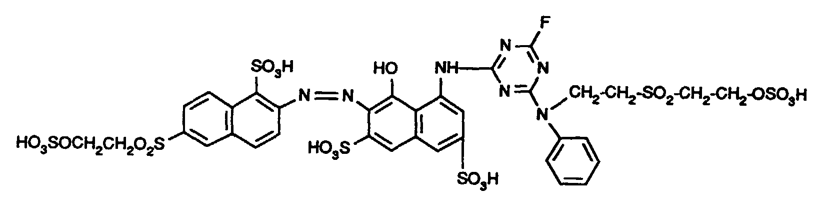 zukommt (λₘₐₓ = 516 und 538 nm (H₂O)) und das Baumwolle nach den für Reaktivfarbstoffe üblichen Färbe- oder Druckverfahren in roten Farbtönen färbt.

### Beispiel 120

0,1 Mol 4-(β-Sulfatoethylsulfonyl)anilin wurden in 200 Teilen Wasser und 100 Teilen Eis bei 0°C suspendiert. Man gab 28 Teile konzentrierte Salzsäure zu. Innerhalb von 15 Minuten tropfte man eine Lösung von 7 Teilen Natriumnitrit in 70 Teilen Wasser zu. Nach 30-minütigem Nachrühren war die Diazotierung beendet. Es resultierte eine hellgelbe Suspension. Der Nitritüberschuß wurde mit Amidosulfonsäure zerstört.

Diese Suspension wurde nun über einen Zeitraum von 15-20 Minuten zu 0,1 Mol des H-Säure-Kondensationsproduktes aus Beispiel 119 B zudosiert. Gekuppelt wurde bei 20°C und einem pH-Wert von 7-8. Der Farbstoff wurde durch Zugabe von Ethanol ausgefällt und isoliert. Nach Trocknung erhielt man ca. 40 g eines Farbstoffpulvers, dem die Struktur 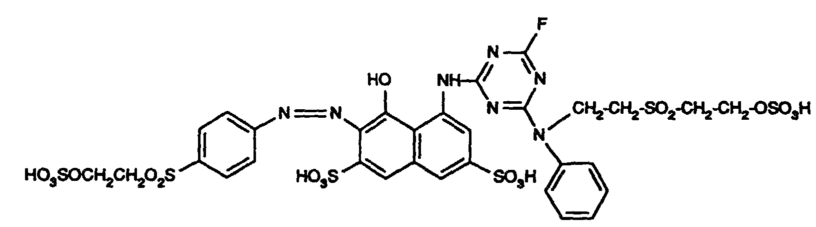 zukommt (λₘₐₓ= 517 nm (H₂O)) und das Baumwolle nach den für Reaktivfarbstoffe üblichen Färbe- und Druckverfahren in roten Farbtönen färbt.

Weitere rote Reaktivfarbstoffe erhält man durch Kondensation der folgenden Komponenten.

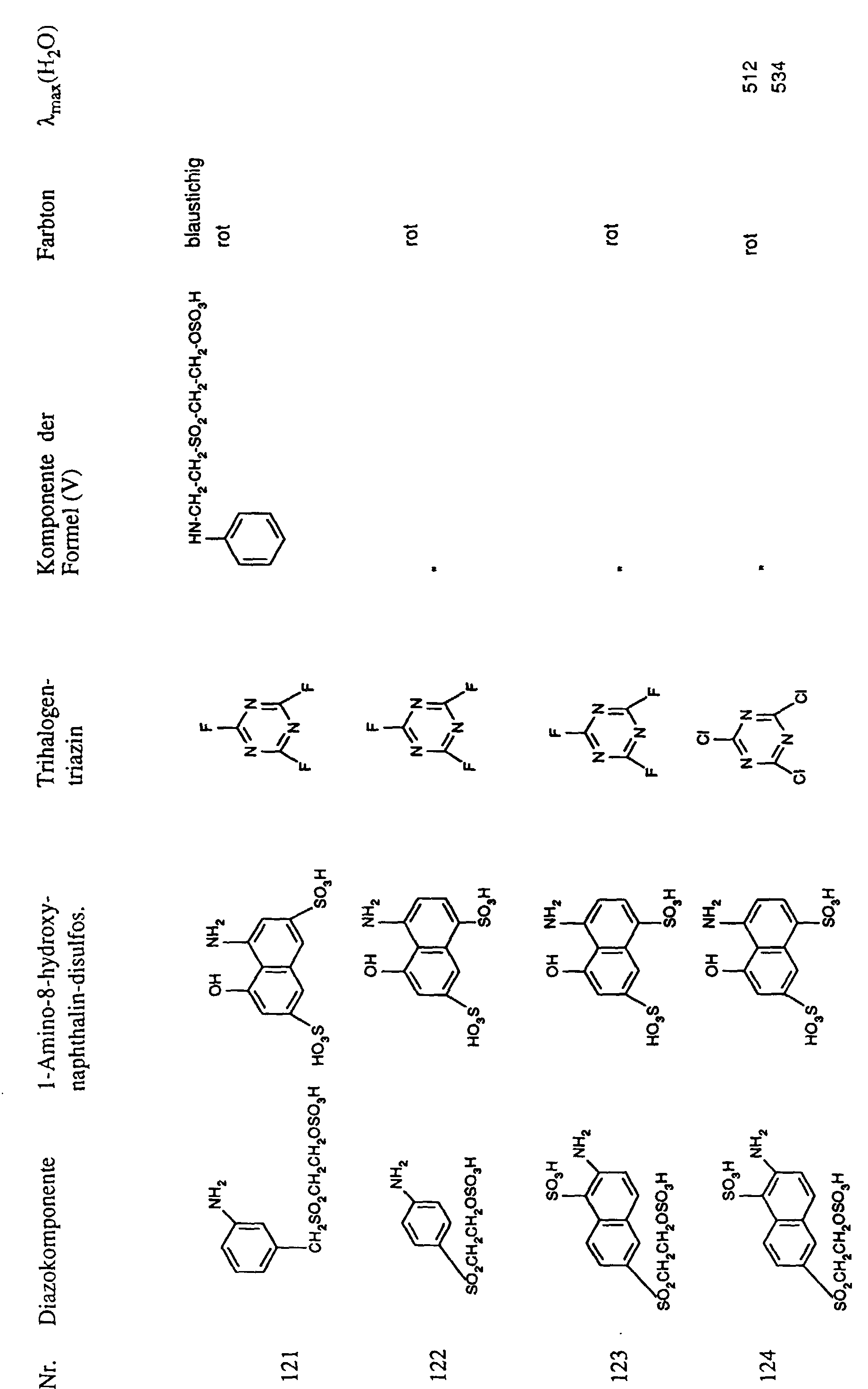

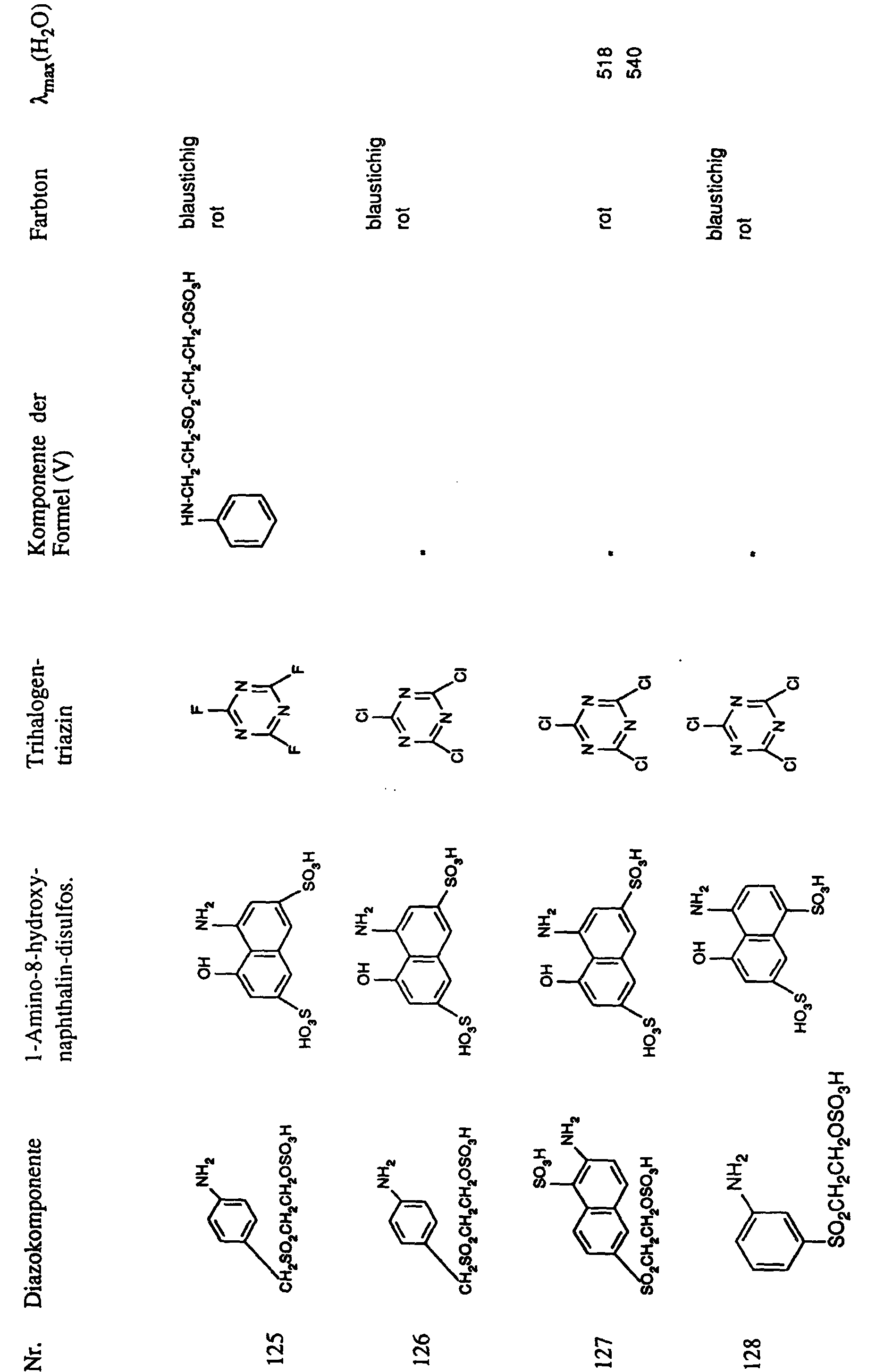

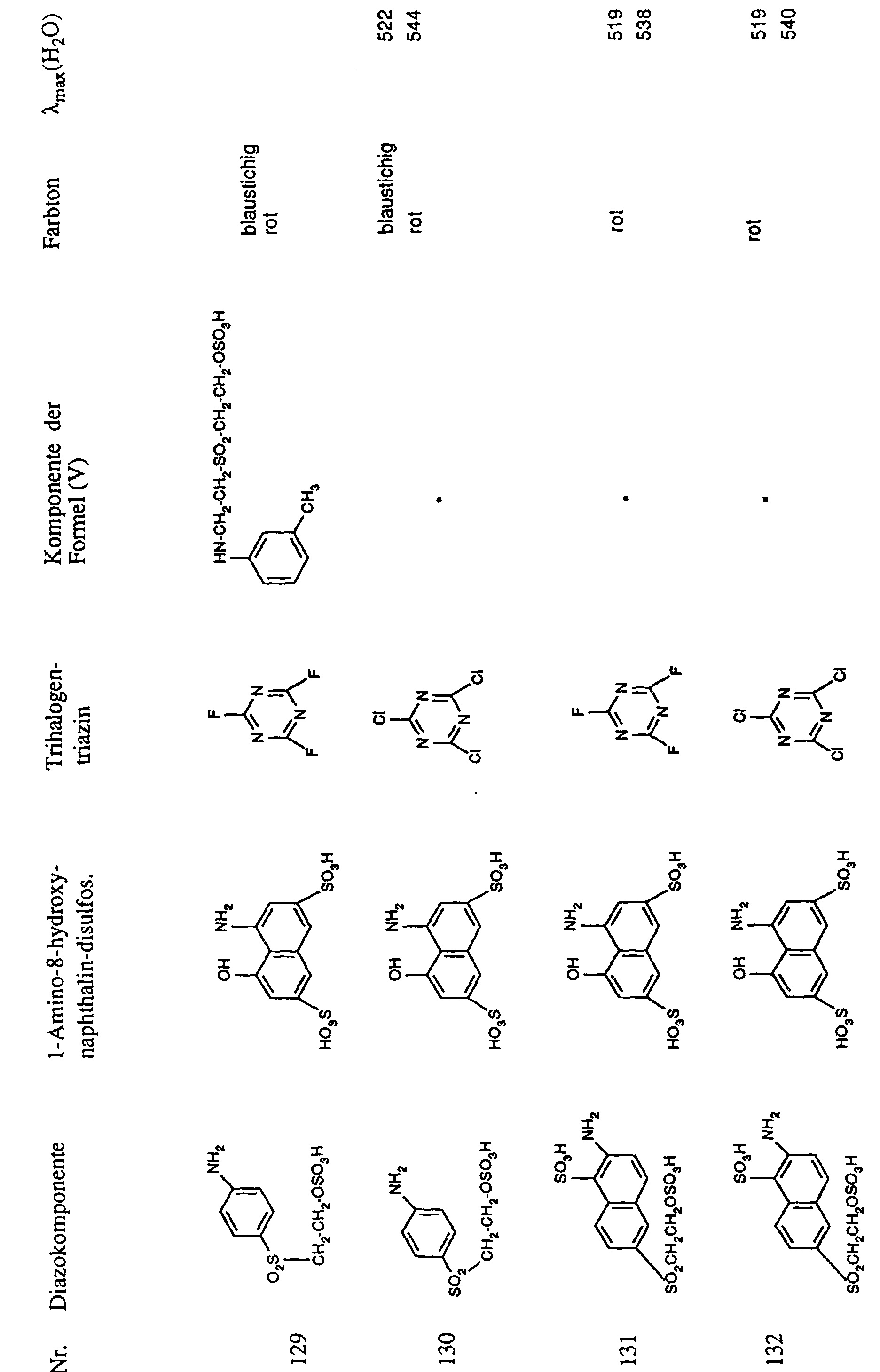

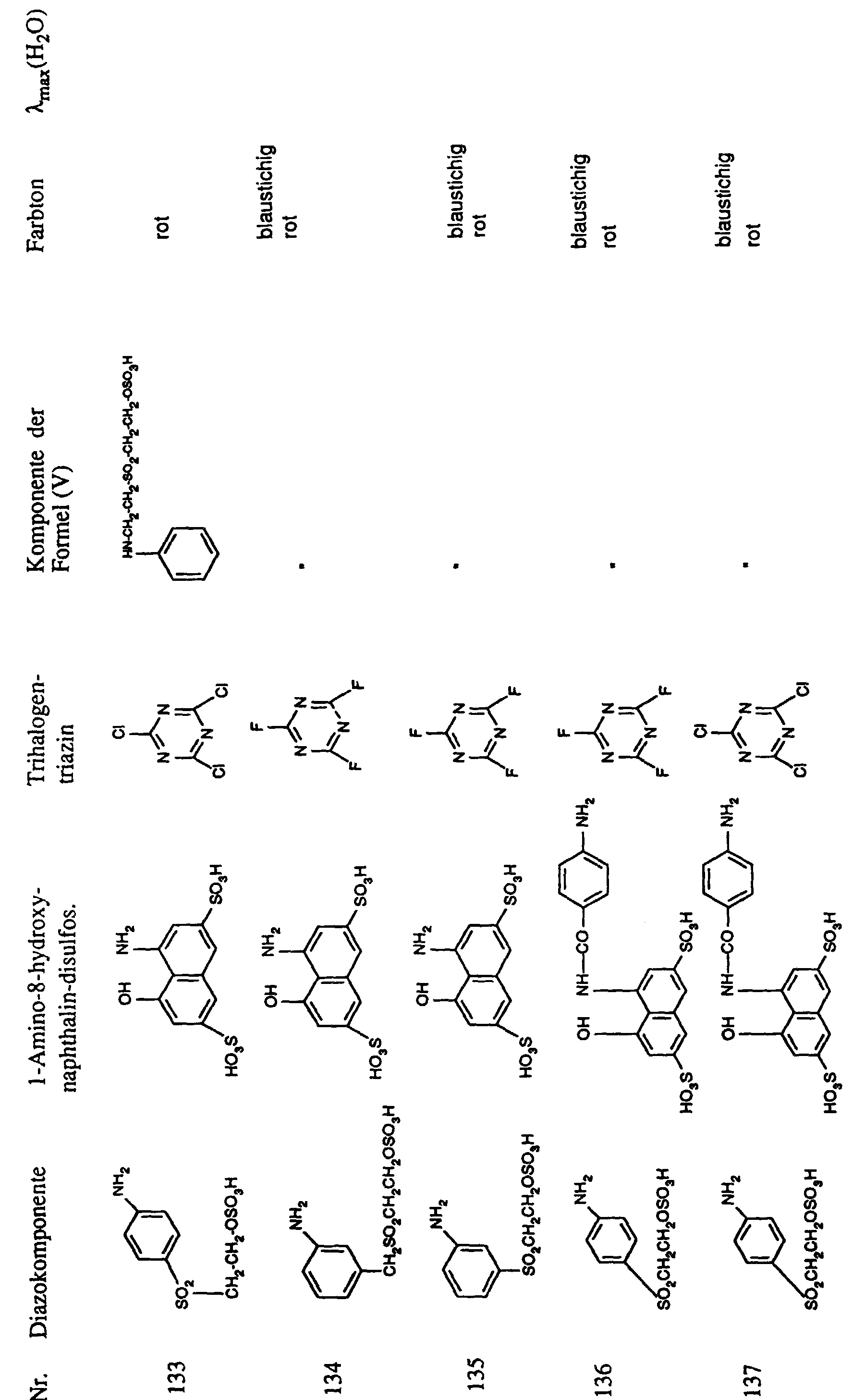

### Beispiel 138

0,1 Mol der Monoazoverbindung mit der Formel 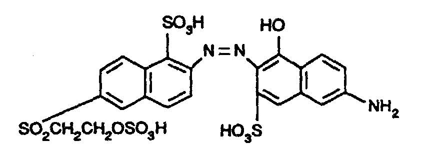 wurden in ca. 500 Teilen Wasser neutral gelöst. Man gab 0,15 Mol der in Beispiel 119 hergestellten Kondensationslösung zu. Mit Sodalösung wurde bei 20°C ein pH-Wert von 7-8 gehalten. Nach 2 Stunden war die Reaktion zum größten Teil beendet. Der Farbstoff wurde durch Zugabe von Kaliumchlorid ausgesalzen, isoliert und getrocknet Man erhielt ca. 82 g eines salzhaltigen Farbstoffpulvers, dem die Struktur 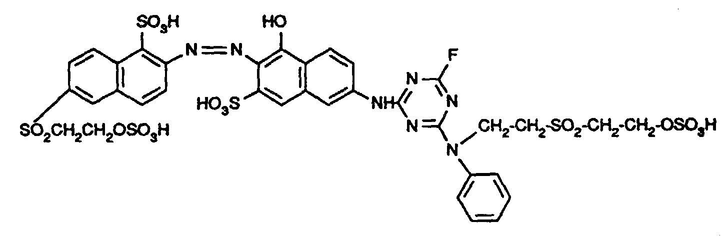 zukommt (λₘₐₓ = 492 nm (H₂O)) und das Baumwolle nach den für Reaktivfarbstoffe üblichen Färbe- oder Druckverfahren in orangen Farbtönen färbt.

### Beispiel 139

0,1 Mol (4-Aminophenyl-β-sulfatoethyl)sulfon wurden in 100 Teilen Wasser und 100 Teilen Eis neutral gelöst. Man gab 28 Teile konzentrierter Salzsäure zu. Innerhalb von 15 Minuten tropfte man eine Lösung von 7 Teilen Natriumnitrit in 70 Teilen Wasser zu. Nach 30-minütigem Nachrühren war die Diazotierung beendet. Der Nitritüberschuß wurde mit Amidosulfonsäure zerstört

0,12 Mol 7-Amino-4-hydroxy-naphthalinsulfonsäure-2 wurden in 300 Teilen Wasser suspendiert und mit 10 %iger Lithiumhydroxidlösung bei pH = 6-6,5 gelöst. Man gab 0,18 Mol der in Beispiel 119 A hergestellten Kondensationslösung zu. Mit Sodalösung wurde bei 20°C ein pH-Wert von 4-5 gehalten. Nach 2 Stunden war die Kondensation beendet Man erhielt 900 Teile einer Lösung. Das Produkt wurde mit 180 Teilen Kaliumchlorid ausgesalzen und isoliert Man erhielt ca. 220 Teile einer grauen Paste. Die Paste wurde in 600 Teilen Wasser gelöst. Man tropfte die obige Diazotierungsmischung hinzu und hielt gleichzeitig durch Zugabe von NaHCO₃ einen pH-Wert von 6-6,5. Anschließend stellte man mit Sodalösung einen pH-Wert von 7,5-8 ein. Die Lösung rührte über Nacht.

Der Farbstoff wurde durch Zugabe von Natriumchlorid ausgesalzen und isoliert. Nach Trocknung erhielt man ca. 105 g eines salzhaltigen Farbstoffpulvers, dem die Struktur 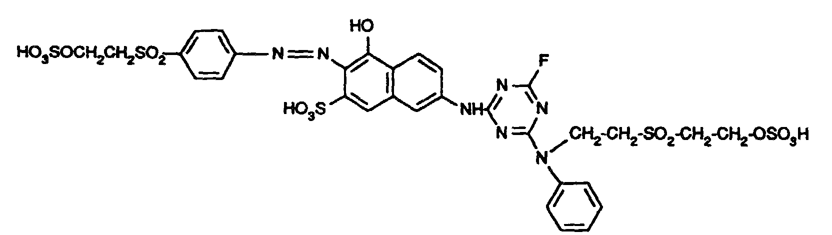 zukommt und das Baumwolle nach den für Reaktivfarbstoffe üblichen Färbe- oder Druckverfahren in Scharlachtönen färbt.

Weitere Reaktivfarbstoffe werden durch Kondensation der folgenden Komponenten analog Beispiel 138 oder 139 erhalten.

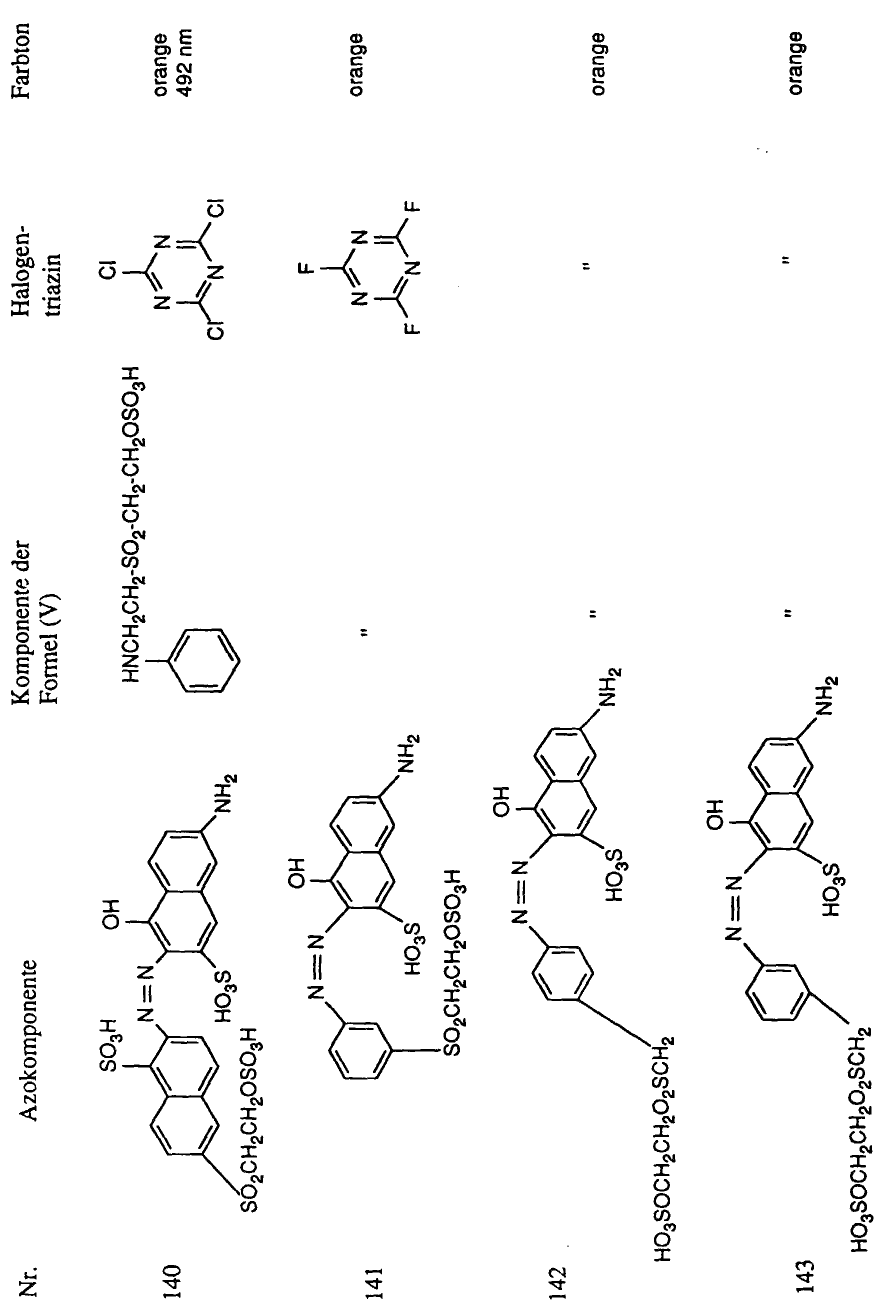

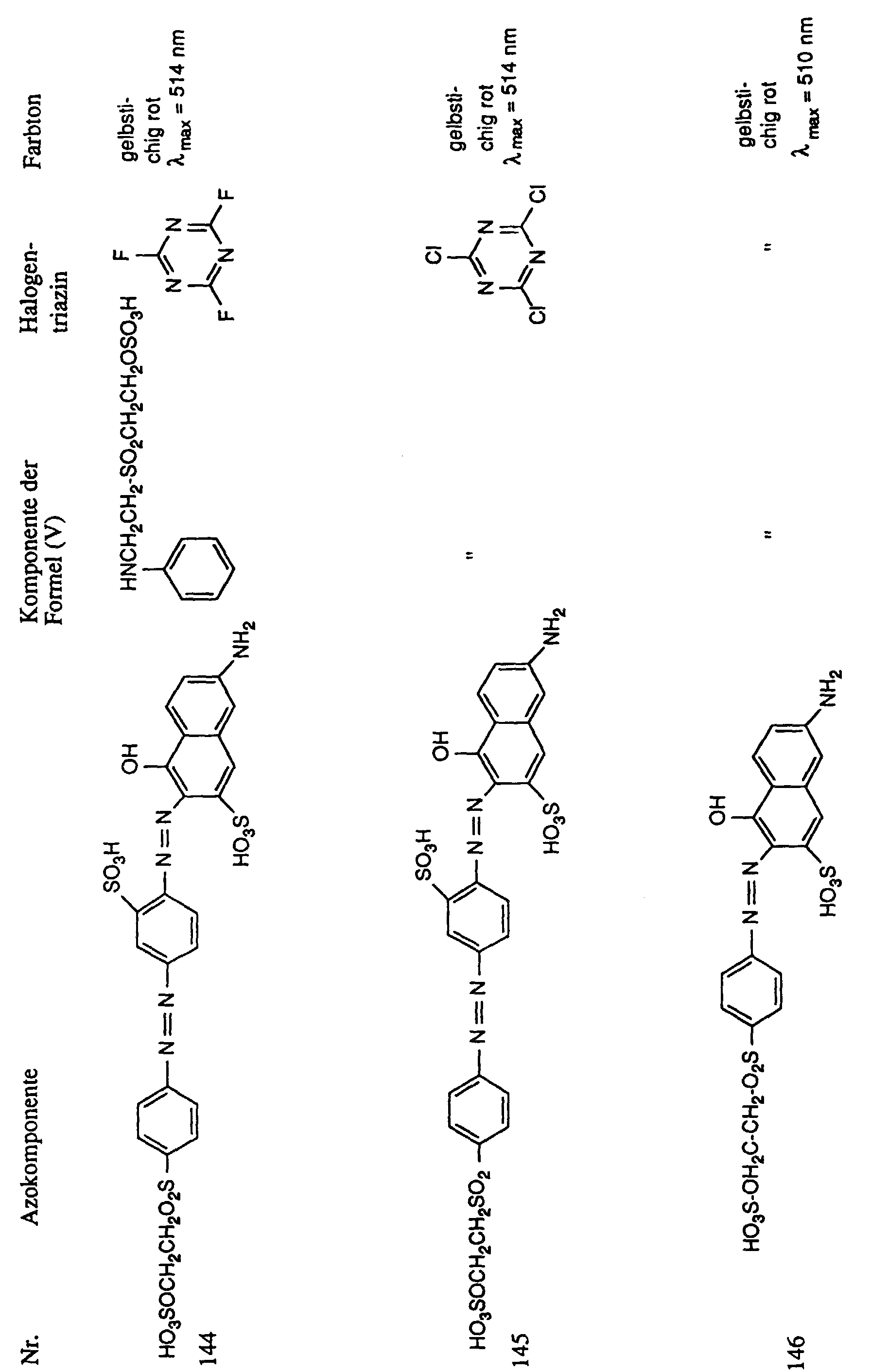

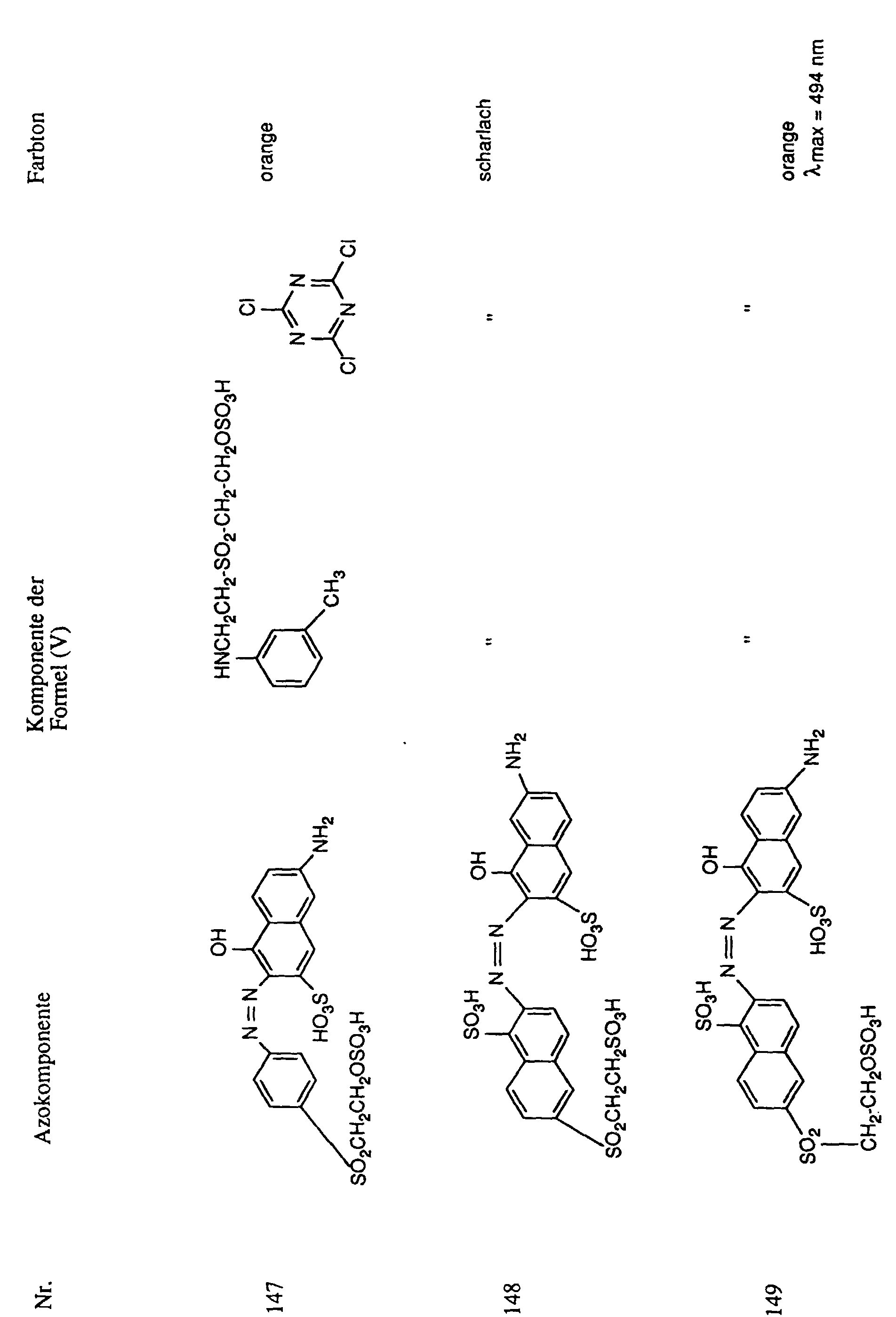

### Beispiel 150

35,2 g der bekannten Verbindung (0,05 Mol) 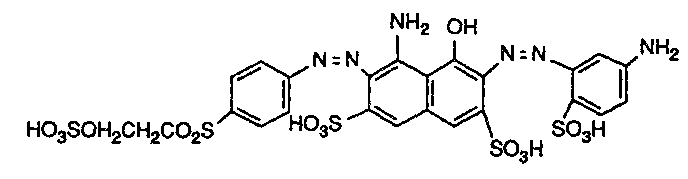 werden in 350 ml Wasser gelöst und der pH-Wert der Lösung auf 7,0 gestellt. Man gibt zu dieser Lösung 0,06 Mol der im Beispiel 119 A hergestellten Kondensationslösung. Über 6-7 Stunden wird bei pH 7-8 und 20-25°C umgesetzt.

Nach abgeschlossener Kondensation wird der Farbstoff nach Pufferung auf pH 6 entweder direkt durch Sprühtrocknung oder durch Aussalzen, Absaugen und Vakuumtrocknung bei 40°C isoliert. Der Farbstoff hat die Formel 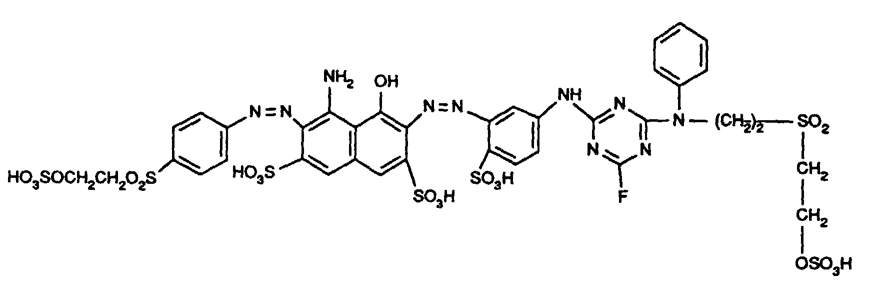 und färbt Baumwolle nach den für Reaktivfarbstoffe üblichen Färbetechniken sehr ergiebig in marineblauen Tönen.

Weitere ähnliche Cellulosefasern marineblau bis schwarz färbende Reaktivfarbstoffe werden erhalten, wenn man die in nachfolgender Zusammenstellung angegebenen Aminodisazokomponenten der allgemeinen Formel 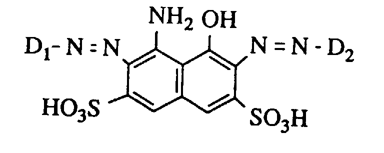 mit den Trihalogentriazinen und Verbindungen der Formel (V) kondensiert.

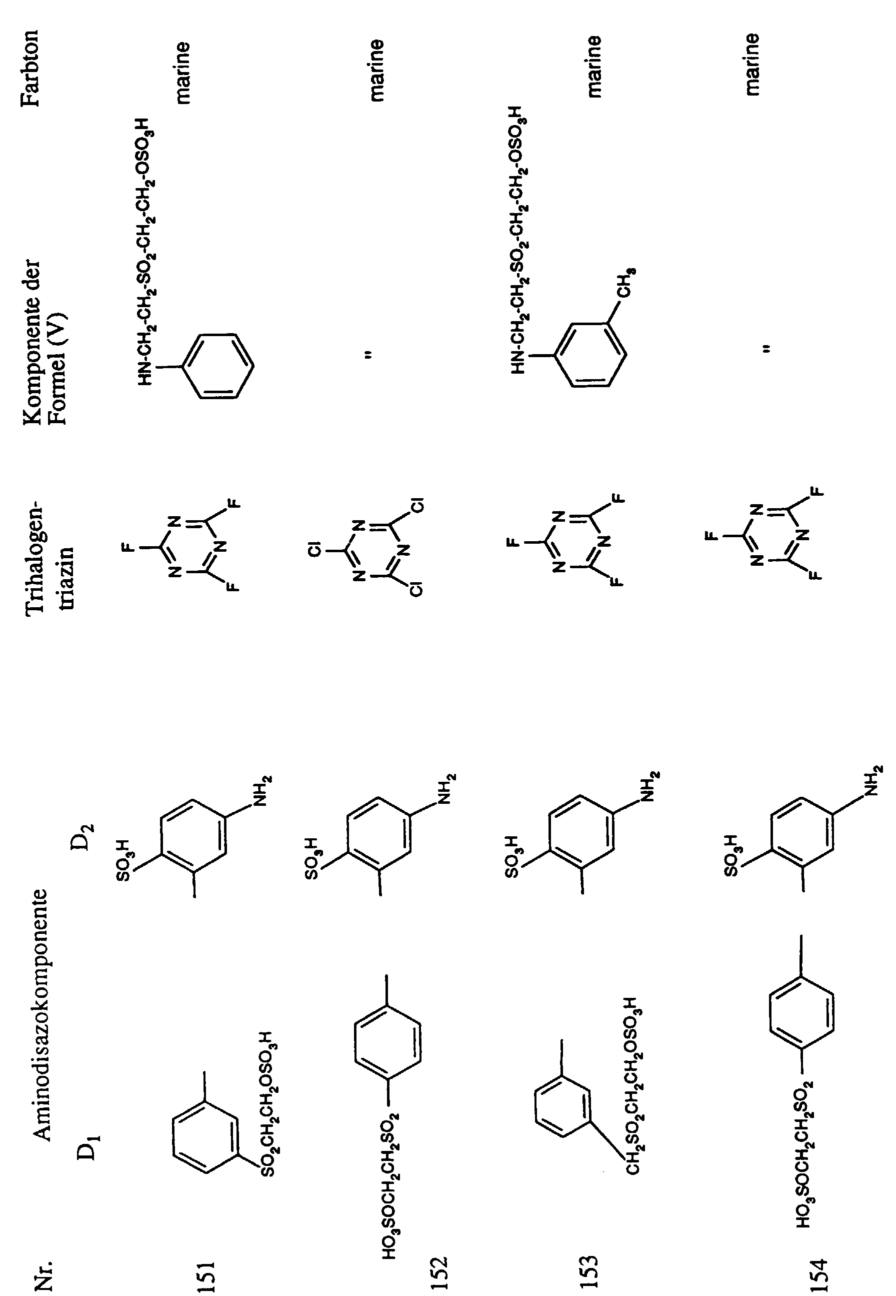

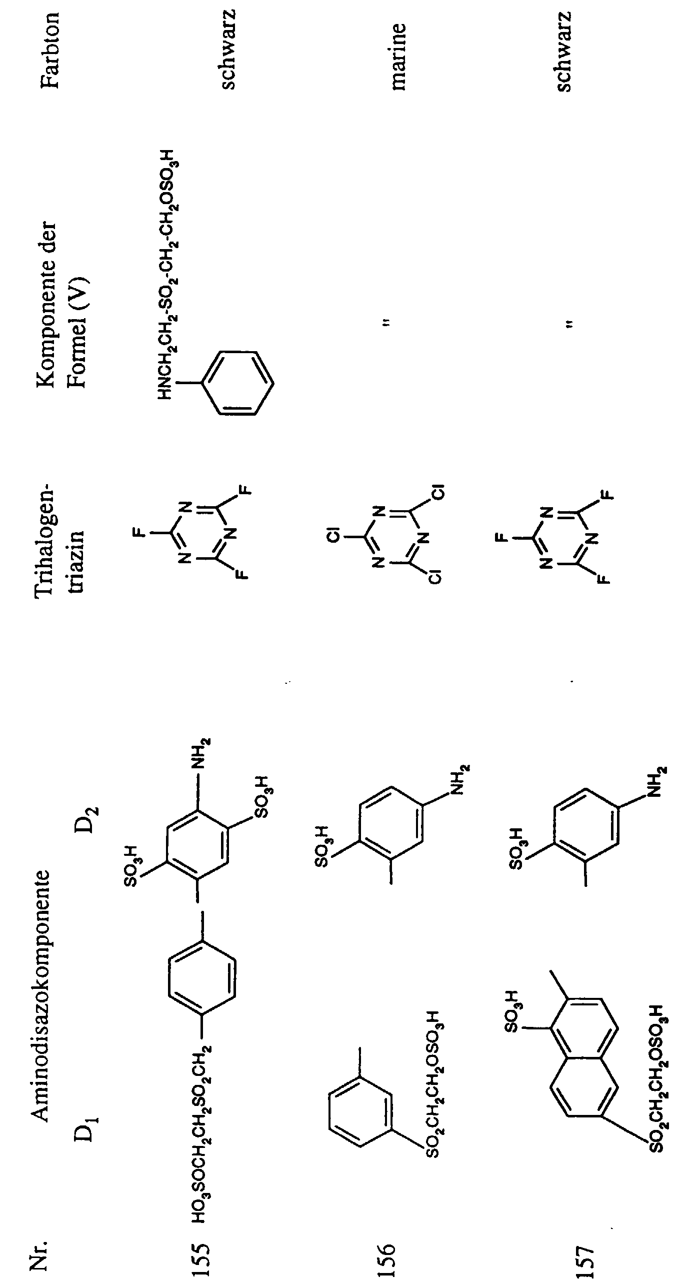

## Patentansprüche

1. Reaktivfarbstoffe der Formel 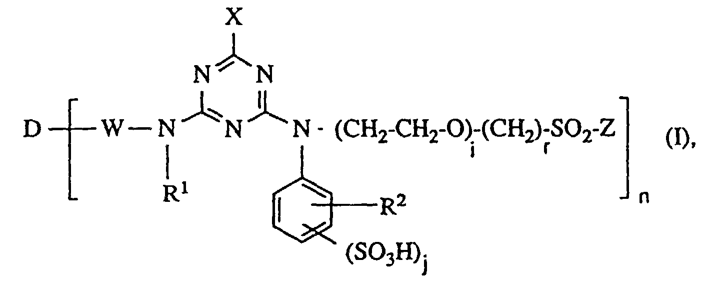 worin
D Rest eines organischen Farbstoffes aus der Monoazo, Polyazo- oder Metallkomplexazoreihe,
W direkte Bindung oder Brückenglied,
R¹ H, C₁-C₄-Alkyl, das durch OR, OSO₃H, SO₃H, COOR oder Halogen substituiert sein kann.
R H, CH₃ oder C₂H_{5,}
R² H, C₁-C₄-Alkyl, Cl, Br, C₁-C₄-Alkoxy oder COOH,
i 0 oder 1,
j 0, 1 oder 2,
r 2 oder 3, insbesondere 2,
X F, Cl oder Br,
Z -CH=CH₂, -CH₂-CH₂-OSO₃H, -CH₂-CH₂-S₂O₃H, -CH₂-CH₂-O-CO-CH₃, -CH₂-CH₂-OPO₃H₂, -CH₂-CH₂-OH,
n 1 oder 2 bedeuten.

2. Reaktivfarbstoffe gemäß Anspruch 1, dadurch gekennzeichnet, daß sie folgender Formel entsprechen 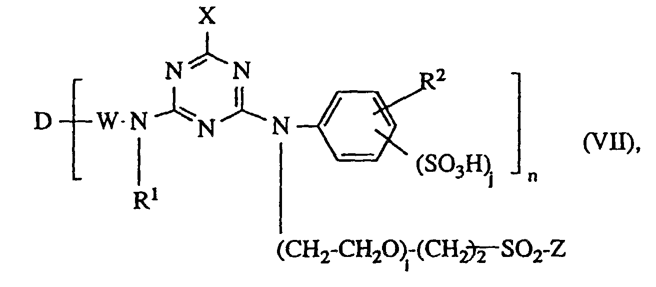 worin
D Rest eines organischen Farbstoffes,
aus der Monoazo-, Polyazo-, Metallkomplexazoreihe ist, der keine weiteren faserreaktiven Gruppen enthält,
R¹, R² unabhängig voneinander H, CH₃ oder C₂H₅ sind,
W direkte Bindung oder Brückenglied,
i 0 oder 1,
j 0 oder 1,
X F oder Cl,
Z CH₂-CH₂-OSO₃H oder CH=CH₂ und
n 1 oder 2 ist.

3. Reaktivfarbstoffe gemäß wenigstens einem der vorhergehenden Ansprüche, dadurch gekennzeichnet, daß sie folgender Formel entsprechen 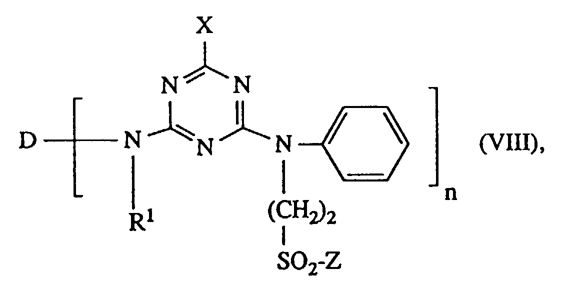 worin
D Rest eines organischen Farbstoffes,
aus der Monoazo-, Polyazo, Metallkomplexazoreihe ist, der keine weiteren faserreaktiven Gruppen enthält,
X F,
Z -CH₂-CH₂-OSO₃H,
R¹ H,
n 1 ist.

4. Reaktivfarbstoffe gemäß wenigstens einem der vorhergehenden Ansprüche, dadurch gekennzeichnet, daß sie einer der folgenden Formeln entsprechen, worin
B einen Rest der Formel 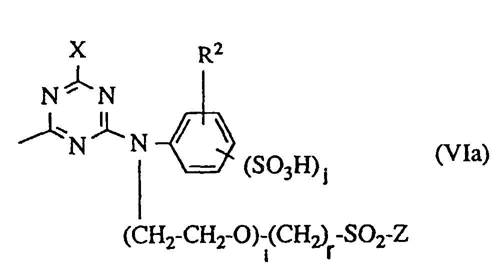
beinhaltet und X, i, R², j, r und Z die in Anspruch 1 angegebene Bedeutung haben: 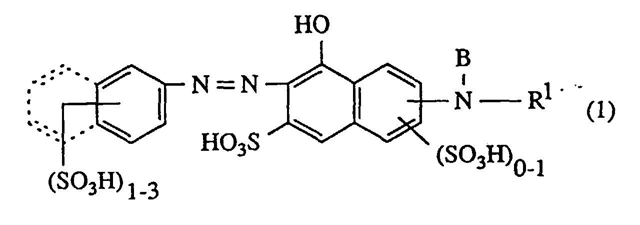 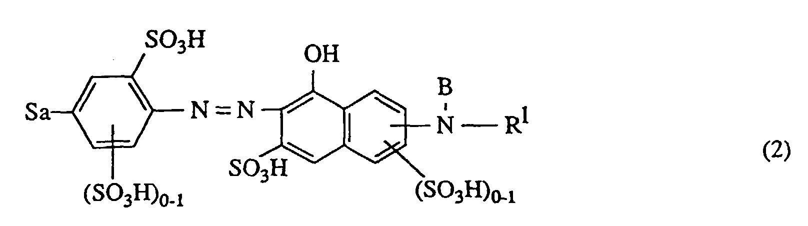 mit Sa = OCH₃ oder OC₂H₅ 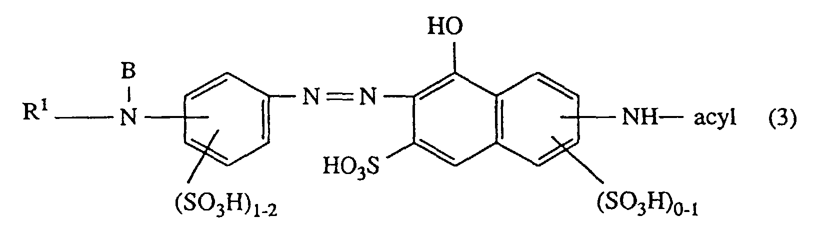 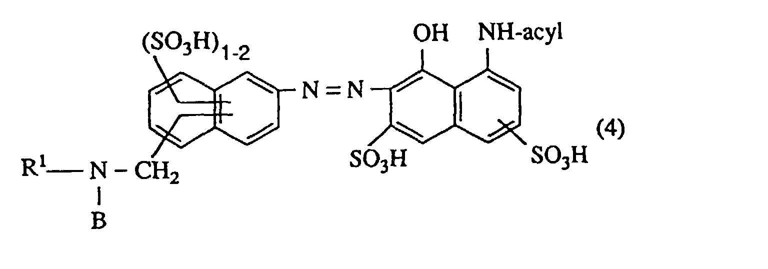 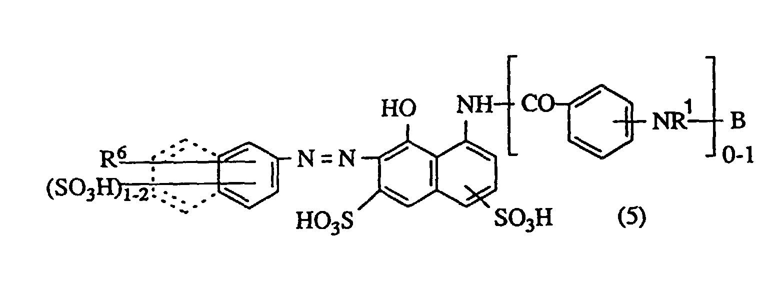 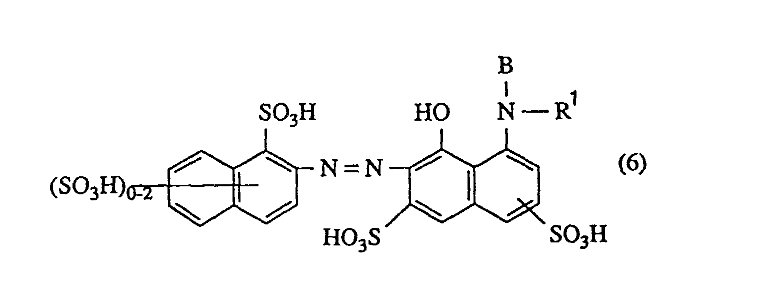 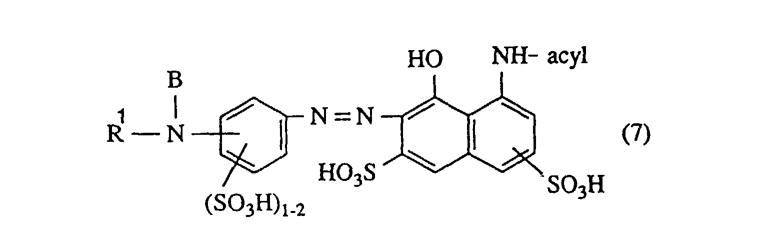 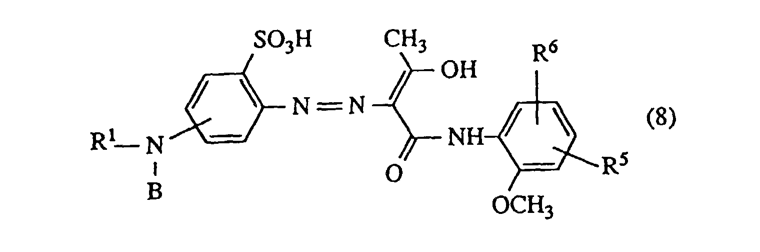 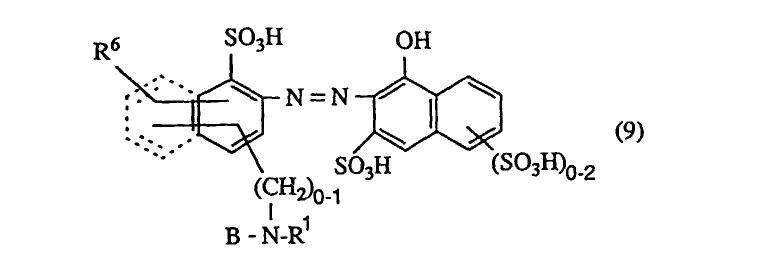 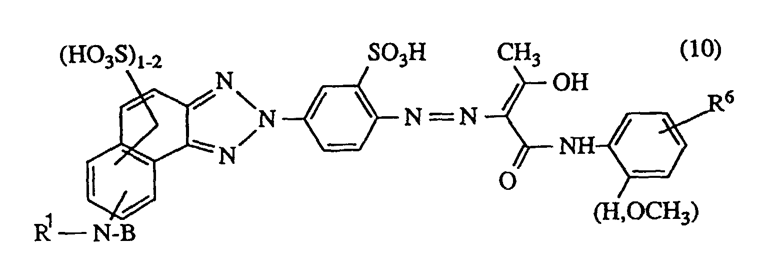 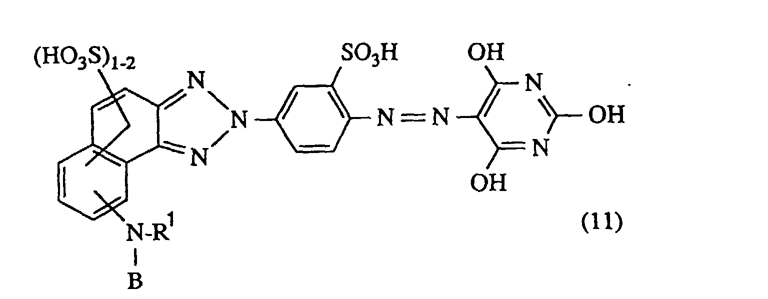 Metallkomplexe von Farbstoffen der Formeln (12) bis (42): 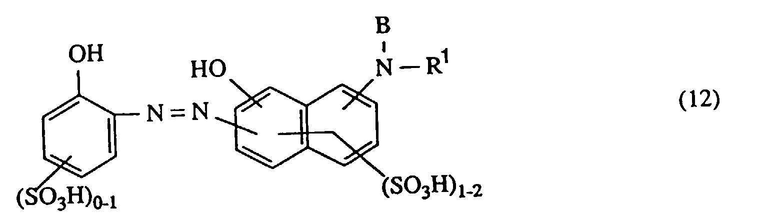 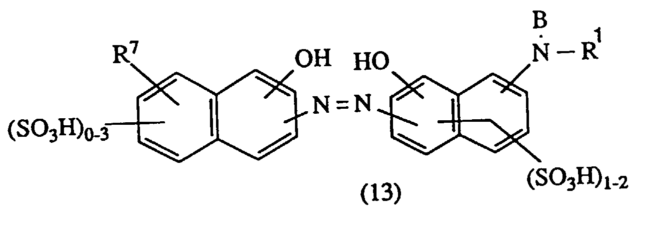 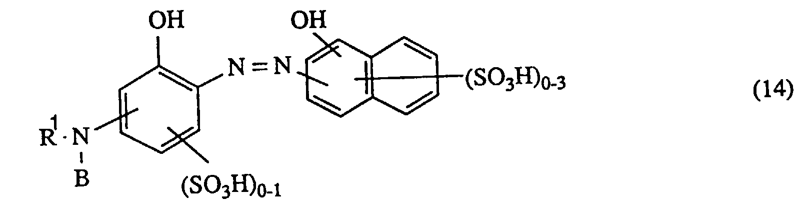 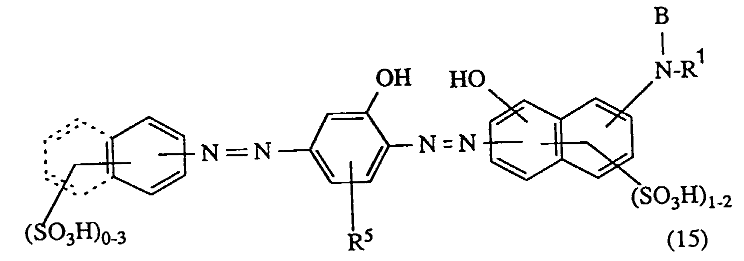 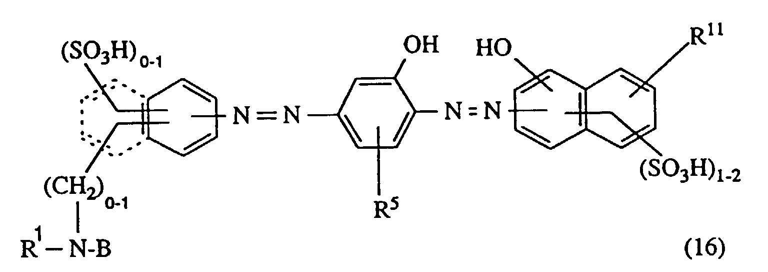 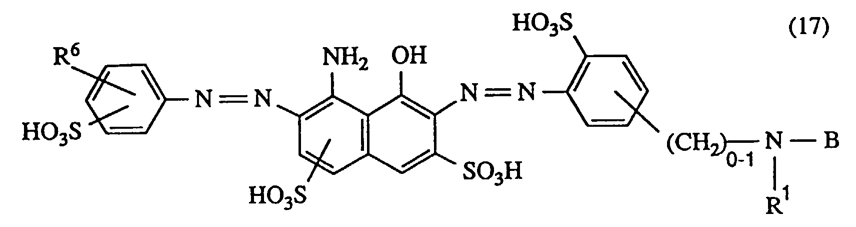 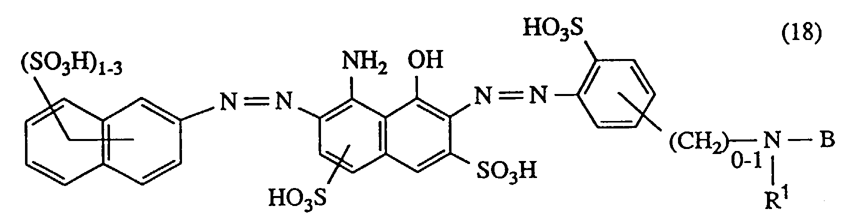 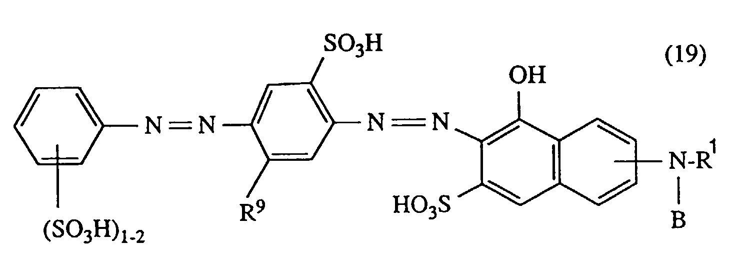 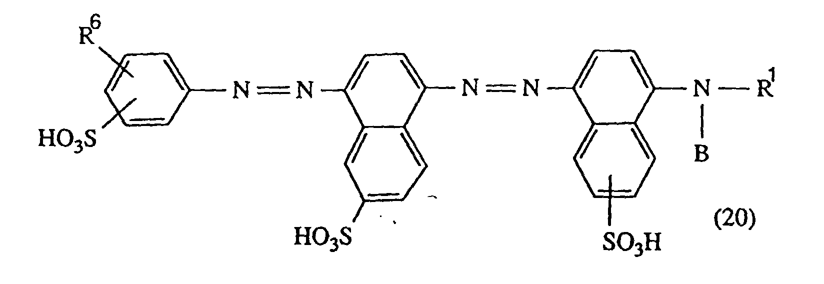 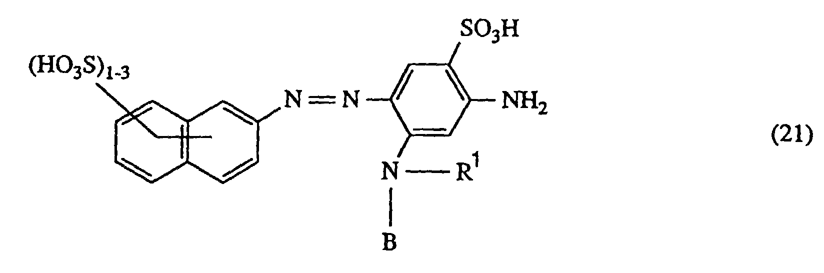 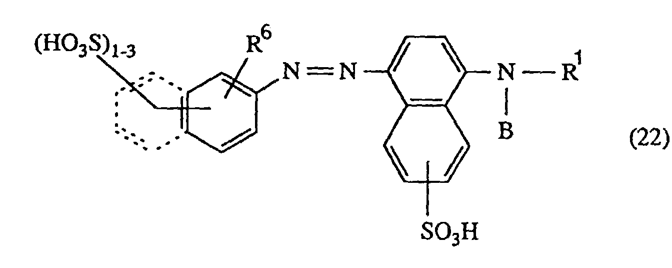 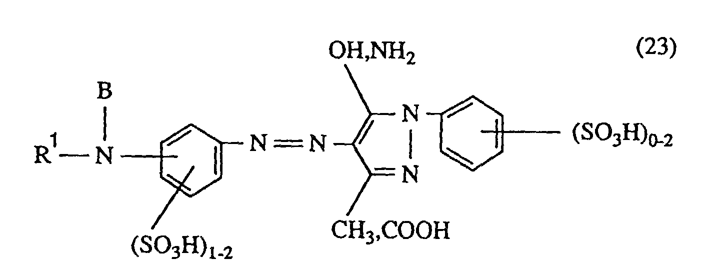 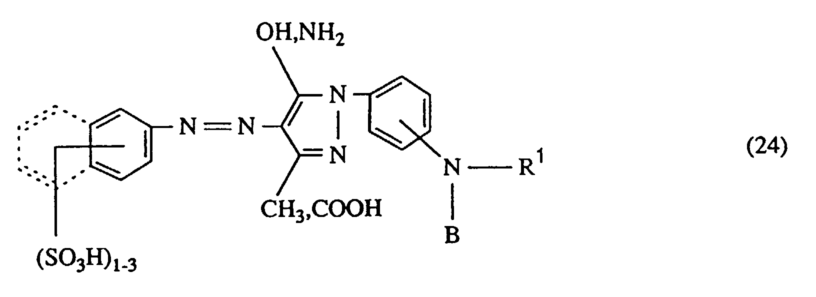 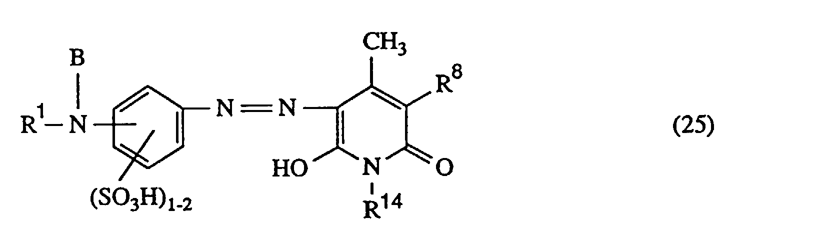 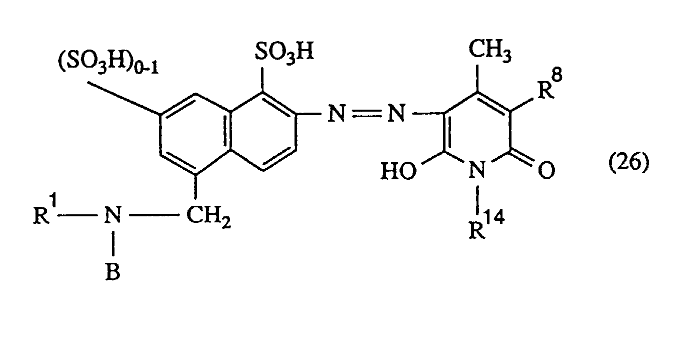 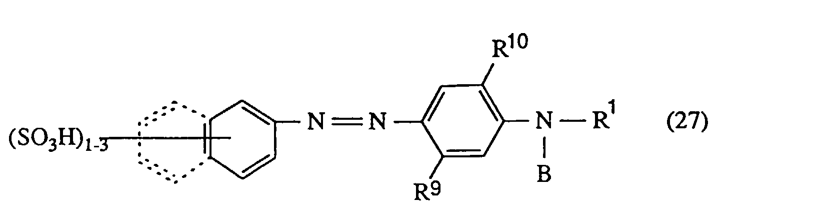 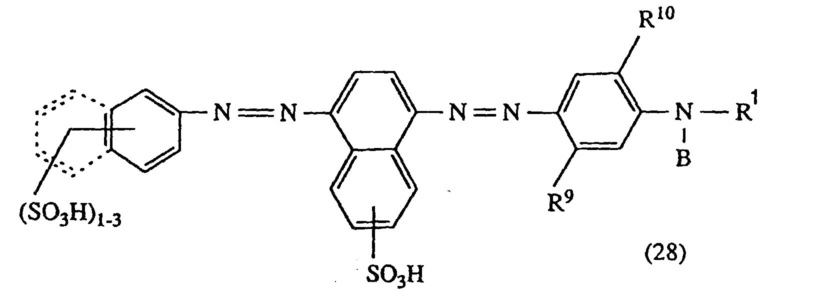 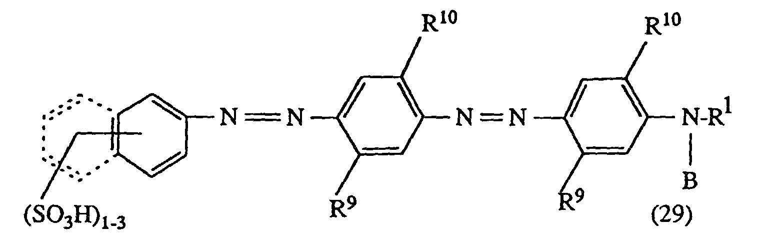 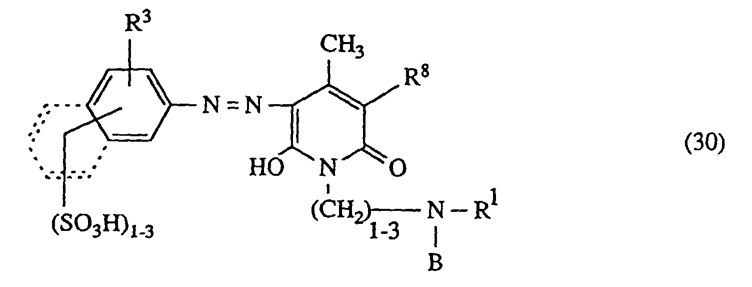 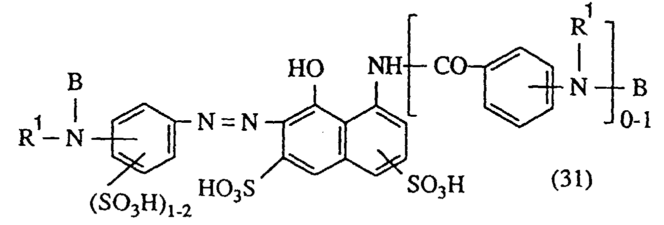 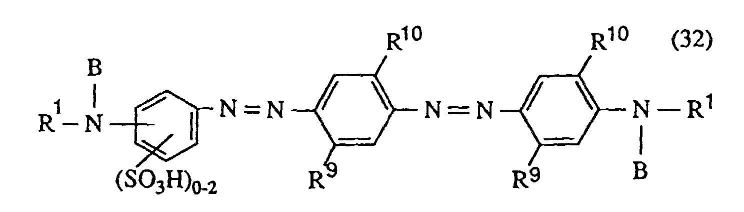 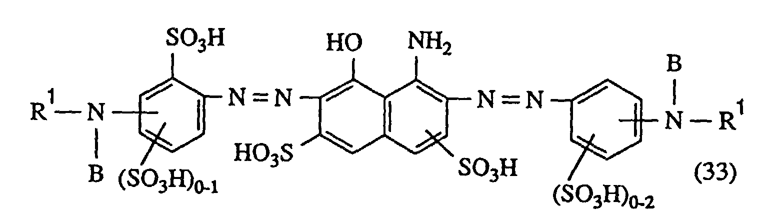 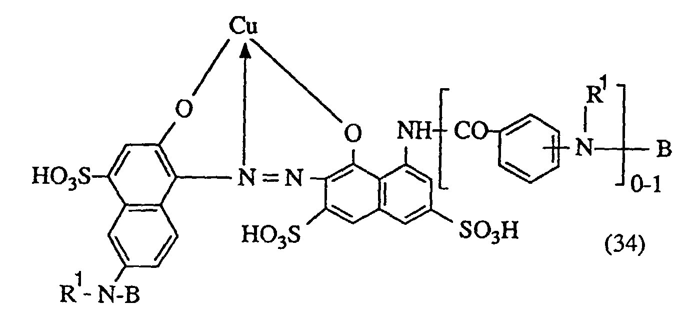 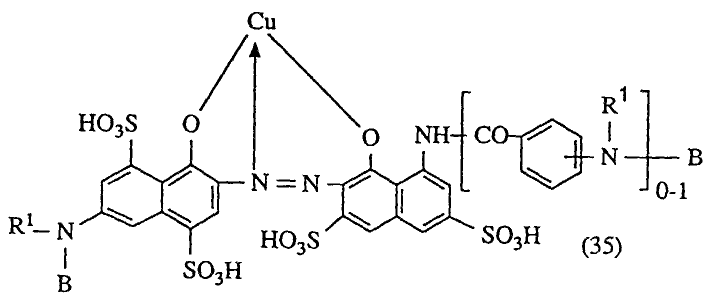 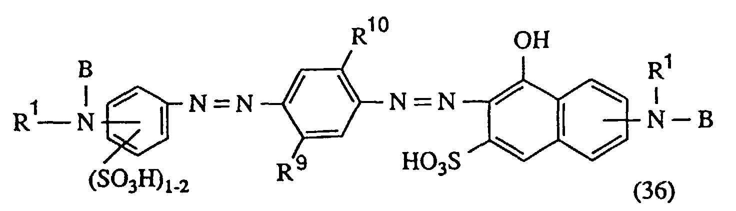 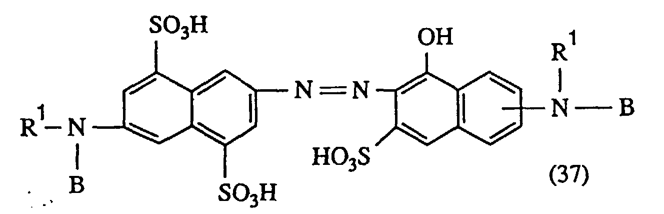 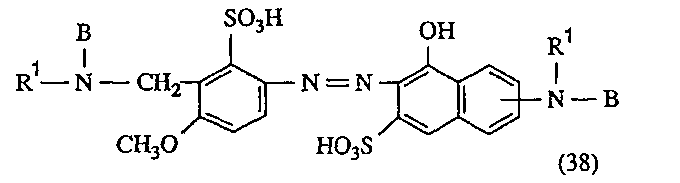 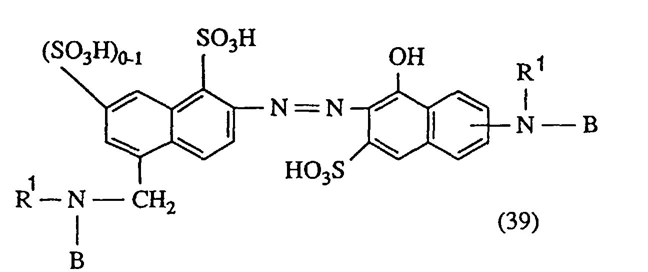 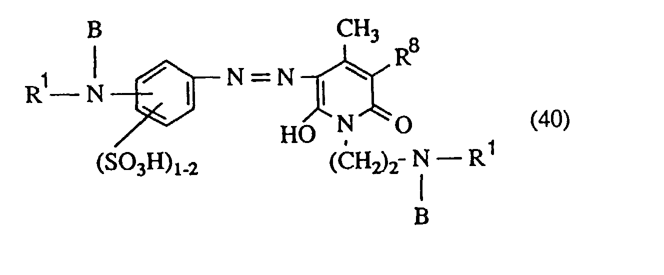 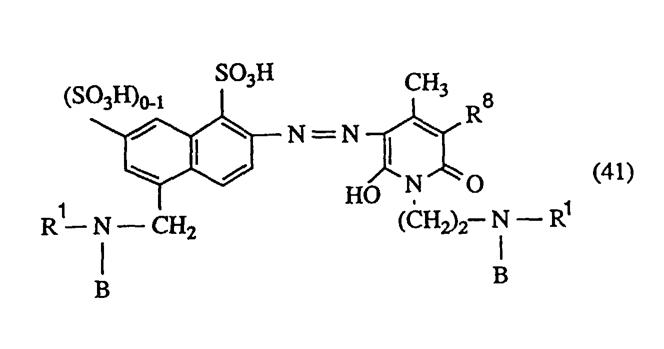 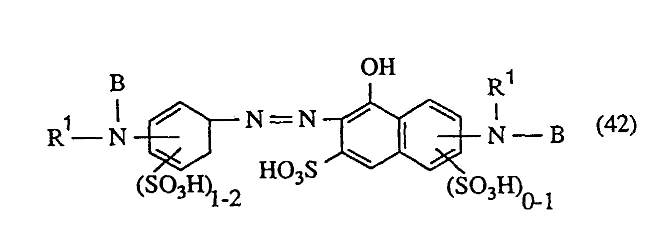 worin
Acyl = Acetyl oder gegebenenfalls substituiertes Benzoyl ist,
R¹⁴ = H, C₁-C₂-Alkyl, gegebenenfalls substituiert durch SO₃H oder NH₂,
R¹ = H, CH₃ oder C₂H₅,
R³ = H oder Sulfo,
R⁵ = H, CH₃, OCH₃ oder Cl,
R⁶ = H, C₁-C₄-Alkyl, C₁-C₄-Alkoxy, Cl, Br, COOH, SO₃H.
R⁷ = H, OH, NH₂, NHCOCH₃, NHCOPh, Cl, C₁-C₄-Alkoxy oder C₁-C₄-Alkyl ist,
R⁸ = H, SO₃H, CH₂SO₃H, Cl, C₁-C₄-Alkylsulfonyl, CN, Carbonamid, insbesondere CONH₂,
R⁹ = H, C₁-C₄-Alkyl, C₁-C₄-Alkoxy, Cl, Br, Acylamino, insbesondere C₁-C₄-Alkylcarbonylamino oder Arylcarbonylamino wie gegebenenfalls substituiertes Phenylcarbonylamino, C₁-C₄-Alkylsulfonylamino, Aminocarbonylamino, C₁-C₄-Alkylsulfonylamino, Arylsulfonylamino,
R¹⁰ = H, C₁-C₄-Alkyl, C₁-C₄-Alkoxy, OH, SO₃H,
und wobei die durch Strichelungen angedeuteten kondensierten Ringe für alternativ mögliche Naphthalinsysteme stehen.

5. Reaktivfarbstoffe gemäß wenigstens einem der vorhergehenden Ansprüche,
worin
X = Cl,
i = 0,
j = 0 oder 1,
Z = -CH₂-CH₂-OSO₃H oder -CH=CH₂.
R² = H.

6. Reaktivfarbstoffe gemäß wenigstens einem der vorhergehenden Ansprüche,
worin
X = F,
i = 0,
j = 0,
Z = -CH₂-CH₂-OSO₃H, -CH=CH₂,
R² = H.

7. Reaktivfarbstoffe gemäß wenigstens einem der vorhergehenden Ansprüche,
worin
Z = -CH₂-CH₂-OSO₃H ist.

8. Reaktivfarbstoffe gemäß Anspruch 1, dadurch gekennzeichnet, daß der Rest D ein- oder zweimal mit der Gruppe 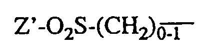 substituiert ist, worin
Z' -CH=CH₂, -CH₂-CH₂-OSO₃H, -CH₂-CH₂-Cl, -CH₂-CH₂-Br, -CH₂-CH₂-S₂O₃H, -CH₂-CH₂-O-CO-CH₃, -CH₂-CH₂-OPO₃H₂ oder -CH₂-CH₂-OH bedeutet.

9. Reaktivfarbstoffe gemäß Anspruch 8, dadurch gekennzeichnet, daß
n = 1,
W = direkte Bindung und
D ein Rest der allgemeinen Formel (IX) ist, 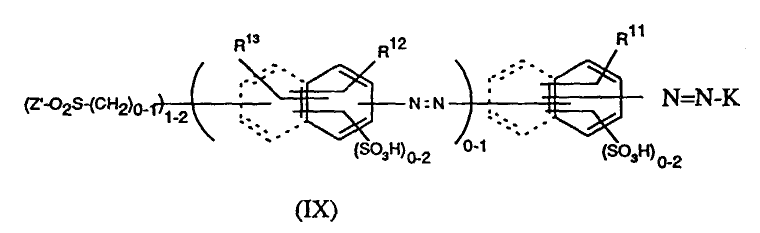
worin
R¹¹ H, C₁-C₄-Alkyl, C₁-C₄-Alkoxy, Cl, Br oder Acylamino,
R¹² H, C₁-C₄-Alkyl, Cl, Br, C₁-C₄-Alkoxy oder COOH,
R¹³ H, C₁-C₄-Alkyl, C₁-C₄-Alkoxy, SO₃H, Cl oder Br,
K eine bivalenter Rest der allgemeinen Formeln (Xa) - (Xd) 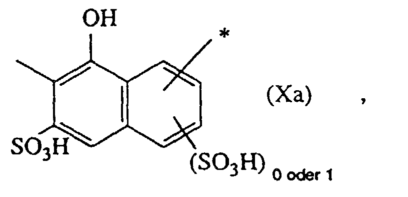 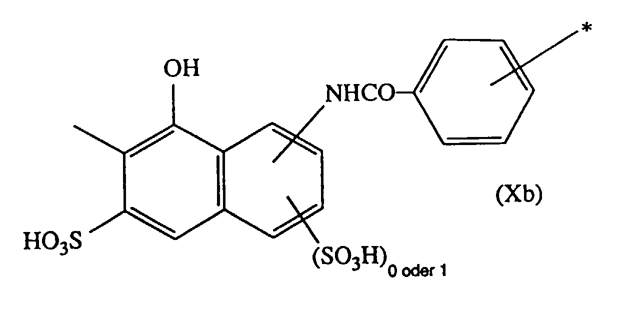 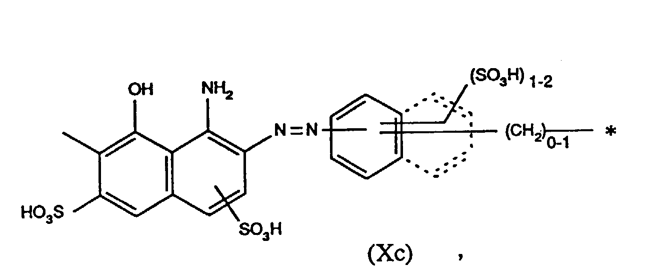 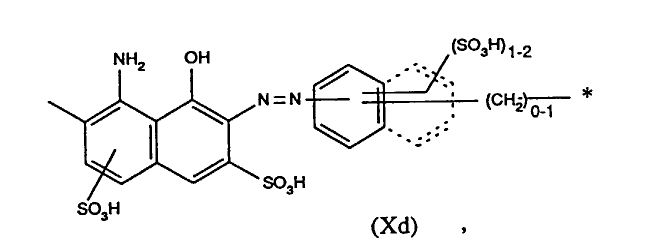
bedeutet, wobei die mit * gekennzeichneten Bindungen an die Gruppe -NR¹-B gebunden sind und B die in Anspruch 4 genannte Bedeutung hat.

10. Reaktivfarbstoffe gemäß Anspruch 9, dadurch gekennzeichnet, daß
R¹,R² unabhängig voneinander H, CH₃ oder C₂H₅,
j 0 oder 1,
X F oder Cl und
Z, Z' unabhängig voneinander CH₂-CH₂-OSO₃H oder CH=CH₂
bedeuten.

11. Reaktivfarbstoffe gemäß Anspruch 1, dadurch gekennzeichnet, daß
D für einen Farbstoffrest der Formel (IX) steht, und
R¹, R² = H,
i = 0,
j = 0,
r = 2 und
Z',Z = -CH₂CH₂OSO₃H
bedeutet

12. Reaktivfarbstoffe gemäß Anspruch 9, dadurch gekennzeichnet, daß sie einer der folgenden Formeln entsprechen, worin
B ein Rest der Formel 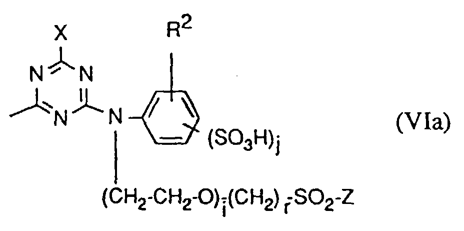
beinhaltet: 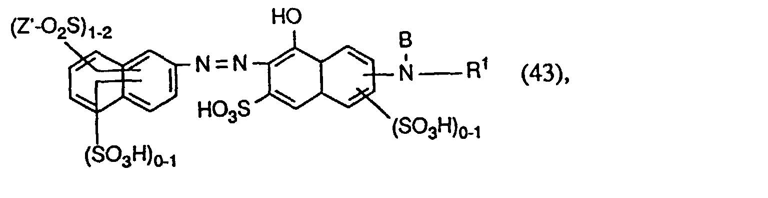 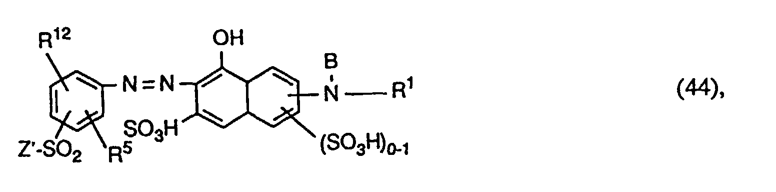 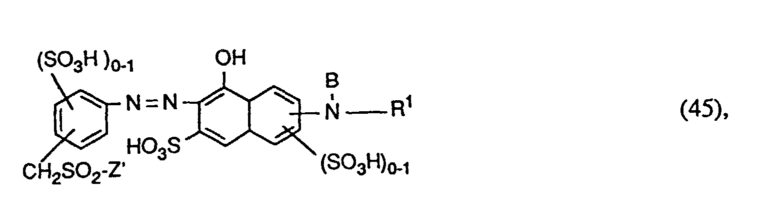 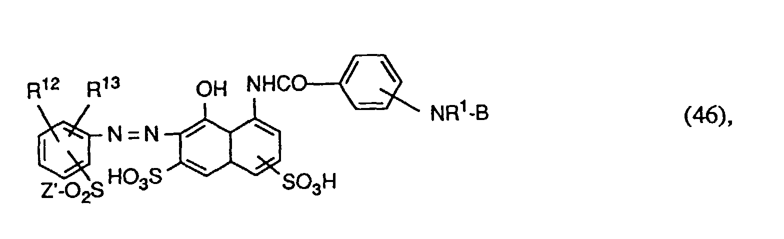 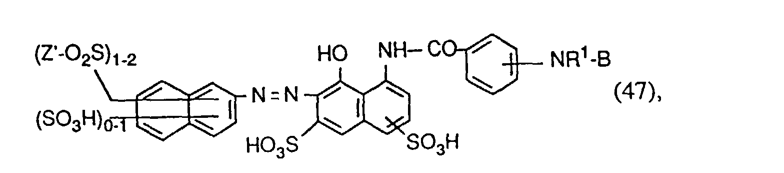 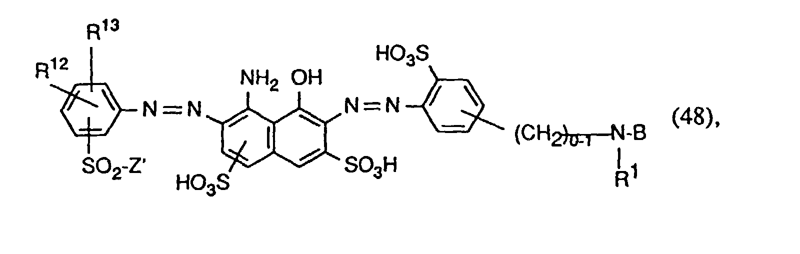 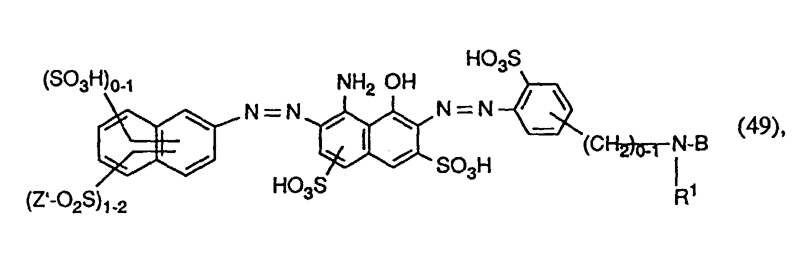 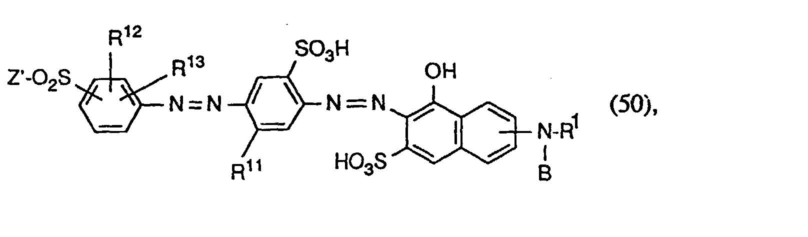 worin
R¹ = H, CH₃ oder C₂H₅,
R¹¹= H, C₁-C₄-Alkyl, C₁-C₄-Alkoxy, Acylamino, Cl oder Br,
R¹²= H, C₁-C₄-Alkyl, C₁-C₄-Alkoxy, Cl, Br oder COOH,
R¹³= H, C₁-C₄-Alkyl, C₁-C₄-Alkoxy, Cl, Br, SO₃H und
die durch Strichelungen angedeuteten kondensierten Ringe für alternativ mögliche Naphthalinsysteme stehen.

13. Verfahren zur Herstellung von Reaktivfarbstoffen der Formel 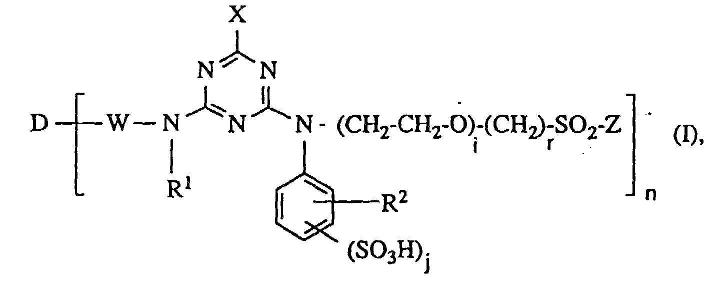 worin
D Rest eines organischen Farbstoffes aus der Monoazo, Polyazo- oder Metallkomplexazoreihe,
W direkte Bindung oder Brückenglied,
R¹ H, C₁-C₄-Alkyl, das durch OR, OSO₃H, SO₃H, COOR oder Halogen substituiert sein kann,
R H, CH₃ oder C₂H_{5,}
R² H, C₁-C₄-Alkyl, Cl, Br, C₁-C₄-Alkoxy oder COOH,
i 0 oder 1,
j 0, 1 oder 2,
r 2 oder 3, insbesondere 2,
X F, Cl oder Br,
Z -CH=CH₂, -CH₂-CH₂-OSO₃H,
-CH₂-CH₂-S₂O₃H, -CH₂-CH₂-O-CO-CH₃, -CH₂-CH₂-OPO₃H₂, -CH₂-CH₂-OH,
n 1 oder 2
bedeuten, dadurch gekennzeichnet, daß man nach der Methode:
a) durch Kondensation von Farbstoffen der Formel 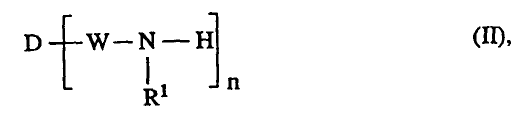 mit n-Molen Trihalogentriazinen der Formel 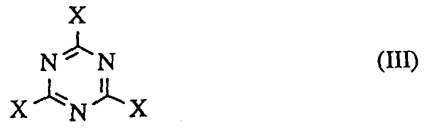 zu Verbindungen der Formel 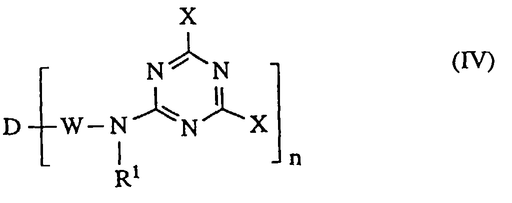 und weitere Kondensation der Verbindungen der Formel (IV) mit n-Molen der Komponenten der Formel 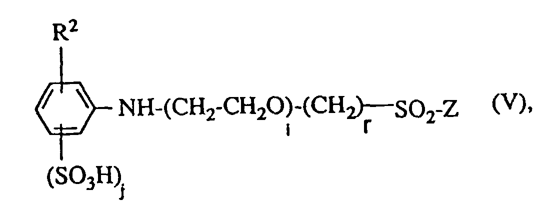 oder
b) in umgekehrter Reihenfolge durch Kondensation von Trihalogentriazinen der Formel (III) mit den Komponenten der Formel (V) zu den Primärkondensationsprodukten 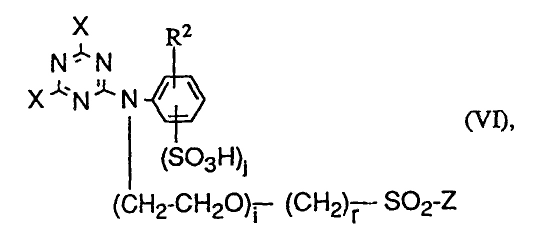 und weitere Kondensation mit n-Molen der Verbindungen der Formel (VI) mit den Farbstoffen der Formel (II), oder
c) durch Kondensation geeigneter Vorprodukte mit den Trihalogentriazinen (III) und den Komponenten der Formel (V) bzw. durch Kondensation geeigneter Vorprodukte mit den Primärkondensationsprodukten der Formel (VI) und anschließender Farbstoffsynthese,
vorgeht

14. Verfahren zum Färben und Bedrucken von Substraten mit einem Reaktivfarbstoff, dadurch gekennzeichnet, daß ein Reaktivfarbstoff gemäß wenigstens einem der vorhergehenden Ansprüche verwendet wird.

15. Verbindungen der allgemeinen Formel 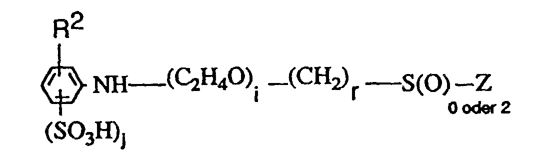 einschließlich ihrer Salze und ihrer Umsetzungsprodukte mit Schutzgruppen,
worin R², Z, j und i die in Anspruch 1 angegebene Bedeutung haben und r = 3.

16. Verfahren zur Herstellung von Verbindungen der Formel 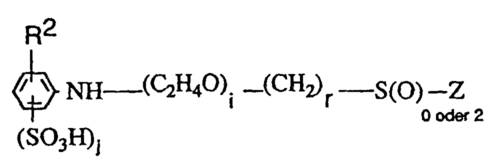 worin
R², Z, j, i und r die in Anspruch 1 angegebene Bedeutung haben,
durch Sulfatieren von Verbindungen der Formel 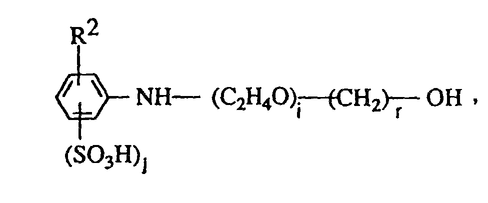 anschließende Umsetzung mit Mercaptoethanol unter Erhalt von 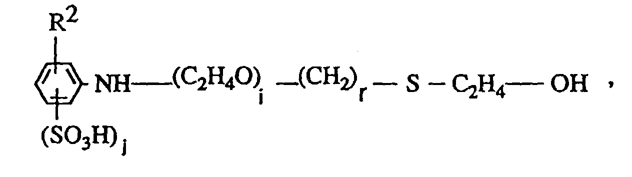 gegebenenfalls Blockierung der NH-Gruppe mit einer Schutzgrupppe und Oxidation zu 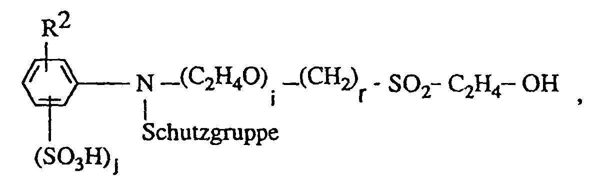 gegebenenfalls Abspalten der Schutzgruppe unter Erhalt von 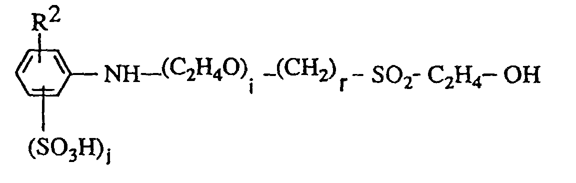 und gegebenenfalls übliche Überführung des Restes SO₂C₂H₄-OH in die Vinylsulfonylgruppe oder Überführung der -CH₂-CH₂-OH Gruppe in die übrigen unter Z aufgeführten abspaltbaren Gruppen.

17. Reaktivfarbstoffe gemäß Anspruch 1, worin i = 1.

18. Verbindungen gemäß Anspruch 15, worin i = 1.

## Claims

1. Reactive dyestuffs of the formula 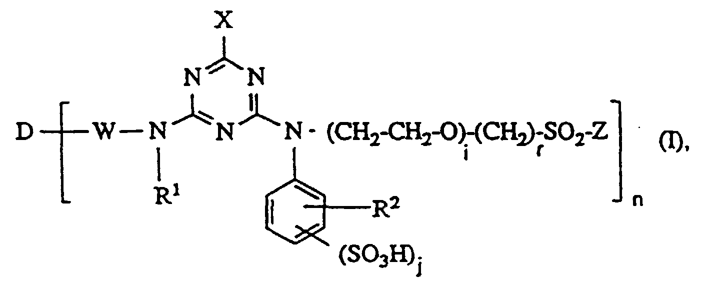 wherein
D denotes the radical of an organic dyestuff of the monoazo, polyazo or metal complex azo series,
W denotes a direct bond or bridge member,
R¹ denotes H or C₁-C₄-alkyl, which can be substituted by OR, OSO₃H, SO₃H, COOR or halogen,
R denotes H, CH₃ or C₂H₅,
R² denotes H, C₁-C₄-alkyl, Cl, Br, C₁-C₄-alkoxy or COOH,
i denotes 0 or 1,
j denotes 0, 1 or 2,
r denotes 2 or 3, in particular 2,
X denotes F, Cl or Br,
Z denotes -CH=CH₂, -CH₂-CH₂-OSO₃H, -CH₂-CH₂-S₂O₃H, -CH₂-CH₂-O-CO-CH₃, -CH₂-CH₂-OPO₃H₂ or -CH₂-CH₂-OH, and
n denotes 1 or 2.

2. Reactive dyestuffs according to Claim 1, characterised in that they correspond to the following formula 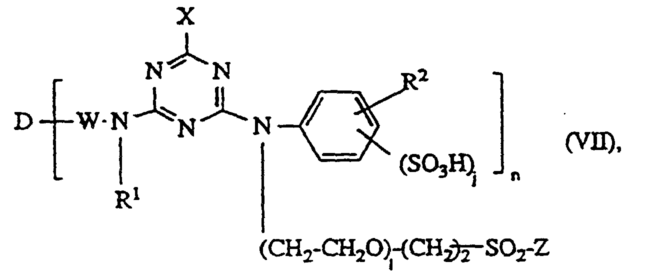 wherein
D is the radical of an organic dyestuff of the monoazo, polyazo or metal complex azo series, which contains no further fibre-reactive groups,
R¹ and R² independently of one another are H, CH₃ or C₂H₅,
W is a direct bond or bridge member,
i is 0 or 1,
j is 0 or 1,
X is F or Cl,
Z is CH₂-CH₂-OSO₃H or CH=CH₂ and
n is 1 or 2.

3. Reactive dyestuffs according to at least one of the preceding claims, characterised in that they correspond to the following formula 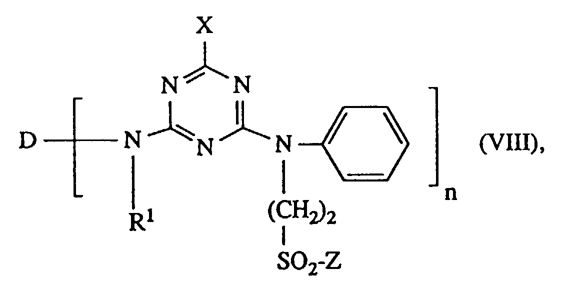 wherein
D is the radical of an organic dyestuff of the monoazo, polyazo or metal complex azo series, which contains no further fibre-reactive groups,
X is F,
Z is -CH₂-CH₂-OSO₃H,
R¹ is H and
n is 1.

4. Reactive dyestuffs according to at least one of the preceding claims, characterised in that they correspond to one of the following formulae,
wherein
B comprises a radical of the formula 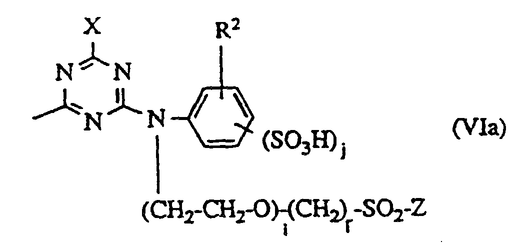
and X, i, R², j, r and Z have the meaning given in Claim 1: 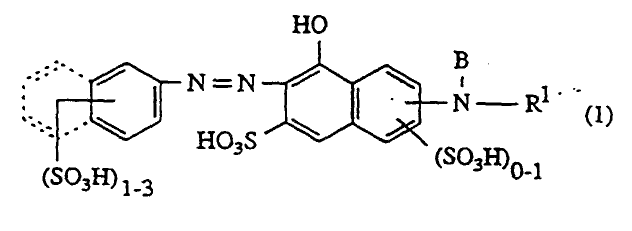 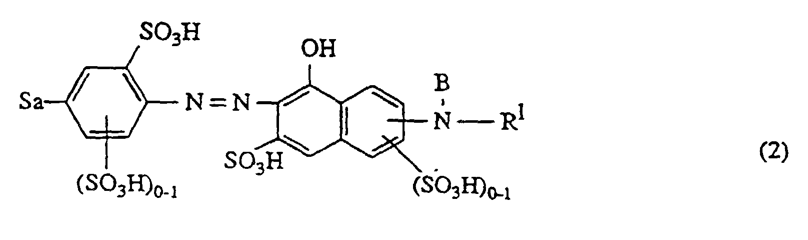 where Sa = OCH₃ or OC₂H₅ 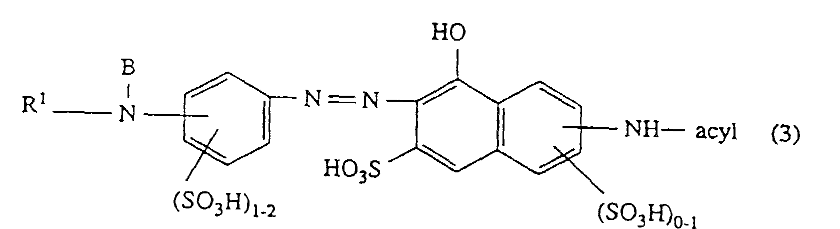 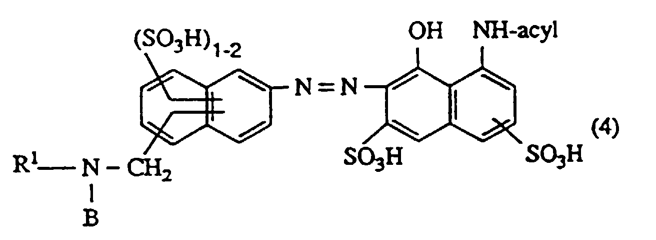 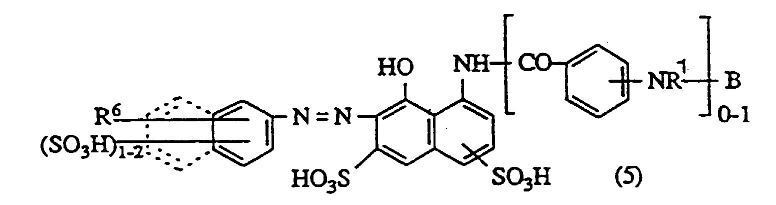 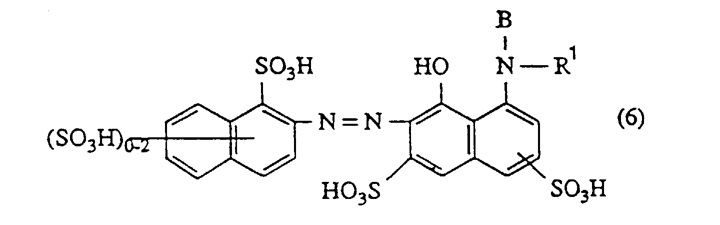 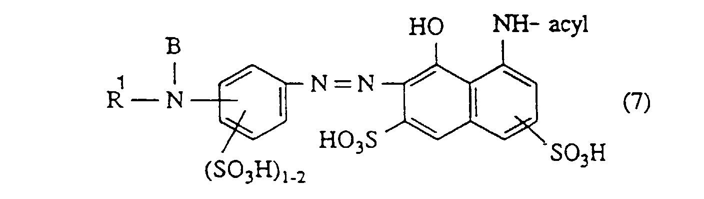 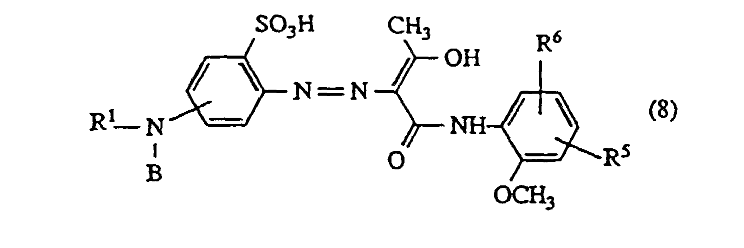 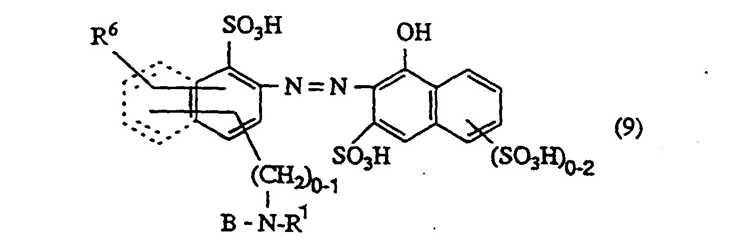 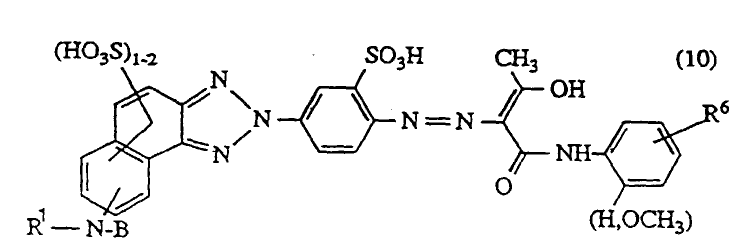 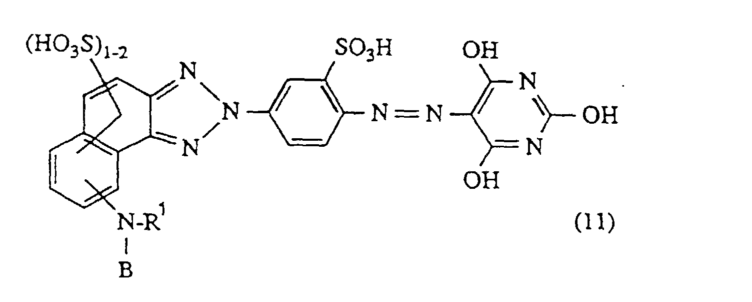 Metal complexes of dyestuffs of the formulae (12) to (42): 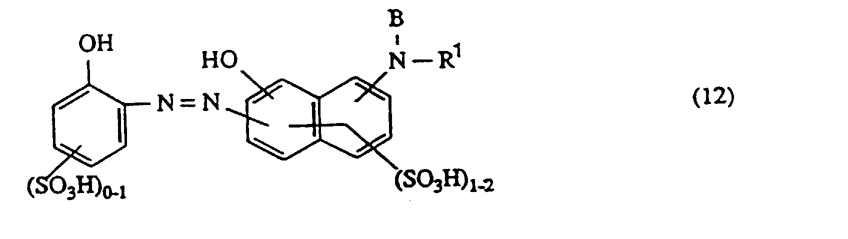 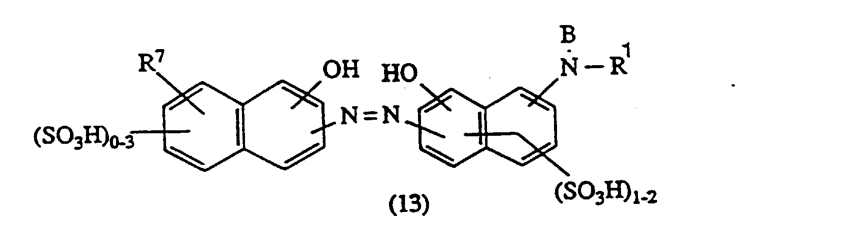 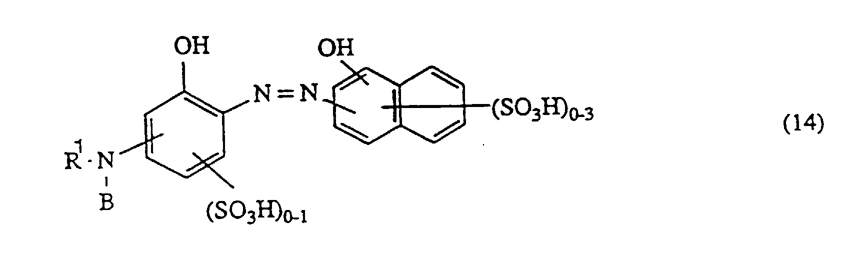 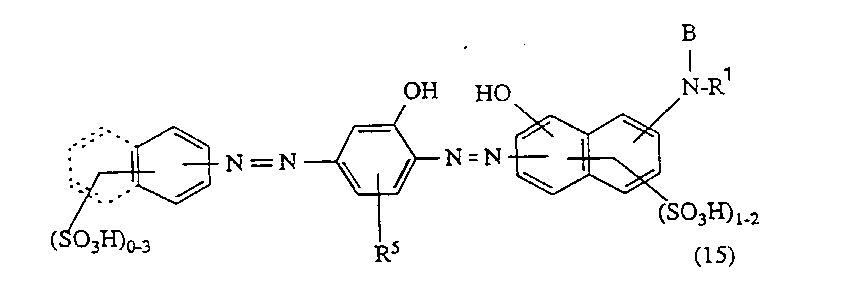 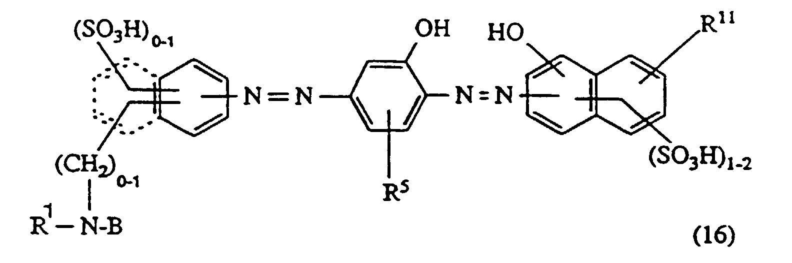 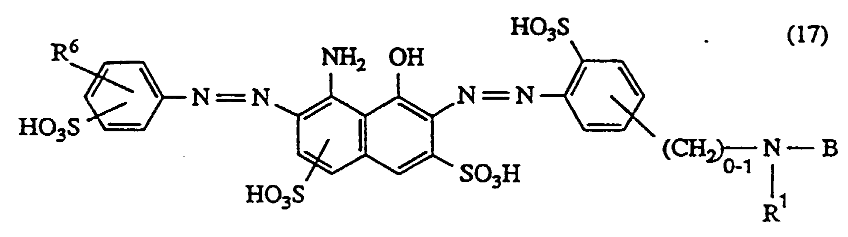 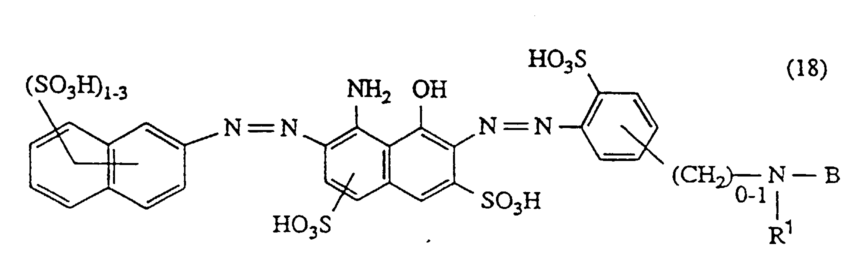 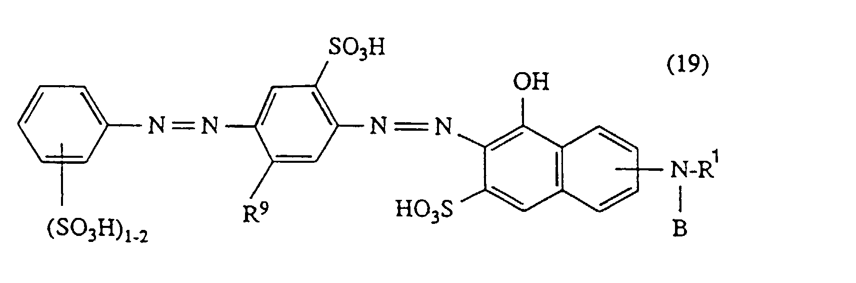 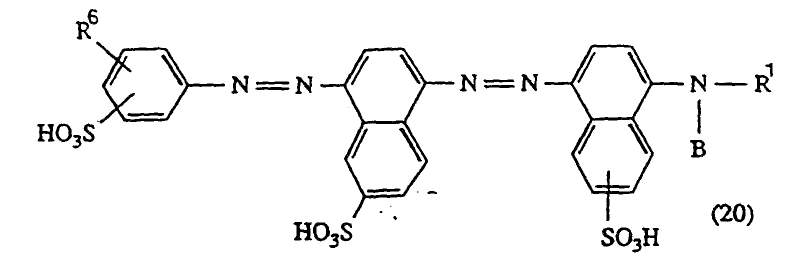 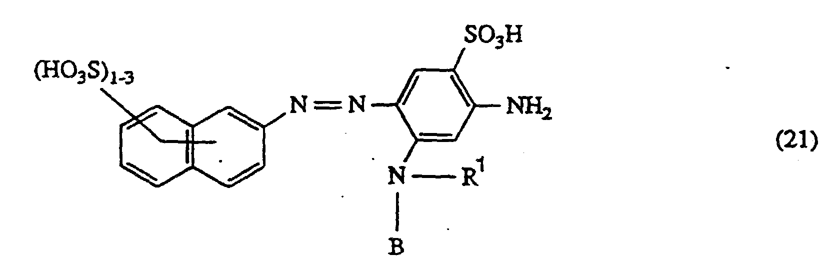 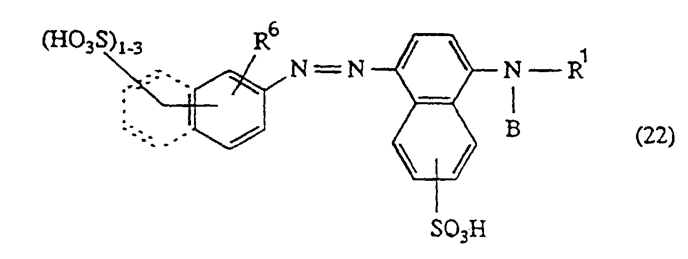 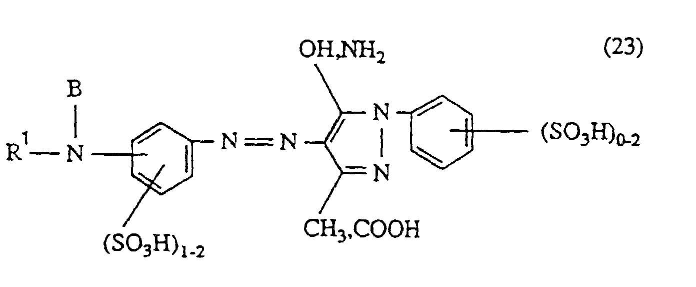 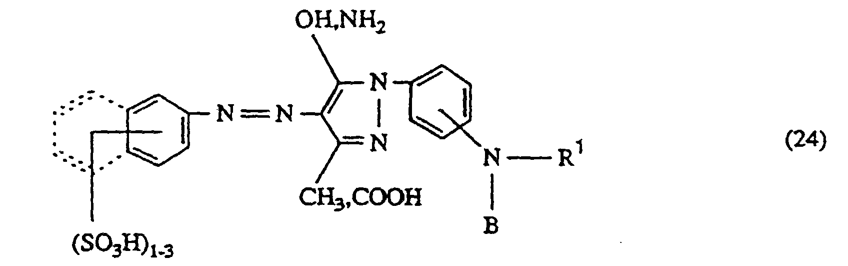 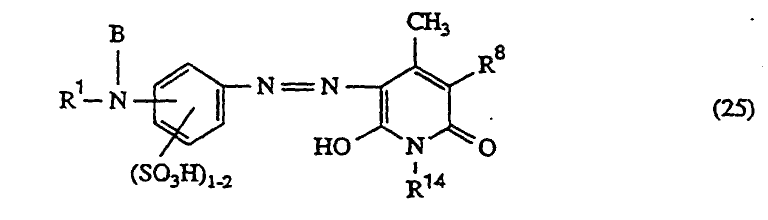 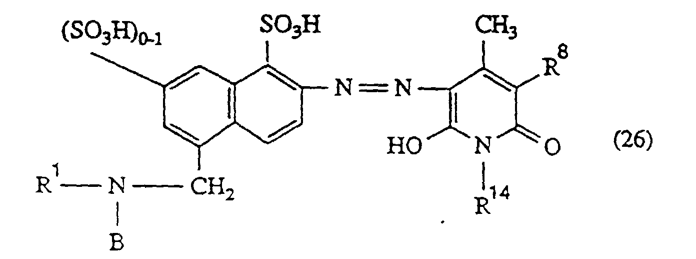 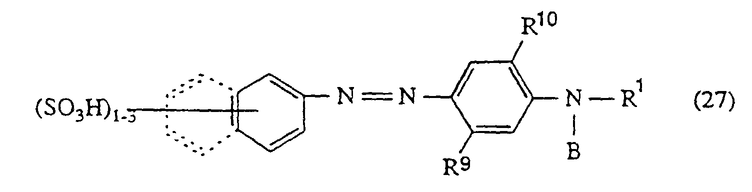 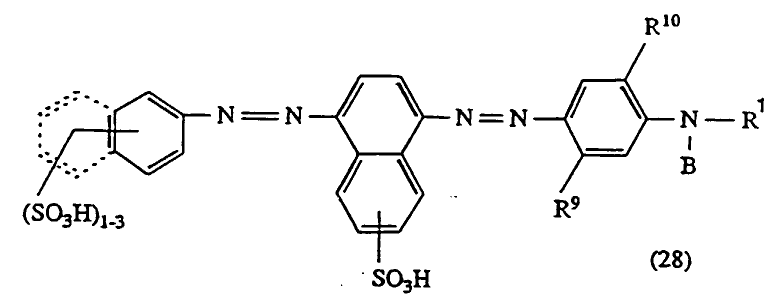 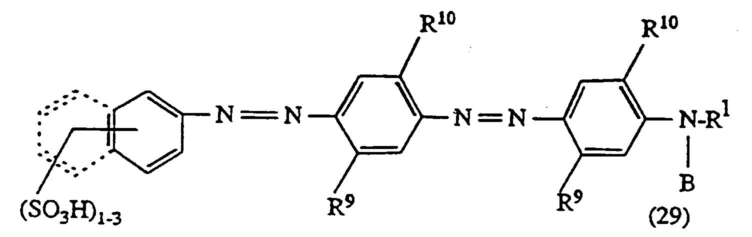 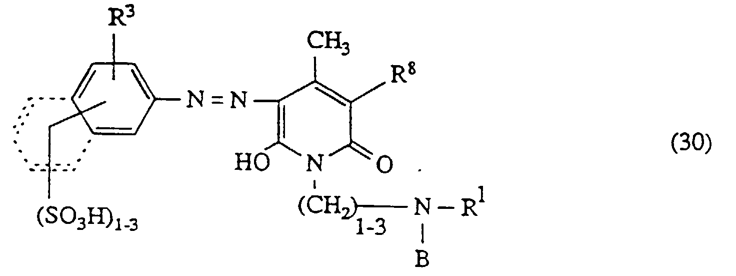 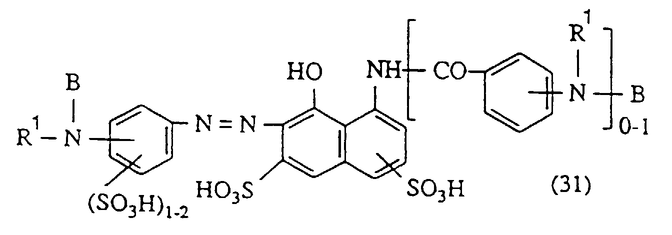 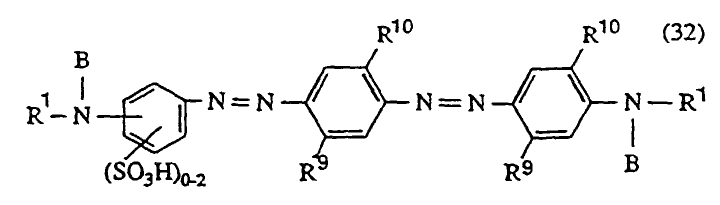 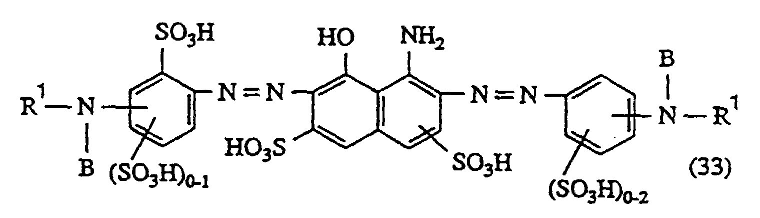 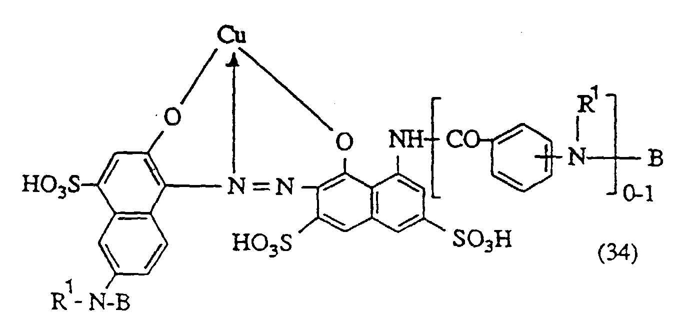 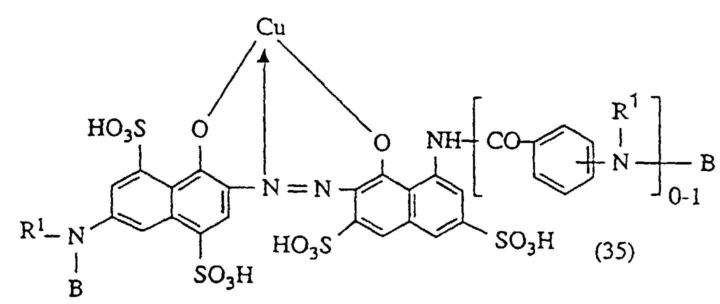 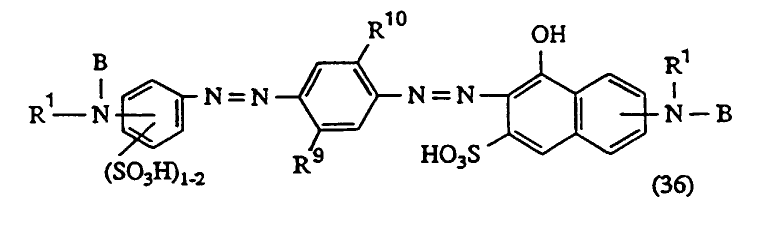 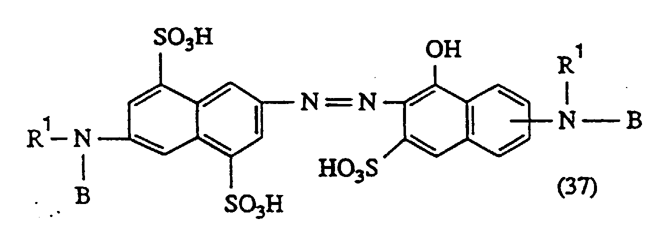 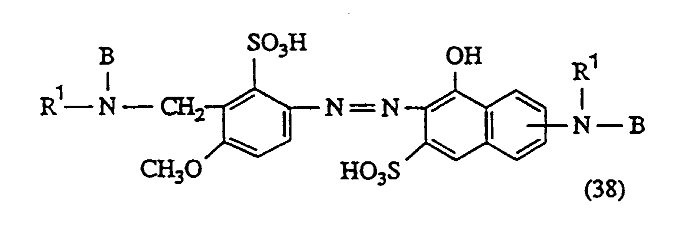 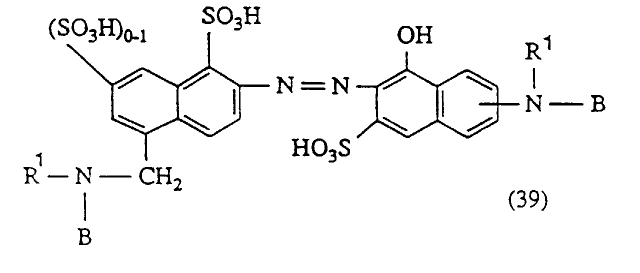 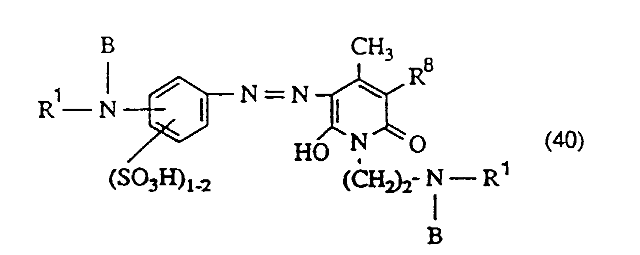 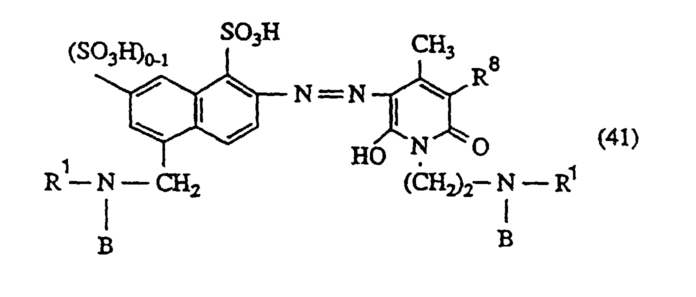 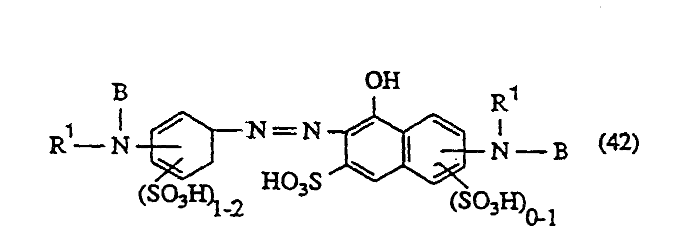 wherein
acyl = acetyl or optionally substituted benzoyl,
R¹⁴= H or C₁-C₂-alkyl which is optionally substituted by SO₃H or NH₂,
R¹ = H, CH₃ or C₂H₅,
R³ = H or sulpho,
R⁵ = H, CH₃, OCH₃ or Cl,
R⁶ = H, C₁-C₄-alkyl, C₁-C₄-alkoxy, Cl, Br, COOH or SO₃H,
R⁷ = H, OH, NH₂, NHCOCH₃, NHCOPh, Cl, C₁-C₄-alkoxy or C₁-C₄-alkyl,
R⁸ = H, SO₃H, CH₂SO₃H, Cl, C₁-C₄-alkylsulphonyl, CN, carbonamide, in particular CONH₂,
R⁹ = H, C₁-C₄-alkyl, C₁-C₄-alkoxy, Cl, Br, acylamino, in particular C₁-C₄-alkylcarbonylamino or arylcarbonylamino, such as optionally substituted phenylcarbonylamino, C₁-C₄-alkylsulphonylamino, aminocarbonylamino, C₁-C₄-alkylsulphonylamino or arylsulphonylamino, and
R¹⁰= H, C₁-C₄-alkyl, C₁-C₄-alkoxy, OH or SO₃H,
and wherein the fused rings indicated by broken lines represent alternatively possible naphthalene systems.

5. Reactive dyestuffs according to at least one of the preceding claims,
wherein
X = Cl,
i = 0,
j = 0 or 1,
Z = -CH₂-CH₂-OSO₃H or -CH=CH₂ and
R² = H.

6. Reactive dyestuffs according to at least one of the preceding claims,
wherein
X = F,
i = 0,
j = 0,
Z = -CH₂-CH₂-OSO₃H or -CH=CH₂ and
R² = H.

7. Reactive dyestuffs according to at least one of the preceding claims,
wherein
Z = -CH₂-CH₂-OSO₃H.

8. Reactive dyestuffs according to Claim 1, characterised in that the radical D is substituted once or twice by the group
Z'-O₂S-(CH₂)₀₁)
wherein
Z' denotes -CH=CH₂, -CH₂-CH₂-OSO₃H, -CH₂-CH₂-Cl, -CH₂-CH₂-Br, -CH₂-CH₂-S₂O₃H, -CH₂-CH₂-O-CO-CH₃, -CH₂-CH₂OPO₃H₂ or -CH₂-CH₂-OH.

9. Reactive dyes according to Claim 8, characterised in that
n = 1,
W = a direct bond and
D is a radical of the general formula (IX) 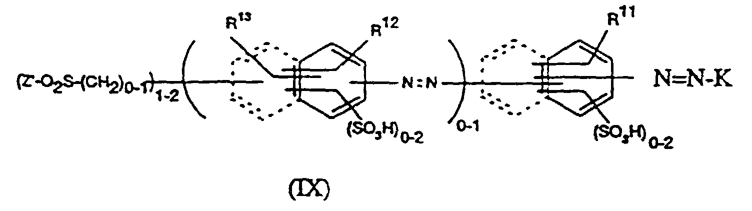
wherein
R¹¹ denotes H, C₁-C₄-alkyl, C₁-C₄-alkoxy, Cl, Br or acylamino,
R¹² is H, C₁-C₄-alkyl, Cl, Br, C₁-C₄-alkoxy or COOH,
R¹³ denotes H, C₁-C₄-alkyl, C₁-C₄-alkoxy, SO₃H, Cl or Br,
K denotes a bivalent radical of the general formulae (Xa) - (Xd) 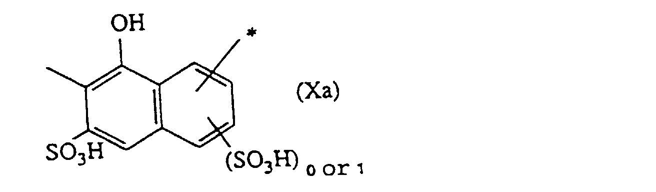 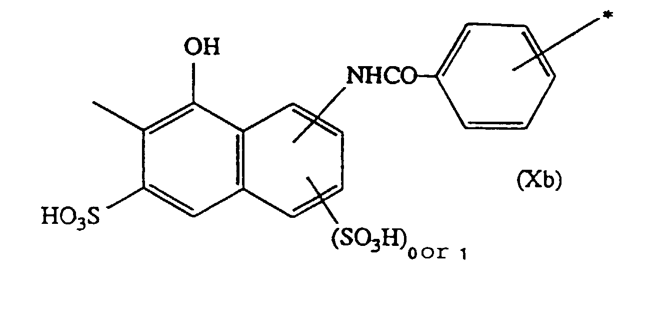 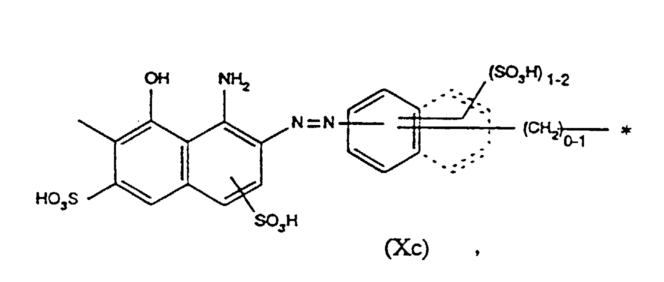 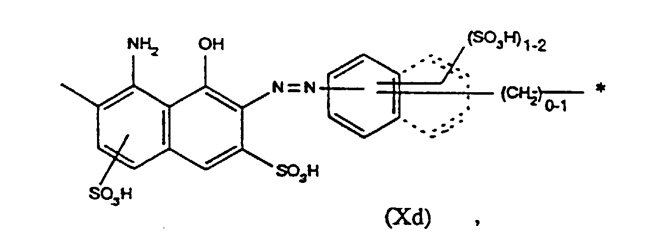
in which the bonds marked with * are attached to the group -NR¹-B and B has the meaning given in Claim 4.

10. Reactive dyes according to Claim 9, characterised in that
R¹ and R² independently of one another denote H, CH₃ or C₂H₅,
j denotes 0 or 1,
X denotes F or Cl, and
Z and Z' independently of one another denote CH₂-CH₂-OSO₃H or CH=CH₂.

11. Reactive dyes according to Claim 1, characterised in that
D represents a dyestuff radical of the formula (IX), and
R¹ and R² = H,
i = 0,
j = 0,
r = 2, and
Z' and Z = -CH₂CH₂OSO₃H.

12. Reactive dyes according to Claim 9, characterised in that they correspond to one of the following formulae, wherein
B comprises a radical of formula 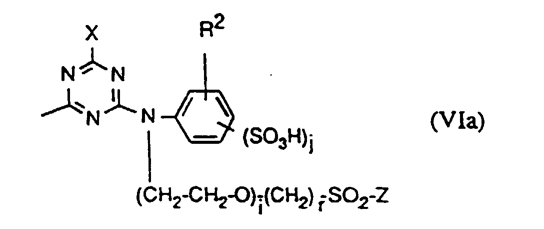 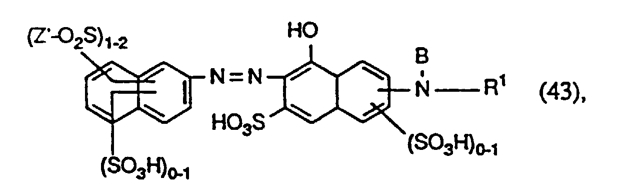 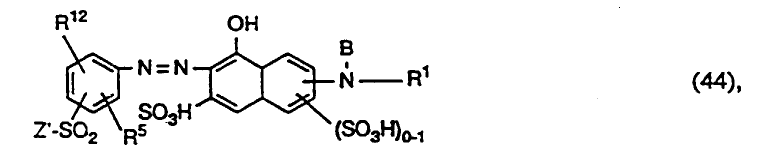 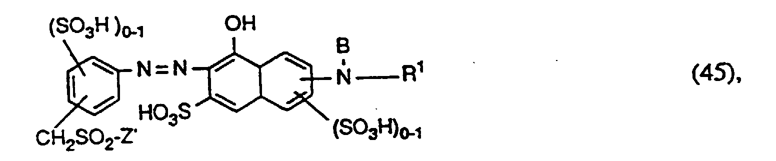 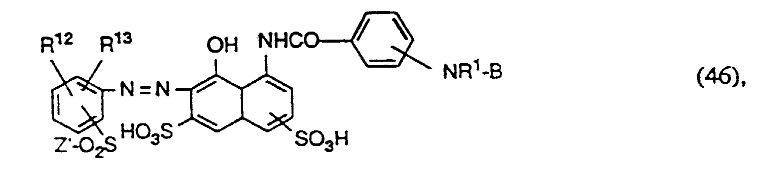 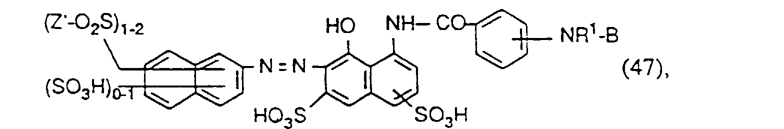 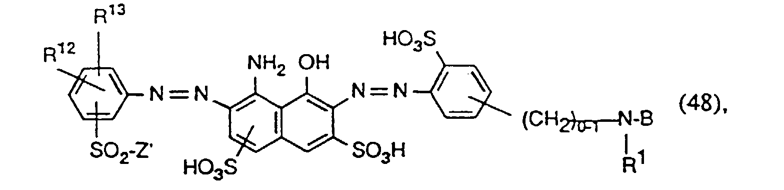 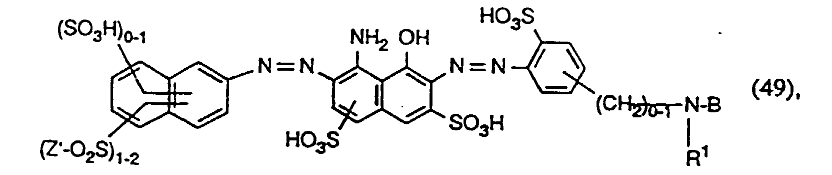 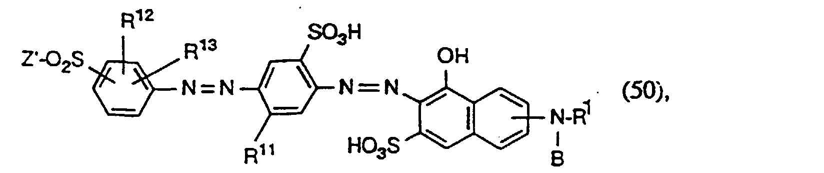
in which
R¹ = H, CH₃ or C₂H₅,
R¹¹= H, C₁-C₄-alkyl, C₁-C₄-alkoxy, acylamino, Cl or Br,
R¹²= H, C₁-C₄-alkyl, C₁-C₄-alkoxy, Cl, Br or COOH,
R¹³= H, C₁-C₄-alkyl, C₁-C₄-alkoxy, Cl, Br, SO₃H, and
the fused rings indicated by broken lines represent alternatively possible naphthalene systems.

13. Process for the preparation of reactive dyestuffs of the formula 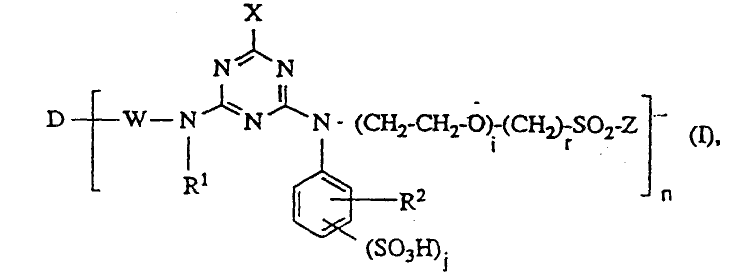 wherein
D denotes the radical of an organic dyestuff from the monoazo, polyazo or metal complex azo series,
W denotes a direct bond or bridge member,
R¹ denotes H, C₁-C₄-alkyl which can be substituted by OR, OSO₃H, SO₃H, COOR or by halogen,
R denotes H, CH₃ or C₂H₅,
R² denotes H, C₁-C₄-alkyl, Cl, Br, C₁-C₄-alkoxy or COOH,
i denotes 0 or 1,
j denotes 0, 1 or 2,
r denotes 2 or 3, in particular 2,
X denotes F, Cl or Br,
Z denotes -CH=CH₂, -CH₂-CH₂-OSO₃H, -CH₂-CH₂-S₂O₃H, -CH₂-CH₂-O-CO-CH₃, -CH₂-CH₂-OPO₃H₂, -CH₂-CH₂-OH,
n denotes 1 or 2,
characterised in that the procedure of the method used is:
a) by condensation of dyestuffs of the formula 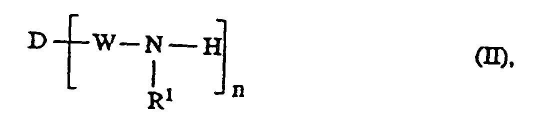 with n mol of trihalogenotriazines of the formula 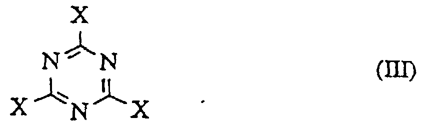 to give compounds of the formula 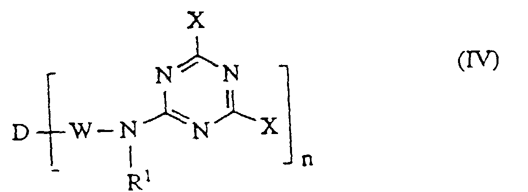 and further condensation of the compounds of the formula (IV) with n mol of components of the formula 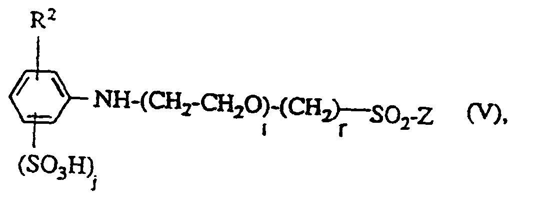 or
b) in reverse sequence, by condensation of trihalogenotriazines of the formula (III) with components of the formula (V) to give the primary condensation products 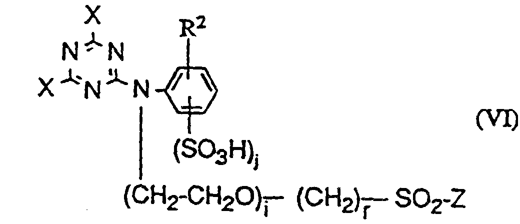 and further condensation with n mol of the compounds of the formula (VI) with the dyestuffs of the formula (II),
or
c) by condensation of suitable precursors with the trihalogenotriazines (III) and components of the formula (V), or by condensation of suitable precursors with the primary condensation products of the formula (VI) and subsequent dyestuff synthesis.

14. Process for dyeing and printing substrates with a reactive dyestuff, characterised in that a reactive dyestuff according to at least one of the preceding claims is used.

15. Compounds of the general formula 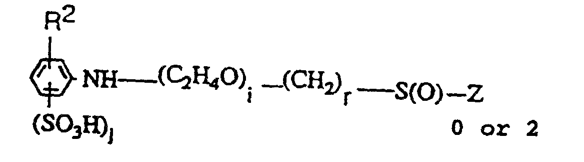 including their salts and their reaction products with protective groups,
wherein R², Z, j and i have the meaning given in Claim 1 and r = 3.

16. Process for the preparation of compounds of the formula 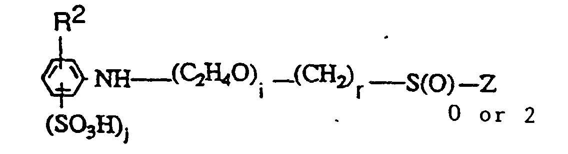 wherein
R², Z, j, i and r have the meaning given in Claim 1,
by sulphation of compounds of the formula 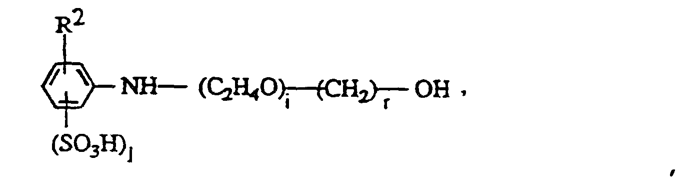 subsequent reaction with mercaptoethanol to give 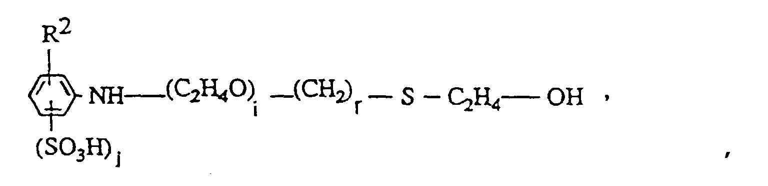 if appropriate blocking of the NH group with a protective group and oxidation to give 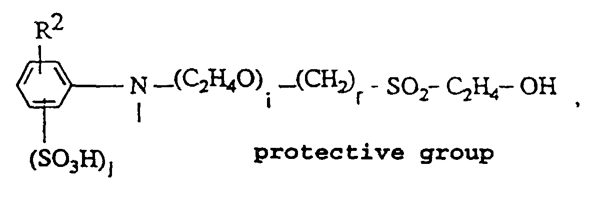 if appropriate detachment of the protective group to give 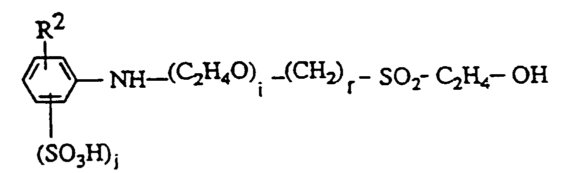 and if appropriate customary conversion of the radical SO₂C₂H₄-OH into the vinylsulphonyl group or conversion of the -CH₂-CH₂-OH group into the other detachable groups listed under Z.

17. Reactive dyestuffs according to Claim 1, wherein i = 1.

18. Compounds according to Claim 15, wherein i = 1.

## Revendications

1. Colorants réactifs de formule : 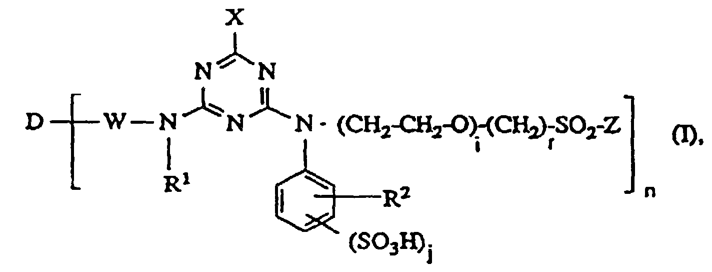 dans laquelle:
D représente le radical d'un colorant organique de la série monoazoïque, polyazoïque ou de la série des complexes métalliques de colorants azoïques,
W représente une liaison directe ou un pont,
R¹ représente H, un groupe alkyle en C₁-C₄ qui peut être substitué par OR, OSO₃H, SO₃H, COOR ou des halogènes,
R représente H, CH₃ ou C₂H₅,
R² représente H, un groupe alkyle en C₁-C₄, Cl, Br, un groupe alcoxy en C₁-C₄ ou COOH,
i est égal à 0 ou 1,
j est égal à 0, 1 ou 2,
r est égal à 2 ou 3, plus particulièrement à 2,
X représente F, Cl ou Br,
Z représente -CH=CH₂, -CH₂-CH₂-OSO₃H, -CH₂-CH₂-S₂O₃H, -CH₂-CH₂-O-CO-CH₃, -CH₂-CH₂-OPO₃H₂, -CH₂-CH₂-OH, et
n est égal à 1 ou 2.

2. Colorants réactifs selon la revendication 1, caractérisés en ce qu'ils répondent à la formule suivante : 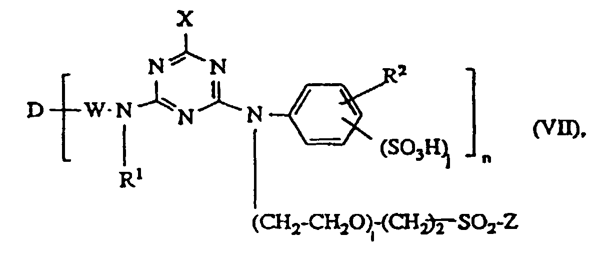 dans laquelle:
D représente le radical d'un colorant organique de la série monoazoïque, polyazoïque ou de la série des complexes métalliques de colorants azoïques, qui ne contient pas d'autres groupes réactifs avec les fibres,
R¹ et R² représentent chacun, indépendamment l'un de l'autre, H, CH₃ ou C₂H₅,
W représente une liaison directe ou un pont,
i est égal à 0 ou 1,
j est égal à 0 ou 1,
X représente Fou Cl,
Z représente CH₂-CH₂-OSO₃H ou CH=CH₂, et
n est égal à 1 ou 2.

3. Colorants réactifs selon au moins une des revendications qui précèdent, caractérisés en ce qu'ils répondent à la formule suivante : 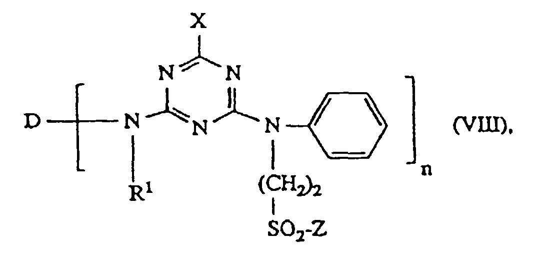 dans laquelle:
D représente le radical d'un colorant organique de la série monoazoïque, polyazoïque ou de la série des complexes métalliques de colorants azoïques, qui ne contient pas d'autres groupes réactifs avec les fibres,
X représente F,
Z représente CH₂-CH₂-OSO₃H,
R¹ représente H, et
n est égal à 1.

4. Colorants réactifs selon au moins une des revendications qui précèdent, caractérisés en ce qu'ils répondent à l'une des formules suivantes, dans lesquelles :
B représente un radical de formule : 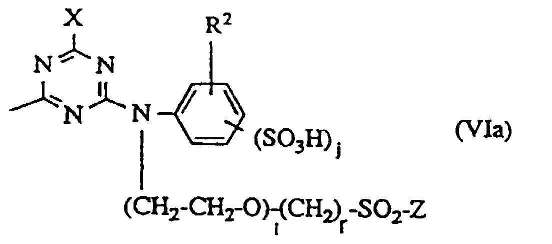
et X, i, R², j, r et Z ont les significations indiquées dans la revendication 1 : 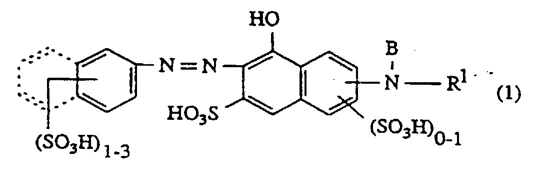 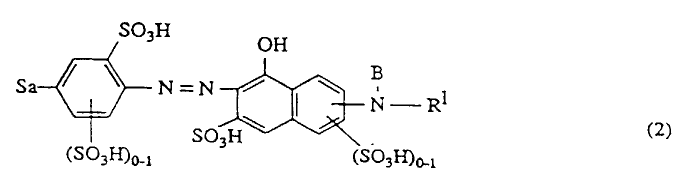 avec Sa = OCH₃ ou OC₂H₅ 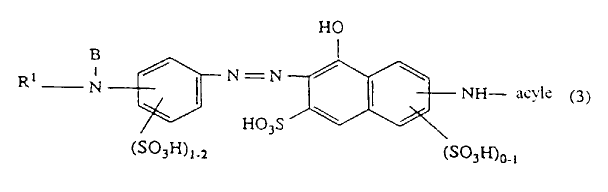 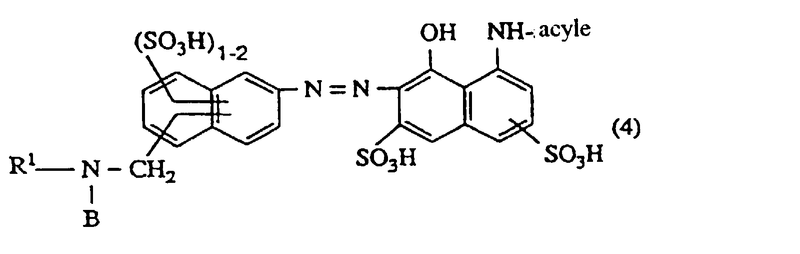 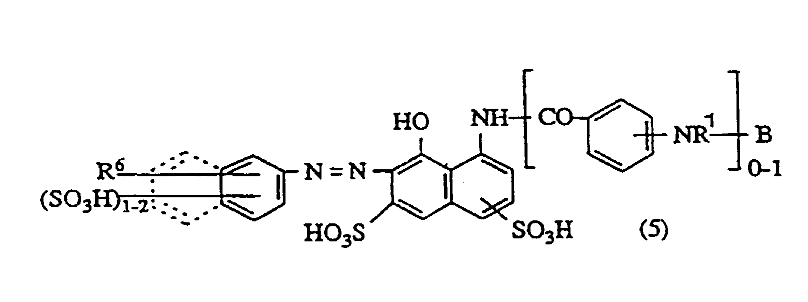 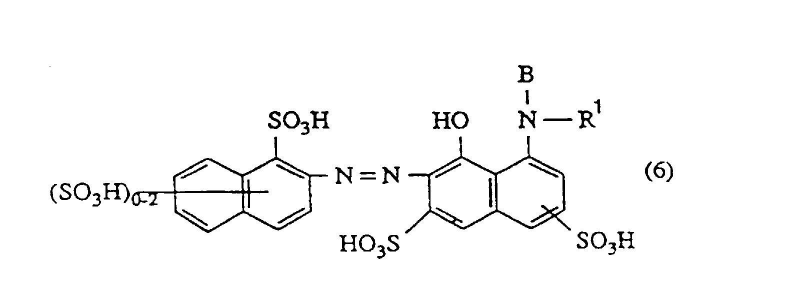 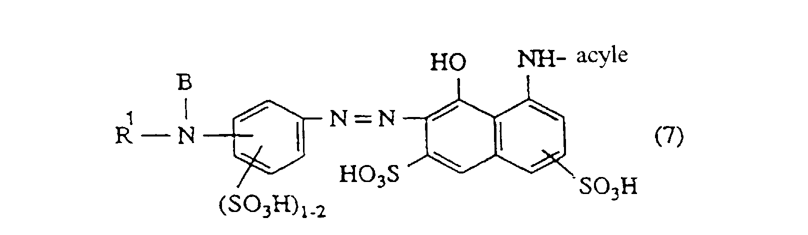 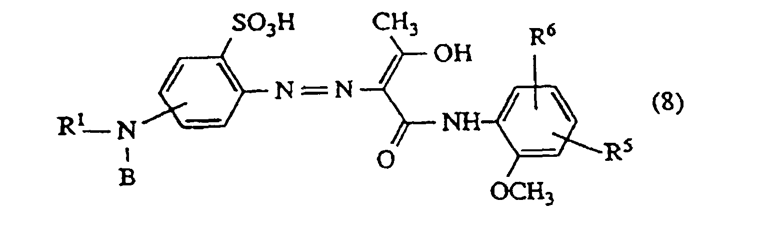 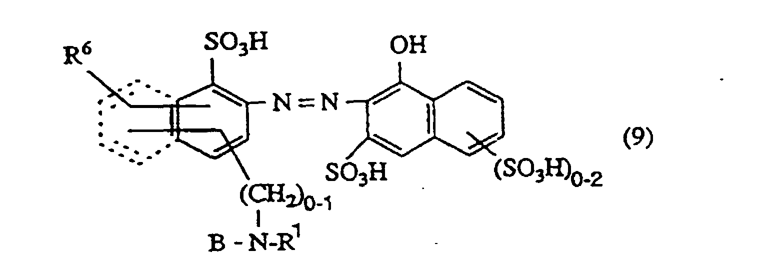 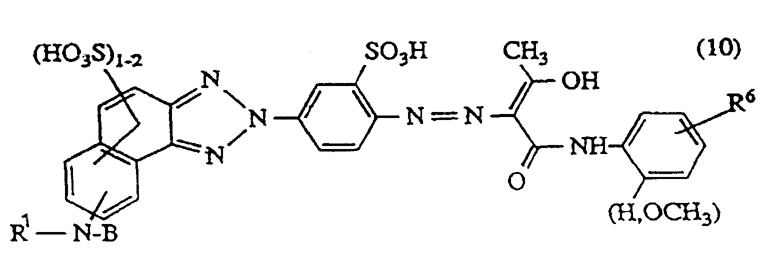 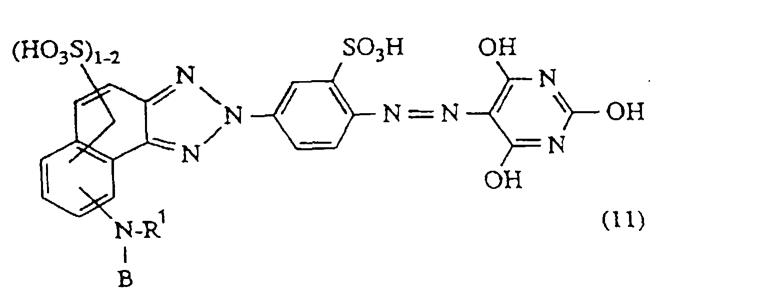 Les complexes métalliques des colorants de formules (12) à (42): 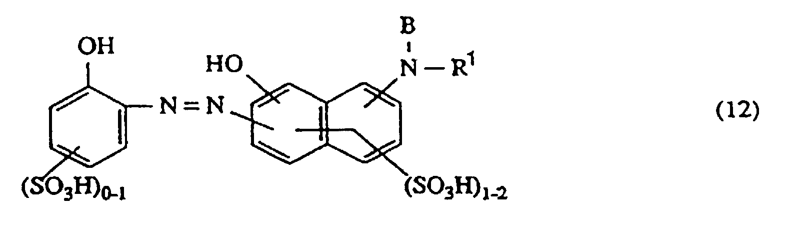 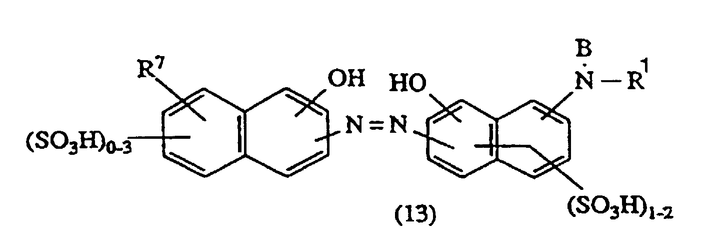 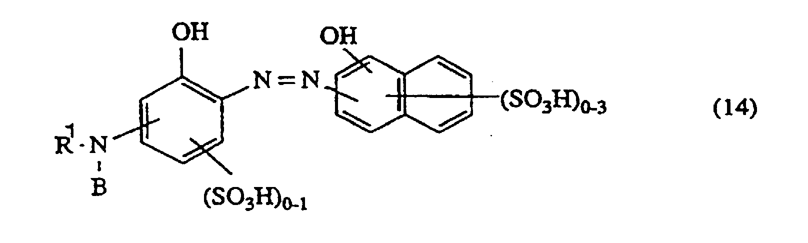 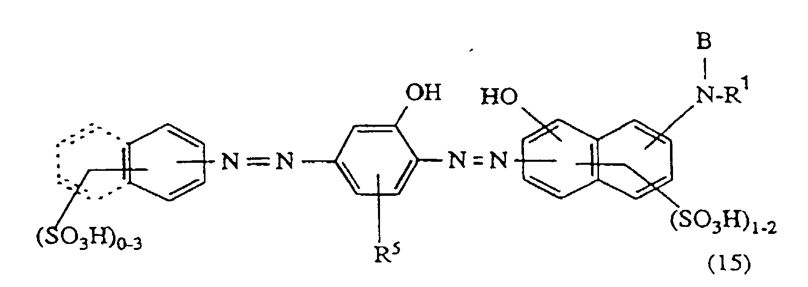 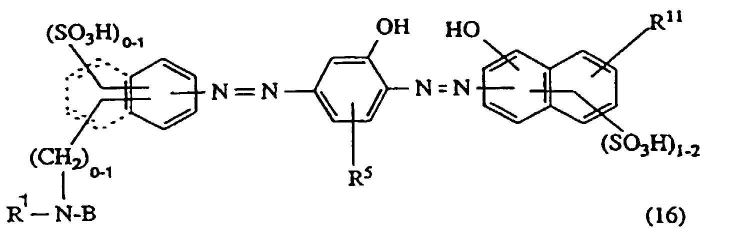 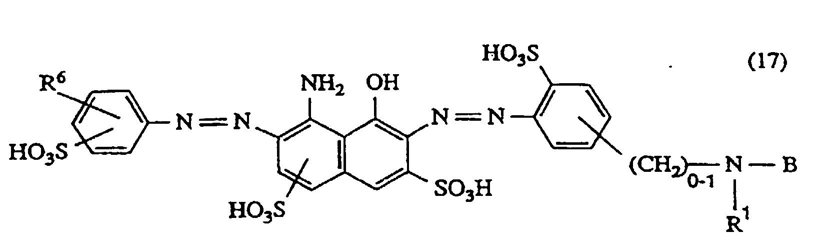 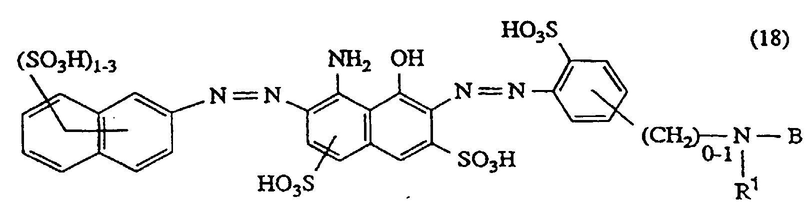 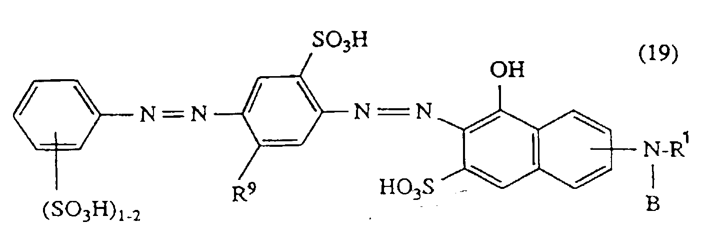 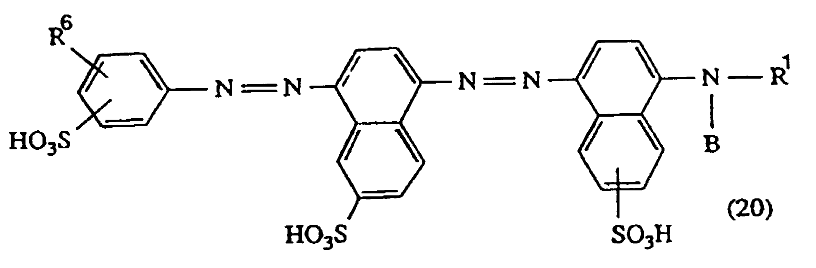 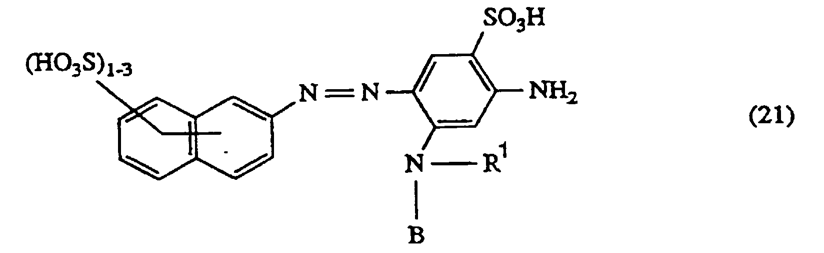 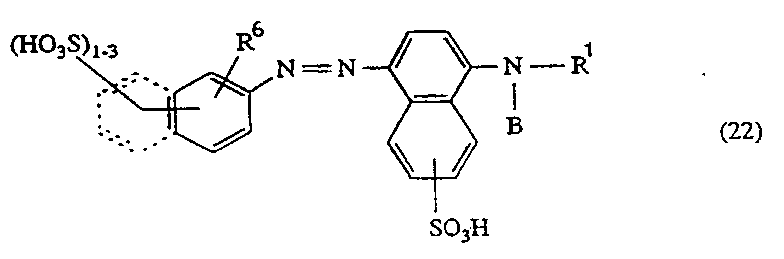 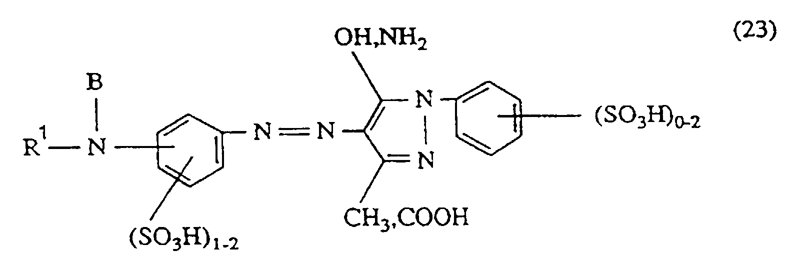 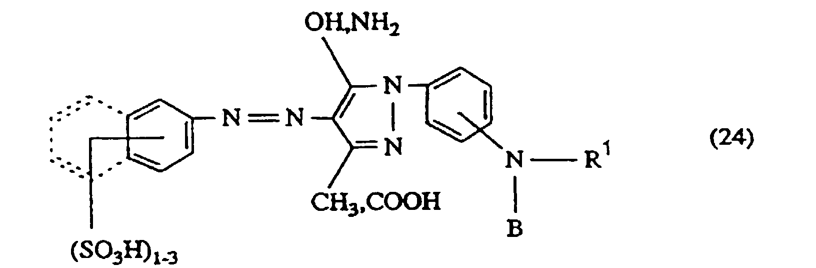 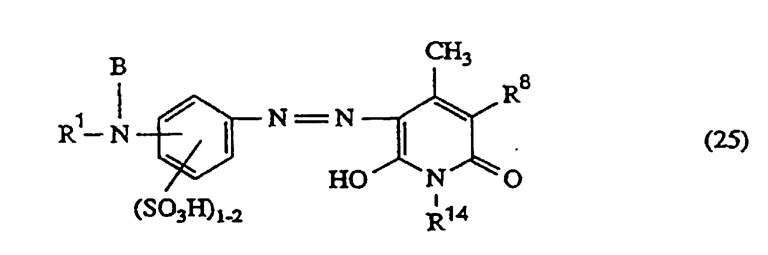 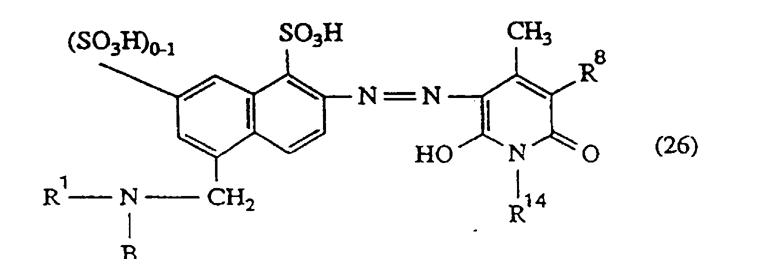 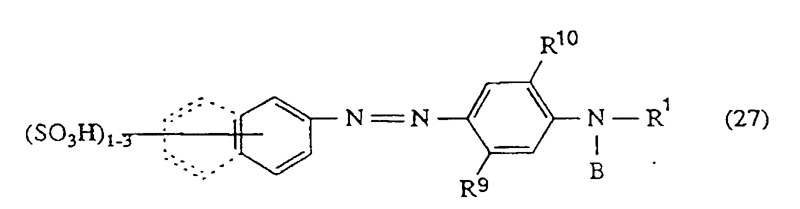 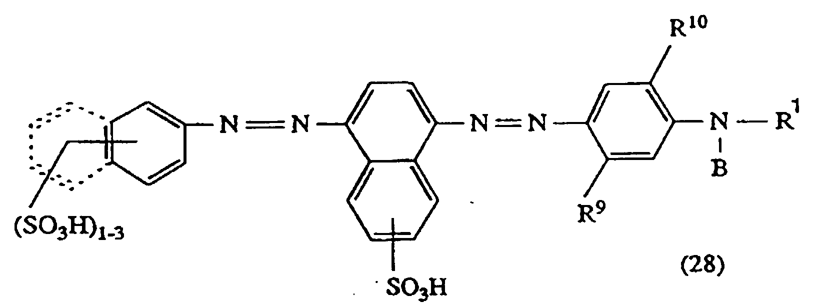 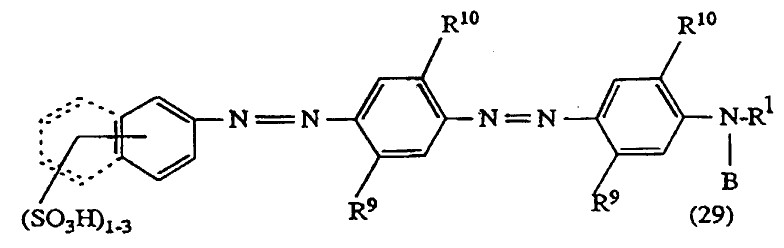 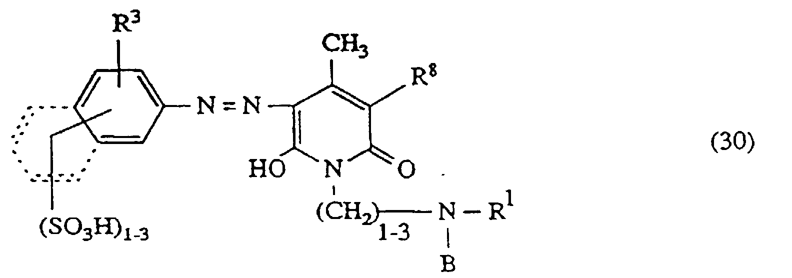 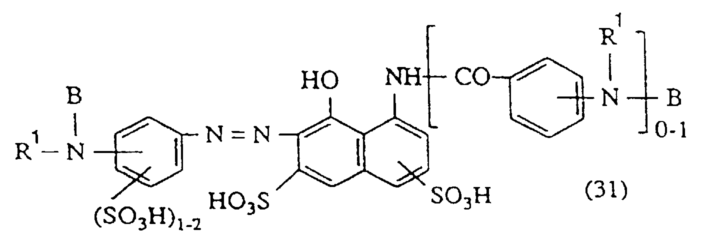 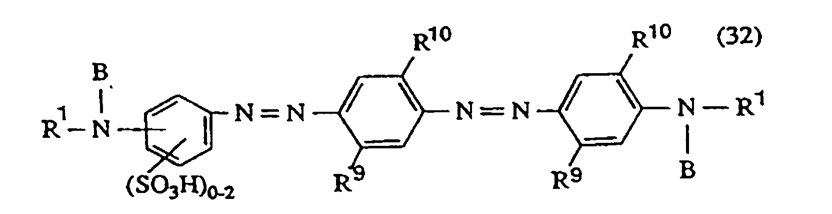 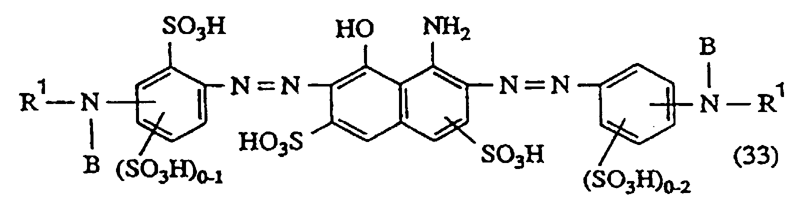 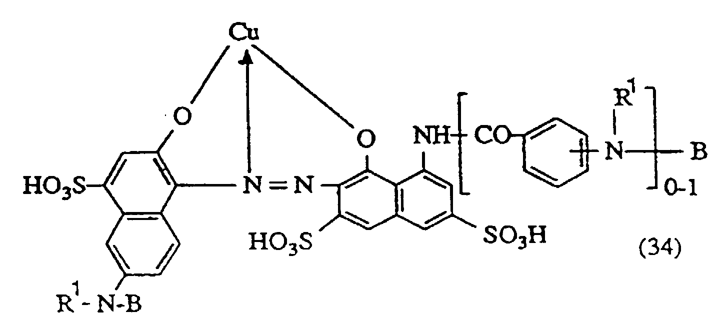 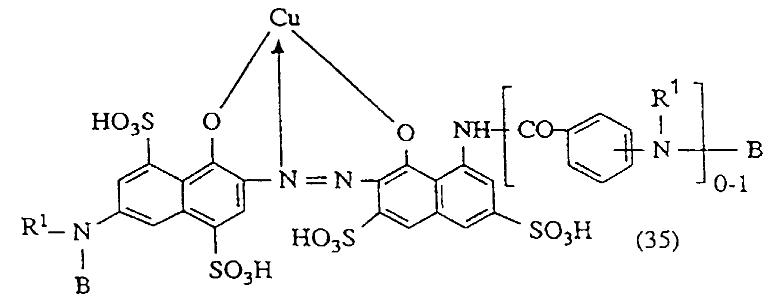 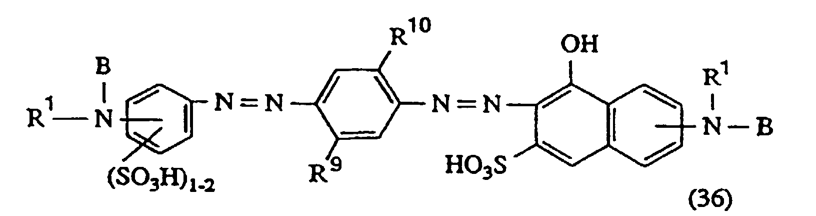 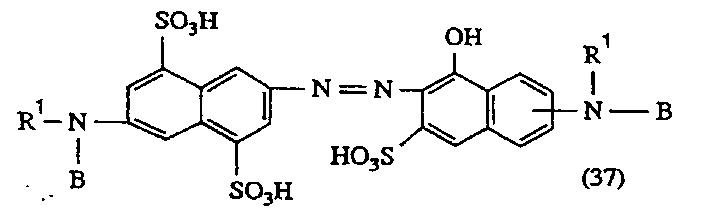 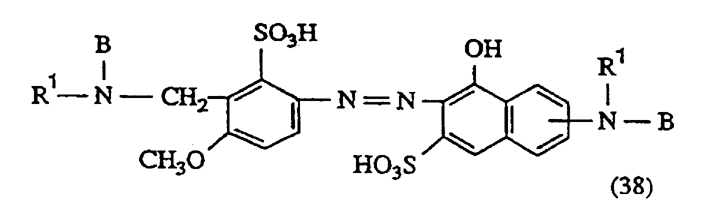 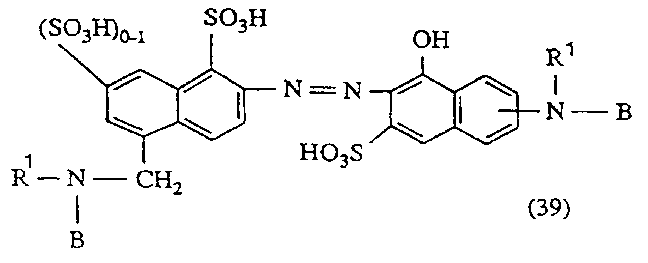 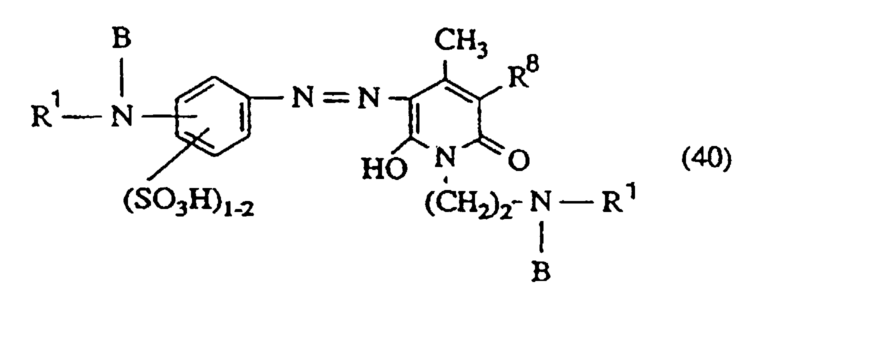 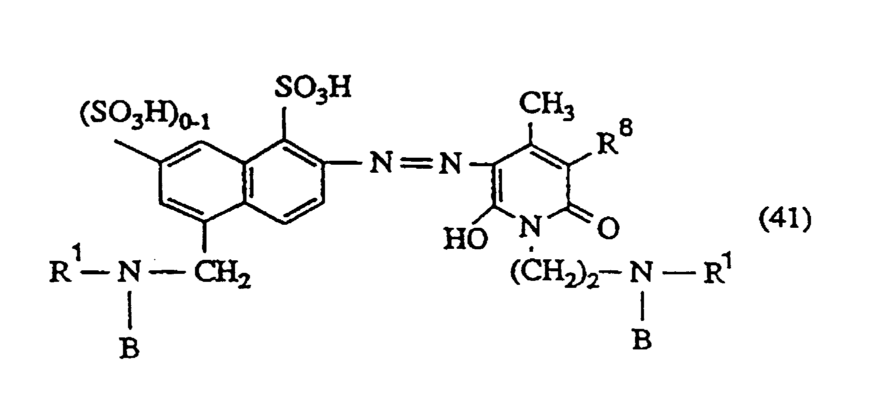 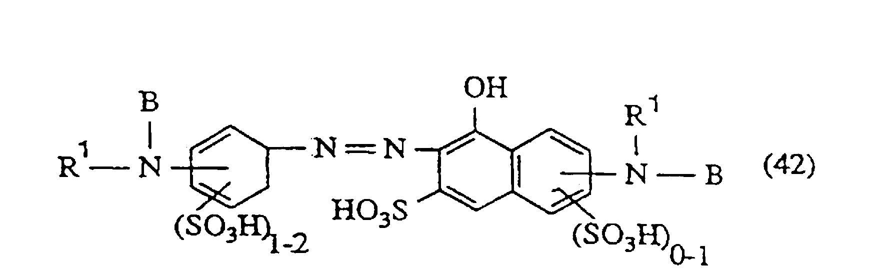 dans lesquelles :
le groupe "acyle" est un groupe acétyle ou benzoyle éventuellement substitué,
R¹⁴ représente H, un groupe alkyle un groupe alkyle en C₁-C₂ éventuellement substitué par SO₃H, NH₂,
R¹ représente H, CH₃ ou C₂H₅,
R³ représente H ou un groupe sulfo,
R⁵ représente H, CH₃, OCH₃ ou Cl,
R⁶ représente H, un groupe alkyle en C₁-C₄, alcoxy en C₁-C₄, Cl, Br, COOH, SO₃H,
R⁷ représente H, OH, NH₂, NHCOCH₃, NHCOPh, Cl, un groupe alcoxy en C₁-C₄ ou alkyle en C₁-C₄,
R⁸ représente H, SO₃H, CH₂SO₃H, Cl, un groupe alkylsulfonyle en C₁-C₄, CN, carboxamide, plus spécialement CONH₂,
R⁹ représente H, un groupe alkyle en C₁-C₄, alcoxy en C₁-C₄, Cl, Br, un groupe acylamino, plus spécialement (alkyle en C₁-C₄)carbonylamino ou arylcarbonylamino tel que phénylcarbonylamino éventuellement substitué, alkylsulfonylamino en C₁-C₄, aminocarbonylamino, alkylsulfonylamino en C₁-C₄, arylsulfonylamino,
R¹⁰ représente H, un groupe alkyle en C₁-C₄, alcoxy en C₁-C₄, OH, SO₃H, et les noyaux condensés représentés en trait interrompu indiquent dans une variante des systèmes naphtaléniques possibles.

5. Colorants réactifs selon au moins une des revendications qui précédent, pour lesquels:
X = Cl,
i = 0,
j = 0 ou 1,
Z = -CH₂-CH₂-OSO₃H ou -CH=CH₂, et
R² = H.

6. Colorants réactifs selon au moins une des revendications qui précèdent, pour lesquels :
X = F,
i = 0,
j = 0,
Z = -CH₂-CH₂-OSO₃H, -CH=CH₂, et
R² = H.

7. Colorants réactifs selon au moins une des revendications qui précèdent, pour lesquels :
Z = -CH₂-CH₂-OSO₃H.

8. Colorants réactifs selon la revendication 1, caractérisés en ce que le radical D est substitué une ou deux fois par le groupe : 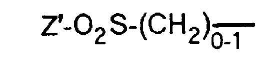 dans lequel :
Z' représente -CH=CH₂, -CH₂-CH₂OSO₃H, -CH₂-CH₂-Cl, -CH₂-CH₂-Br, -CH₂-CH₂-S₂O₃H, -CH₂-CH₂-O-CO-CH₃, -CH₂-CH₂-OPO₃H₂ ou -CH₂-CH₂-OH.

9. Colorants réactifs selon la revendication 8, caractérisés en ce que :
n représente 1,
W est une liaison directe, et
D représente un radical de formule générale (IX): 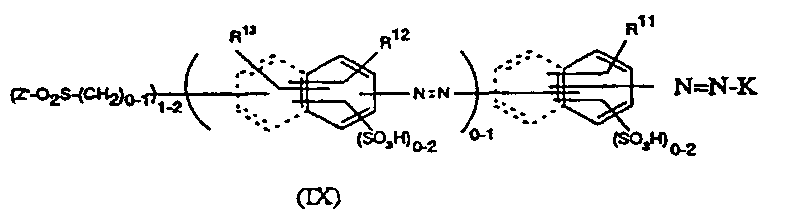
dans laquelle:
R¹¹ représente H, un groupe alkyle en C₁-C₄, alcoxy en C₁-C₄, Cl, Br ou un groupe acylamino,
R¹² représente H, un groupe alkyle en C₁-C₄, Cl, Br, un groupe alcoxy en C₁-C₄, ou COOH,
R¹³ représente H, un groupe alkyle en C₁-C₄, alcoxy en C₁-C₄, SO₃H, Cl ou Br,
K représente un radical bivalent de formule générale (Xa) à (Xd) : 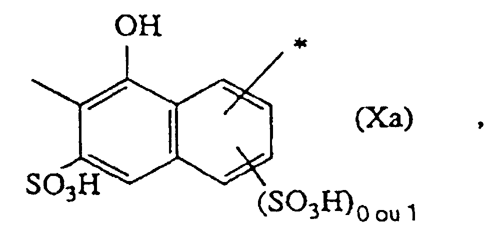 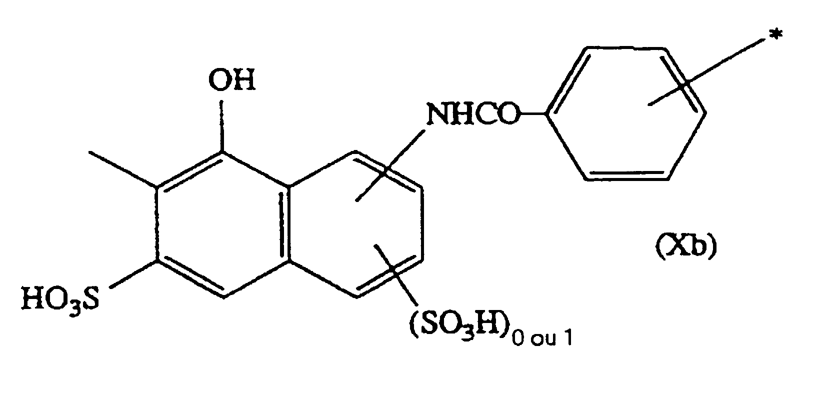 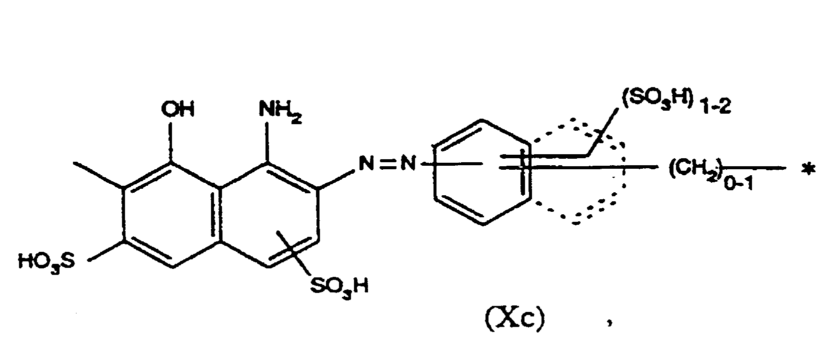 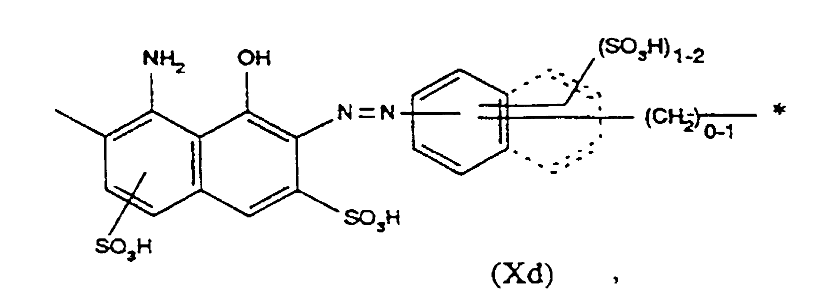
dans lesquelles :
l'astérisque (*) indique des liaisons rejoignant le groupe -NR¹-B, et B a la signification indiquée dans la revendication 4.

10. Colorants réactifs selon la revendication 9, caractérisés en ce que :
R¹ et R² représentent chacun, indépendamment l'un de l'autre, H, CH₃ ou C₂H₅,
j est égal à 0 ou 1,
X représente F ou Cl, et
Z, Z' représentent chacun, indépendamment l'un de l'autre, CH₂-CH₂-OSO₃H ou CH=CH₂.

11. Colorants réactifs selon la revendication 1, caractérisés en ce que:
D représente un radical de colorant de formule (IX), et
R¹, R² = H,
i = 0,
j = 0,
r = 2, et
Z', Z = -CH₂CH₂OSO₃H.

12. Colorants réactifs selon la revendication 9, caractérisés en ce qu'ils répondent à l'une des formules ci-après, dans lesquelles :
B représente un radical de formule : 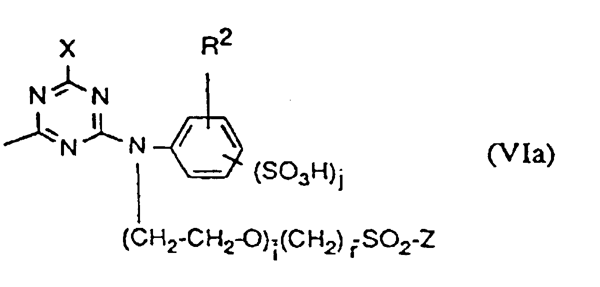 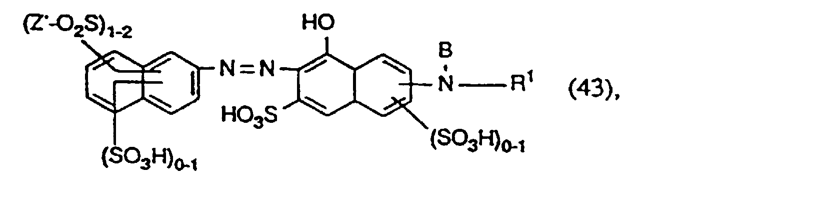 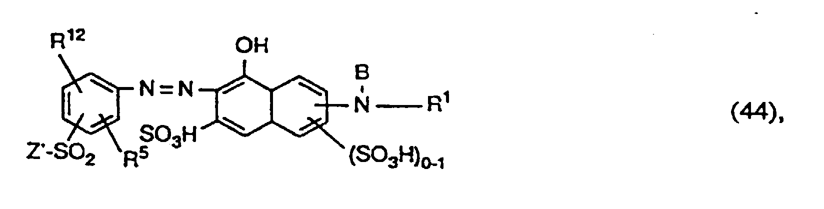 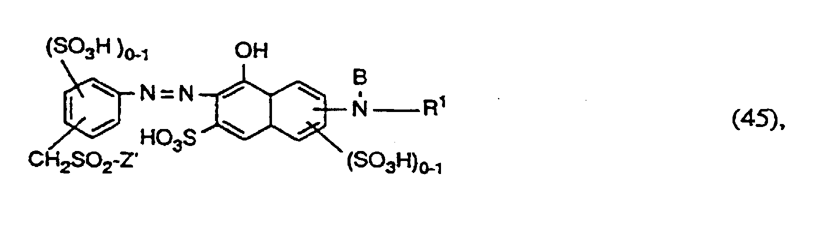 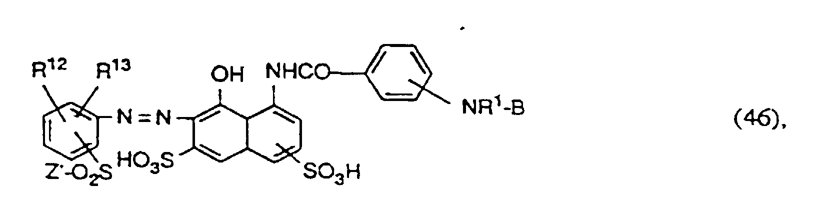 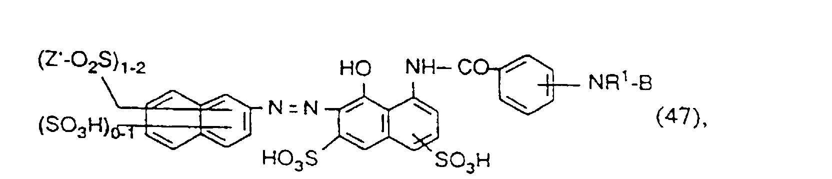 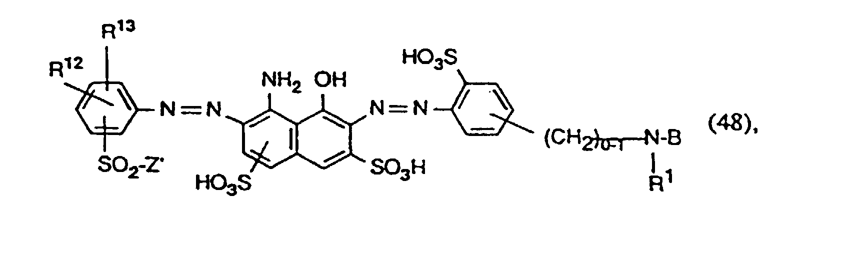 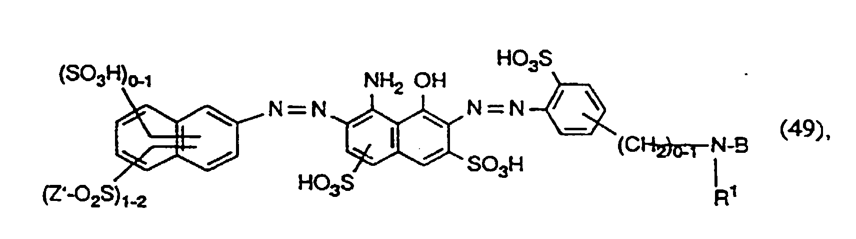 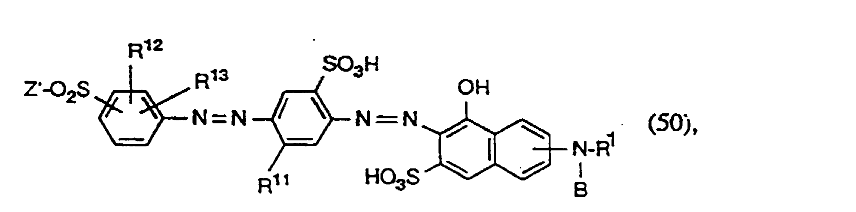
Les symboles de ces formules ayant les significations suivantes :
R¹ représente H, CH₃ ou C₂H₅,
R¹¹ représente H, un groupe alkyle en C₁-C₄, alcoxy en C₁-C₄, Cl, Br, un groupe acylamino, Cl ou Br,
R¹² représente H, un groupe alkyle en C₁-C₄, alcoxy en C₁-C₄, Cl, Br ou COOH.
R¹³ représente H, un groupe alkyle en C₁-C₄, alcoxy en C₁-C₄, Cl, Br, SO₃H, et les noyaux condensés représentés en trait interrompu représentent, dans une variante, un système naphtalénique possible.

13. Procédé de préparation de colorants réactifs de formule : 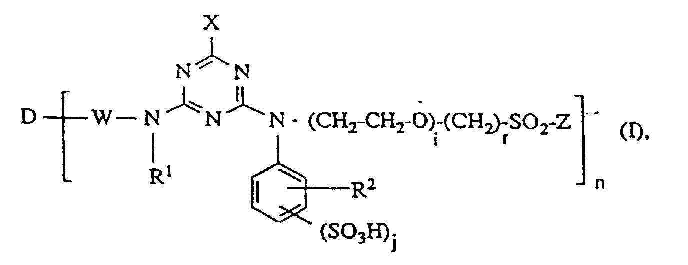 dans laquelle :
D représente le radical d'un colorant organique de la série monoazoïque, polyazoïque ou de la série des complexes métalliques de colorants azoïques,
W représente une liaison directe ou un pont,
R¹ représente H, un groupe alkyle en C₁-C₄ qui peut être substitué par OR, OSO₃H, SO₃H, COOR ou des halogènes,
R représente H, CH₃ ou C₂H₅,
R² représente H, un groupe alkyle en C₁-C₄, Cl, Br, un groupe alcoxy en C₁-C₄ ou COOH,
i est égal à 0 ou 1,
j est égal à 0, 1 ou 2,
r est égal à 2 ou 3, plus particulièrement à 2,
X représente F, Cl ou Br,
Z représente -CH=CH₂, -CH₂-CH₂-OSO₃H, -CH₂-CH₂-S₂O₃H, -CH₂-CH₂-O-CO-CH₃, -CH₂-CH₂-OPO₃H₂, -CH₂-CH₂-OH, et
n est égal à 1 ou 2,
caractérisé en ce que :
a) on condense des colorants de formule : 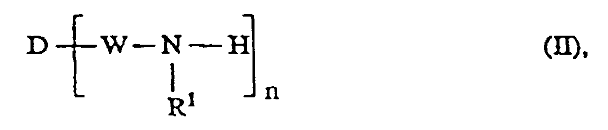 avec n moles de trihalogénotriazines de formule : 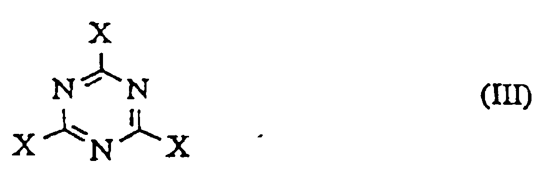 ce qui donne des composés de formule : 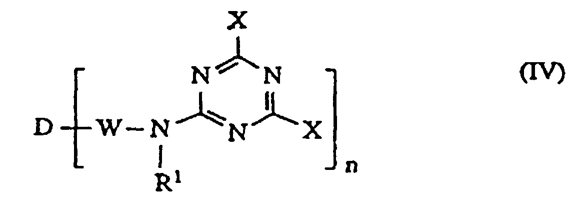 que l'on condense avec n moles des composants de formule : 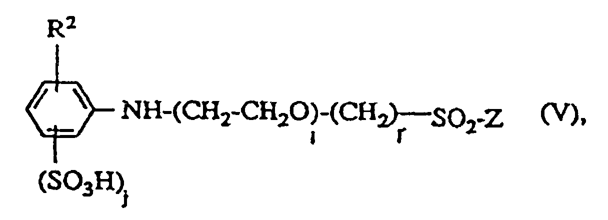 ou bien
b) dans un ordre inverse, on condense des trihalogénotriazines de formule (III) avec les composants de formule (V), ce qui donne les produits de condensation primaire 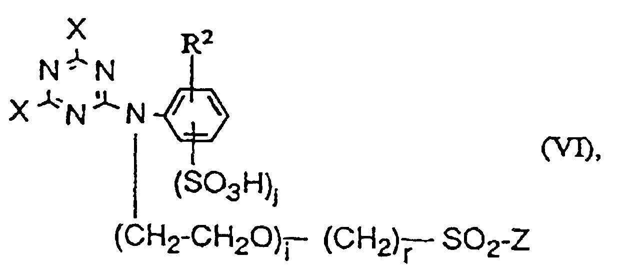 que l'on condense ensuite avec n moles des composés de formule (VI), ce qui donne les colorants de formule (II),
ou bien
c) on condense des produits précurseurs appropriés avec les trihalogénotriazines (III) et les composants de formule (V), ou bien on condense des produits précurseurs appropriés avec les produits de condensation primaire de formule (VI), et on termine ensuite la synthèse des colorants.

14. Procédé pour la teinture et l'impression de substrats à l'aide d'un colorant réactif, caractérisé en ce que l'on utilise un colorant réactif selon au moins une des revendications qui précèdent.

15. Composés de formule générale : 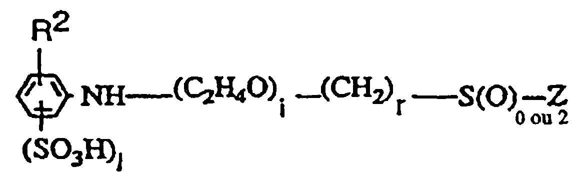 y compris leurs sels et leurs produits de réaction portant des groupes protecteurs, R², Z, j et i ayant les significations indiquées dans la revendication 1, et r est égal à 3.

16. Procédé de préparation des composés de formule : 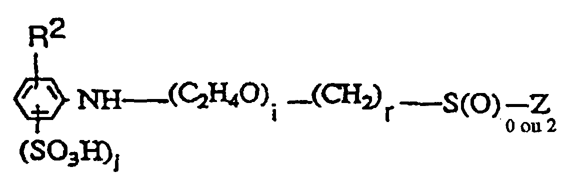 dans laquelle :
R², Z, j, i et r ont les significations indiquées dans la revendication 1, par sulfatation de composés de formule : 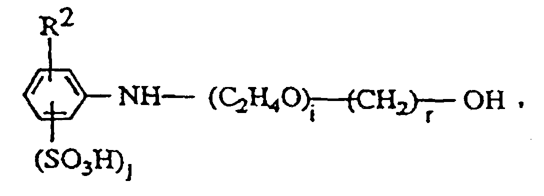 puis réaction avec le mercaptoéthanol, donnant : 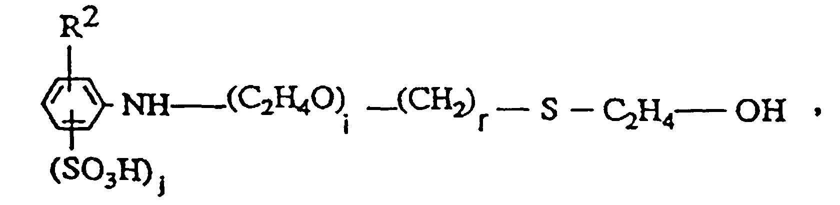 dans lequel, le cas échéant, on bloque le groupe NH par un groupe protecteur, après quoi on oxyde en le composé : 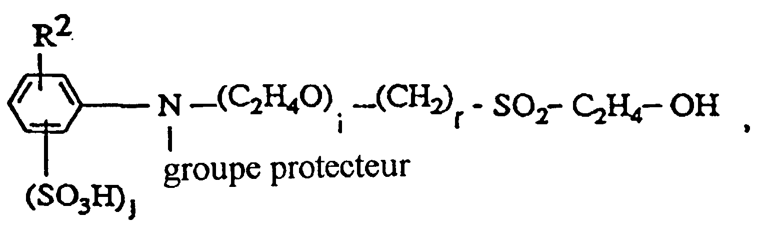 on scinde éventuellement le groupe protecteur, ce qui donne : 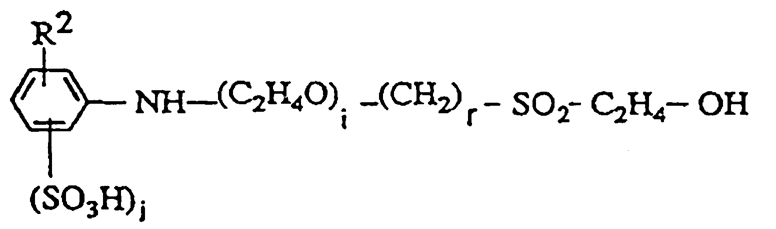 dans lequel, le cas échéant, on convertit de la manière habituelle le groupe SO₂C₂H₄-OH en un groupe vinylsulfonyle ou bien on convertit le groupe -CH₂-CH₂-OH en les autres groupes scindables énumérés en référence à Z.

17. Colorants réactifs selon la revendication 1, pour lesquels i est égal à 1.

18. Composés selon la revendication 15, pour lesquels i est égal à 1.
